# EUROPEAN PATENT SPECIFICATION

(11) **EP 1 350 262 B1**
(45) Date of publication and mention of the grant of the patent: **11.06.2008**
(21) Application number: 01273824.1
(22) Date of filing: 10.12.2001
(51) Int. Cl.: C12N 15/11, A61K 31/70

(54) **CPG-LIKE NUCLEIC ACIDS AND METHODS OF USE THEREOF**
CPG-ARTIGE NUKLEINSÄUREN UND VERFAHREN ZU IHRER VERWENDUNG
ACIDES NUCLEIQUES DU TYPE CPG ET PROCEDES D'UTILISATION CORRESPONDANTS

(30) Priority: 08.12.2000 US 254341 P
(43) Date of publication of application: 08.10.2003
(73) Proprietor: Coley Pharmaceuticals GmbH, 40225 Düsseldorf (DE)
(72) Inventor: SCHETTER, Christian, 40723 Hilden (DE); VOLLMER, Jörg, 40591 Düsseldorf (DE)
(74) Representative: Jump, Timothy John Simon
(86) International application number: PCT/IB2001/002888
(87) International publication number: WO 2002/069369

(56) References cited:
- WO-A-01/93903
- GOECKERITZ B.E. ET AL.: "Multivalent cross-linking of membrane Ig sensitizes murine B cells to a broader spectrum of CpG-containing oligodeoxynucleotide motifs, including their methylated counterparts, for stimulation of proliferation and Ig secretion" INTERNATIONAL IMMUNOLOGY, vol. 11, no. 10, 1999, pages 1693-1700, XP000938323
- BOGGS R.T. ET AL.: "Characterization and modulation of immune stimulation by modified oligonucleotides" ANTISENSE & NUCLEIC ACID DRUG DEVELOPMENT, vol. 7, 1 October 1997 (1997-10-01), pages 461-471, XP002053418 ISSN: 1087-2906
- YAMAMOTO T. ET AL.: "Lipofection of synthetic oligodeoxyribonucleotide having a palindromic sequence of AAGCTT to murine splenocytes enhances Interferon production and natural killer activity" MICROBIOLOGY AND IMMUNOLOGY, vol. 38, no. 10, 1994, pages 831-836, XP008012406
- HENRY S. ET AL.: "Chemically modified olioligonucleotides exhibit decreased immune stimulation in mice" THE JOURNAL OF PHARMACOLOGY AND EXPERIMENTAL THERAPEUTICS, vol. 292, no. 2, February 2000 (2000-02), pages 468-479, XP002310045
- ZHAO Q. ET AL.: "Immunostimulatory activity of CpG containing phosphorothioate oligodeoxynucleotide is modulated by modification of a single deoxynucleoside" BIOORGANIC & MEDICINAL CHEMISTRY LETTERS, OXFORD, GB, vol. 10, no. 10, May 2000 (2000-05), pages 1051-1054, XP004204603 ISSN: 0960-894X
- KRIEG A.M.: "Mechanisms and applications of immune stimulatory CpG oligodeoxynucleotides" BIOCHIMICA ET BIOPHYSICA ACTA . GENE STRUCTURE AND EXPRESSION, ELSEVIER, AMSTERDAM, NL, vol. 1489, no. 1, 10 December 1999 (1999-12-10), pages 107-116, XP004275526 ISSN: 0167-4781
- BAILLY C. ET AL.: "Recognition elements that determine affinity and sequence-specific binding to DNA of 2QN, a biosynthetic bis-quinoline analogue of echinomycin" ANTI-CANCER DRUG DESIGN, vol. 14, 1999, pages 291-303, XP008040033

## Description

### RELATED APPLICATION

The application claims priority to United States provisional patent application 60/254,341 filed on December 8, 2000.

### FIELD OF THE INVENTION

The present invention relates generally to immunostimulatory nucleic acids, compositions thereof, and methods of using the immunostimulatory nucleic acids.

### BACKGROUND OF THE INVENTION

Bacterial DNA, but not vertebrate DNA, has strong immunostimulatory effects for a wide variety of human and murine immune cells. Krieg AM et al. (1995) Nature 374:546-9; Hartmann G et al. (1999) Proc Natl Acad Sci USA 96:9305-10; Hartmann G et al. (2000) J Immunol 164:1617-24; Bauer M et al. (1999) Immunology 97:699-705; Ballas ZK et al. (1996)J Immunol 157:1840-5. Unmethylated CpG dinucleotides are less frequent in eukaryotic DNA than in bacterial DNA. Krieg AM et al. (1995) Nature 374:546-9. It has been reported that unmethylated CpG dinucleotides within the context of specific flanking bases (referred to as CpG motifs) have a wide variety of effects on human immune cells such as B cells, natural killer (NK) cells, T cells, macrophages, monocytes and dendritic cells. Parronchi P et al. (1999) J Immunol 163:5946-53; Krieg AM (1999) Biochim Biophys Acta 1489:107-16; Krieg AM et al: (1995) Nature 374:546-9; Kranzer K et al. (2000) Immunology 99:170-8; Hartmann G et al. (1999) Proc Natl Acad Sci USA 96:9305-10. Methylated CpG dinucleotides, in contrast, have been reported to be nonstimulatory. Parronchi P et al. (1999) J Immunol 163:5946-53; Hartmann G et al. (1999) Proc Natl Acad Sci USA 96:9305-10; Hartmann G et al. (2000) J Immunol 164:944-53; Hartmann G et al. (2000) J Immunol 164:1617-24.

As methylated vertebrate DNA is not immunostimulatory and methylated CpG oligonucleotides were reported to be nonstimulatory for immune cells (Parronchi P et al. (1999) J Immunol 163:5946-53; Hartmann G et al. (1999) Proc Natl Acad Sci USA 96:9305-10; Hartmann G et al. (2000) J Immunol 164:944-53), it was thought that methylation would mainly prevent immunostimulation by CpG-containing nucleic acids. For most applications in the field of antisense technology, where immunostimulation by the applied oligonucleotide is undesirable, many workers in the antisense field started to utilise methylated oligonucleotides for their research for the specific purpose of avoiding unwanted immunostimulation.

Further, while a number of studies reported that methylation of the C of the CpG dinucleotide effectively abrogated the immunostimulatory effect of CpG, no previous studies have reported the immunostimulatory effects of CpI.

Boggs, R.T. et al (1997) Antisense & Nucleic Acid Drug Development vol. 7 pp 461-471 investigated the stimulatory effects of the CpG motif in specific sequence contexts, the effect of phosphorothioate backbones and phosphodiester backbones, methylating the cytosine of the CpG and modifying all of the cytosines in an ODN.

Henry, S. et al (2000). The Journal of Pharmacology and Experimental Therapeutics vol 292, no.2, pp 468-479 investigated the immunostimulatory properties of phosphorothioate oligodeoxynucleotides in mice and the effects of chemical modifications such as 5-methyl cytosine and 2'-methoxyethyl substituents.

### SUMMARY OF THE INVENTION

The invention provides compositions and methods useful in inducing an immune response and in the treatment of allergy, asthma, cancer, infection, anaemia, thrombocytopenia, and neutropenia. The compositions are related to CpG nucleic acids and are termed "CpG-like nucleic acids" because they incorporate specific substitutions for the C, the G, or the C and the G of the CpG dinucleotide while substantially retaining the immunostimulatory properties of CpG nucleic acid molecules.

The present invention provides a composition comprising an immunostimulatory nucleic acid having a sequence including at least the following formula:

5' X₁X₂CGX₃X₄ 3'

wherein C is a nucleotide in which the nucleoside moiety is 2'-methoxy cytidine and wherein X₁, X₂, X₃ and X₄ are nucleotides, in an amount effective to induce an immune response. An immunostimulatory nucleic acid according to this embodiment of this aspect of the invention is referred to herein as a 2'-methoxy-C or 2'-OMe-C CpG nucleic acid or, equivalently, a 2'-methoxy-C or 2'-OMe-C CpG oligonucleotide. Also according to this aspect of the invention in some embodiments the composition further includes a pharmaceutically acceptable carrier and the immunostimulatory nucleic acid is present in an amount effective to induce an immune response.

According to the foregoing aspects and embodiments of the invention, the following further apply. In some embodiments the immunostimulatory nucleic acid is an isolated nucleic acid. In some embodiments the immunostimulatory nucleic acid has between 6 and 100 nucleotides, and in certain preferred embodiments, between 8 and 40 nucleotides.

Also according to preferred embodiments the immunostimulatory nucleic acid has a modified backbone. In more preferred embodiments the modified backbone is a phosphate modified backbone. In some embodiments the immunostimulatory nucleic acid is a synthetic nucleic acid.

The immunostimulatory nucleic acid in some embodiments is at least 18 nucleotides long and is not an antisense nucleic acid.

The following also apply to foregoing pharmaceutical compositions of the invention that include a CpG-like nucleic acid and a pharmaceutically acceptable carrier. In some embodiments the pharmaceutically acceptable carrier is a sustained-release device.

The pharmaceutical compositions in some embodiments further include an antigen.

In some embodiments the pharmaceutical composition further includes an anti-cancer medicament. Preferably the anti-cancer medicament is selected from the group consisting of a monoclonal antibody, a chemotherapeutic agent, and a radiotherapeutic agent:

In some embodiments the pharmaceutical composition further includes an antiviral agent, an antibacterial agent, an antifungal agent, or an antiparasitic agent.

In some embodiments the pharmaceutical composition further includes an ulcer medicament.

In some embodiments the pharmaceutical composition further includes an allergy medicament or an asthma medicament.

In some embodiments the pharmaceutical composition further includes an anemia medicament, a thrombocytopenia medicament, or a neutropenia medicament.

In some embodiments the pharmaceutical composition further includes a cytokine. Preferably the cytokine is selected from the group consisting of interleukin-2 (IL,2), IL-3, IL-4, IL-18, interferon alpha (IFN-α), IFN-γ, tumor necrosis factor alpha (TNF-α), Flt3 ligand, granulocyte colony-stimulating factor (G-CSF), and granulocyte-macrophage colony-stimulating factor (GM-CSF).

In some embodiments the pharmaceutical composition includes at least two immunostimulatory nucleic acids having different sequences.

In some embodiments the pharmaceutical composition further includes a CpG nucleic acid having at least one unmethylated CpG motif.

Another aspect of the invention provides a method for inducing an immune response. The method involves administering to a subject an immunostimulatory nucleic acid having a sequence including at least the following formula:

5' X₁X₂CGX₃X₄ 3'

wherein C is a nucleotide in which the nucleoside moiety is 2'-methoxy cytidine and wherein X₁X₂X₃ and X₄ are nucleotides, in an amount effective to induce an immune response.

According to any of the foregoing methods of the invention, the following further apply. In some embodiments the immunostimulatory nucleic acid is an isolated nucleic acid. In some embodiments the immunostimulatory nucleic acid has between 6 and 100 nucleotides, and in certain preferred embodiments, between 8 and 40 nucleotides.

Also according to preferred embodiments the immunostimulatory nucleic acid has a modified backbone. In more preferred embodiments the modified backbone is a phosphate modified backbone.

In some embodiments the subject is selected from the group consisting of dog, cat, horse, cow, pig, sheep, goat, rabbit, guinea pig, non-human primate (e.g., monkey), chicken, and fish (aquaculture species, e.g., sahnon).

In some embodiments the immunostimulatory nucleic acid is a synthetic nucleic acid.

According to some embodiments the method further includes administering an antigen. Preferably the antigen is selected from the group consisting of an allergen, a tumor antigen, a viral antigen, a bacterial antigen, a fungal antigen, and a parasitic antigen. In some embodiments the antigen is administered by a mucosal route. Preferably the mucosal route is selected from the group consisting of oral, nasal, rectal, vaginal, transdermal, and ocular, In some embodiments the antigen is administered by a parenteral route. Preferably the parenteral route is selected from the group consisting of intravenous, subcutaneous, intramuscular, and direct injection.

In certain embodiments the subject is at risk of developing an infectious disease and the immunostimulatory nucleic acid is administered in an effective amount for preventing the infectious disease.

In certain embodiments the subject has an infectious disease and the immunostimulatory nucleic acid is administered in an effective amount for treating the infectious disease.

In certain embodiments the subject is at risk of developing a cancer and the immunostimulatory nucleic acid is administered in an effective amount for preventing or for treating the cancer.

In certain embodiments the subject has or is at risk of developing an allergy and the immunostimulatory nucleic acid is administered in an effective amount for treating or preventing the allergy.

In some embodiments the subject has or is at, risk of developing asthma and the immunostimulatory nucleic acid is administered in an effective amount for treating or preventing asthma.

In some embodiments the method further includes administering an anti-cancer therapy. Preferably the anti-cancer therapy is a monoclonal antibody specific for a tumor cell, a chemotherapy, or a radiotherapy.

In some embodiments the immunostimulatory nucleic acid is administered to a subject that has or is at risk of developing an immunodeficiency, in an effective amount for enhancing stimulating bone marrow proliferation in the subject. In some embodiments the subject that has or is at risk of developing an immunodeficiency is a subject undergoing or at risk of undergoing chemotherapy

In some embodiments the immunostimulatory nucleic acid is administered to a subject that has or is at risk of developing anemia, in an effective amount for enhancing erythropoiesis in the subject.

In some embodiments the immunostimulatory nucleic acid is administered to a subject that has or is at risk of developing thrombocytopenia, in an effective amount for enhancing thrombopoiesis in the subject.

In some embodiments the immunostimulatory nucleic acid is administered to a subject that has or is at risk of developing neutropenia, in an effective amount for enhancing neutrophil proliferation in the subject.

In some embodiments the immunostimulatory nucleic acid is administered in an effective amount for inducing cytokine production. Preferably the cytokine is selected from the group consisting of IL-1β, IL-2, IL-6, IL-12, IL-18, TNF-α, IFN-α, and IFN-γ.

In some embodiments the immunostimulatory nucleic acid is administered in an effective amount for stimulating natural killer cell activity.

In some embodiments the immunostimulatory nucleic acid is administered by a mucosal route. Preferably the mucosal route is selected from the group consisting of oral, nasal, rectal, vaginal, transdermal and ocular.

In some embodiments the immunostimulatory nucleic acid is administered by a parenteral route. Preferably the parenteral route is selected from the group consisting of intravenous, subcutaneous, intramuscular, and direct injection

In some embodiments the immunostimulatory nucleic acid is administered in a sustained-release vehicle.

### BRIEF DESCRIPTION OF THE DRAWINGS

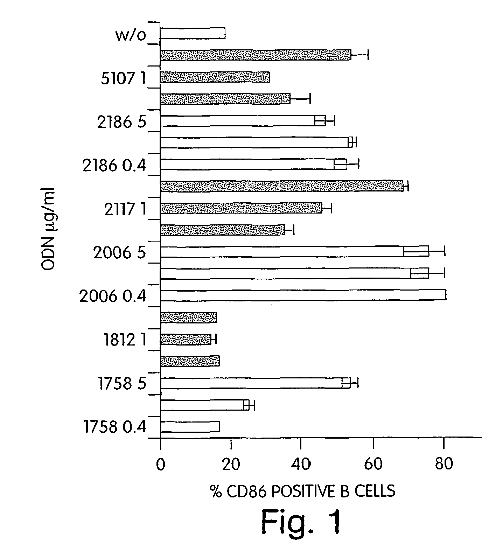

**Figure 1** is a graph depicting human B cell activation by methylated and unmethylated CpG ODN. Peripheral blood mononuclear cells (PBMC; 2x10⁶ cells/ml) of one representative donor (n = 4) were incubated with 0.4 µg/ml, 1.0 µg/ml or 5.0 µg/ml of the oligodeoxynucleotide (ODN) 2006,1758, 2186, or the corresponding methylated ODN 2117, 1812 or 5107. Negative controls were incubated without any ODN (w/o). Expression of the activation marker CD86 on CD19+ B cells was measured by flow cytometry.

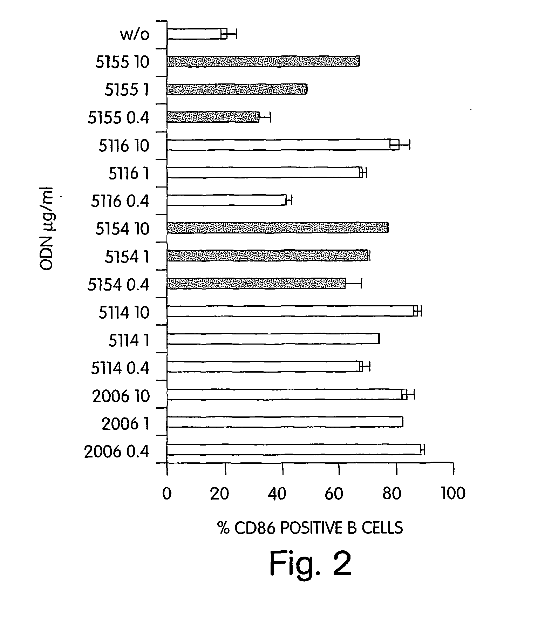

**Figure 2** is a graph depicting activation of human B cells by methylated and unmethylated CpG ODN. PBMC of one representative donor (n = 3) were incubated with 0.4 µg/ml, 1.0 µg/ml or 10.0 µg/ml of CpG ODN 2006, the antisense CpG ODNs 5114 and 5116, or the corresponding methylated ODNs 5154 and 5155. Negative controls were incubated without any ODN(w/o). B cell activation was analyzed by flow cytometry as described for **Fig. 1****.**

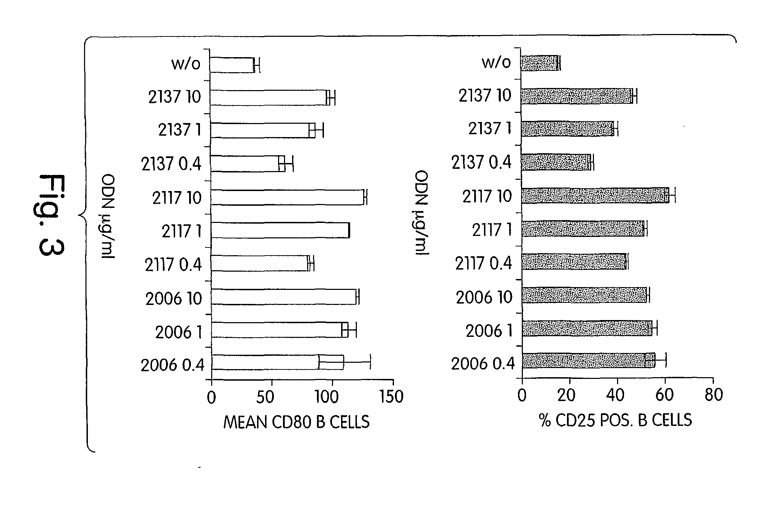

**Figure 3** is a graph depicting increased expression of CD80 (left panel) and CD25 (right panel) on human B cells following incubation of PBMC. with 0.4 µg/ml, 1.0 µg/ml or 10.0 µg/ml of ODN 2006,2117 (methylated 2006) and 2137 (2006 GpC motif). Negative controls were incubated without any ODN (w/o). The expression of B cell activation markers CD80 and CD25 on CD19+ B cells was determined by flow cytometry.

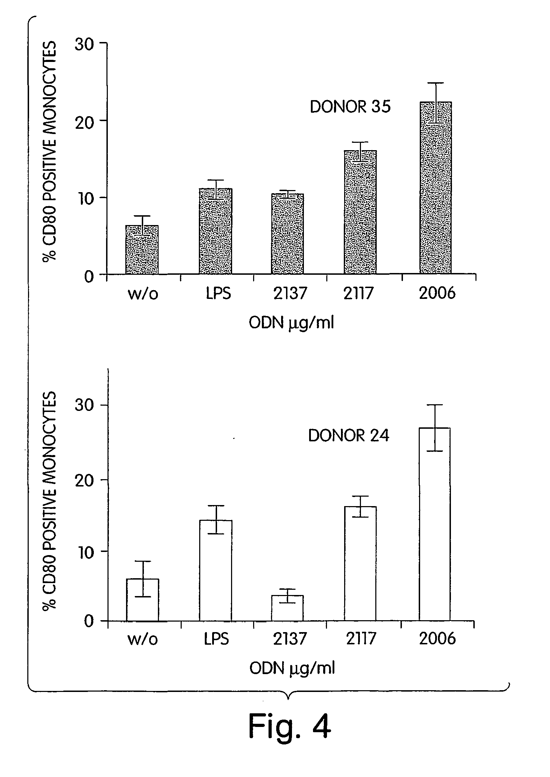

**Figure 4** is a pair of graphs depicting human peripheral blood monocyte activation by methylated ODN. PBMC (2x10⁶ cells/ml) of donor 35 (left panel) and donor 24 (right panel) were incubated with 6µg/ml ODN 2006,2117, or 2137. Positive controls were incubated with 1ng/ml LPS, and negative controls were incubated without any ODN (w/o). CD80 expression was determined on CD14+ monocytes by flow cytometry.

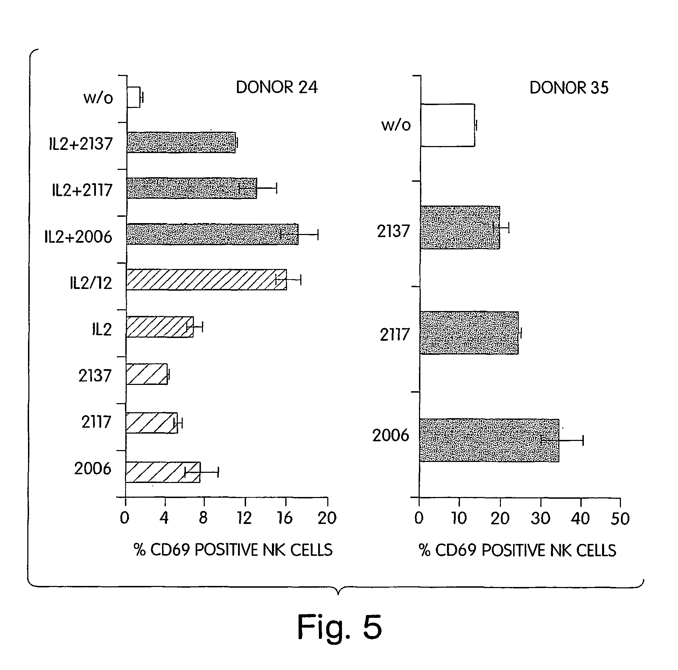

Figure 5 is a pair of graphs depicting human NK cell activation by methylated CpG ODN. PBMC (2x10⁶ cells/ml) from donors 24 (left panel) and 35 (light panel) we= incubated with 6µg/ml ODN 2006, 2117, or 2137. As a positive control (for donor 24) PBMC were incubated with 100U/ml IL-2 together with 50ng/ml IL-12. To enhance CD69 expression a submitogenic dose of IL-2 (10U/ml) was added with the ODN. Negative controls were incubated without any ODN (w/o). PBMC were stained for CD56 (N-CAM, NK cell marker), CD3 (T cell marker) and CD69 (early activation marker). Expression of CD69 in the CD56+ cell population was measured by flow cytometry.

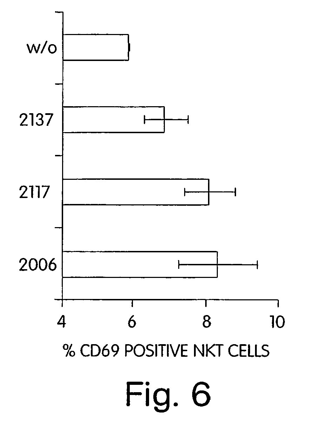

**Figure 6** is a graph depicting human natural killer T (NKT) cell activation by methylated CpG ODN. PBMC (2x10⁶ cells/ml) were incubated with 6µg/ml of ODN 2006, 2117, or 2137. Expression of CD69 on CD56+/CD3+ NKT cells was analyzed by flow cytometry.

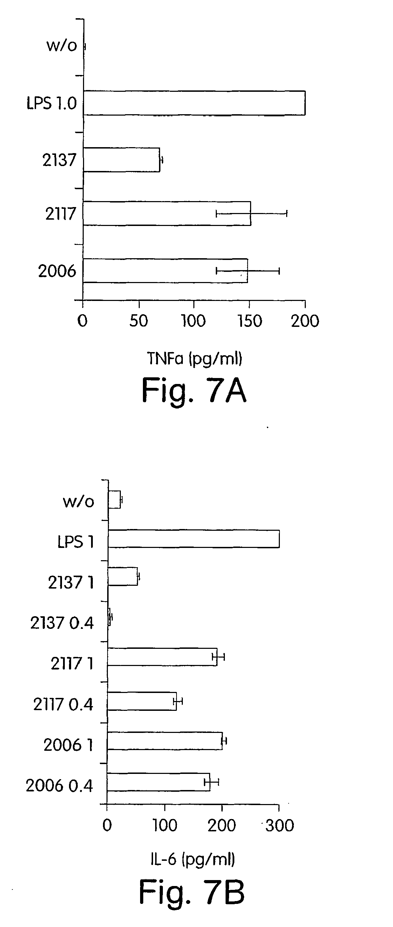

**Figure 7** is a pair of graphs depicting TNF-α and IL-6 secretion by human PBMC following cultivation with methylated and unmethylated ODN. PBMC (3x10⁶ cells/ml) were incubated with ODN 2006, 2117, or 2137. Positive controls were incubated with 1µg/ml lipopolysaccharide (LPS), and negative controls were incubated without any ODN (w/o). Cytokines present in supernatants were measured by enzyme-linked immunosorbent assay (ELISA). A. TNF-α secretion (pg/ml) after cultivation with 6µg/ml ODN. B. IL-6 secretion (pg/ml) after cultivation with 0.4 or 1.0 µg/ml ODN.

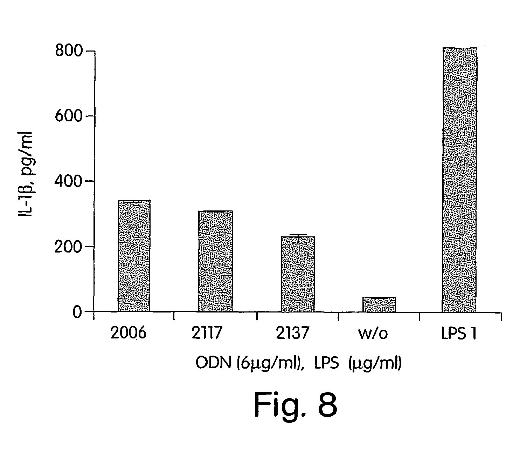

**Figure 8** is a graph depicting IL-1β secretion by human PBMC following cultivation with methylated and unmethylated ODN. PBMC (3x10⁶ cells/ml) were incubated with 6 µg/ml ODNs 2006, 2117, or 2137. Positive controls were incubated with 1µg/ml LPS, and negative controls were incubated without any ODN (w/o). IL-1β (pg/ml) present in supernatants was measured by ELISA.

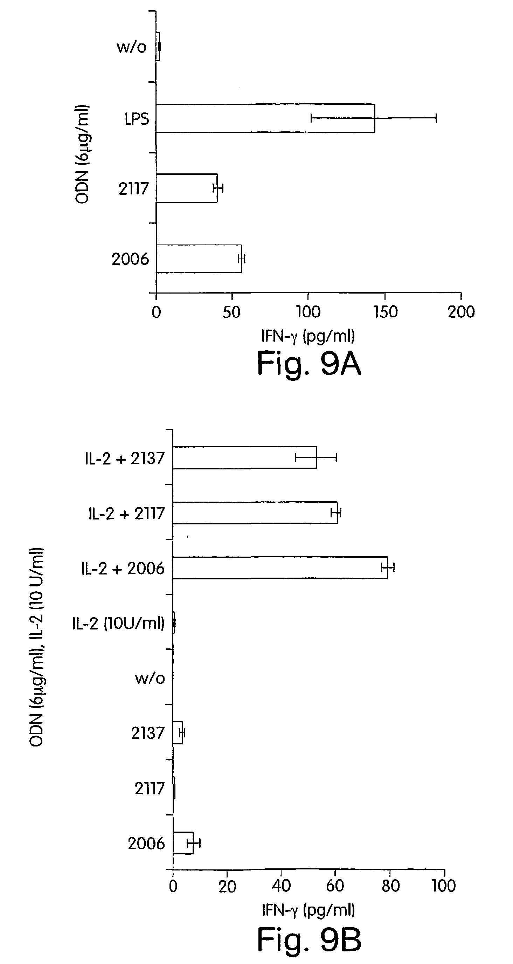

**Figure 9** is a pair of graphs depicting IFN-γ secretion by human PBMC following cultivation with methylated and unmethylated ODN. A. PBMC (5x10⁶ cells/ml) were incubated with 6 µg/ml ODNs 2006 or 2117. Positive controls were incubated with 0.1µg/ml LPS, and negative controls were incubated without any ODN (w/o). IFN-γ (pg/ml) present in supernatants was measured by ELISA. B. PBMC (5x10⁶ cells/ml) were incubated with 6 µg/ml ODNs 2006, 2117, or 2137, both with and without IL-2 (10 U/ml). Negative controls were cells incubated without any ODN (w/o) and cells incubated with IL-2 alone. IFN-γ (µg/ml) present in supernatants was measured by ELISA.

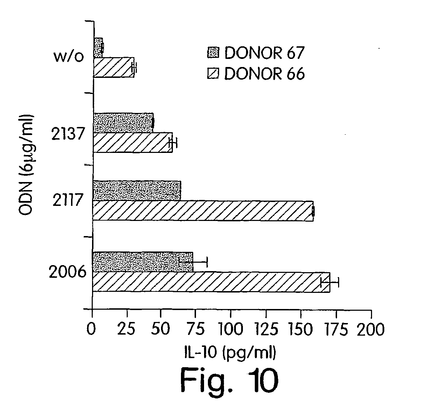

**Figure 10** is a graph depicting IL-10 secretion by human PBMC following cultivation with methylated and unmethylated ODN. PBMC (5x10⁶ cells/ml) were incubated with 6 µg/ml ODNs 2006, 2117, or 2137. Negative controls were incubated without any ODN (w/o). IL-10 (pg/ml) present in supernatants was measured by ELISA.

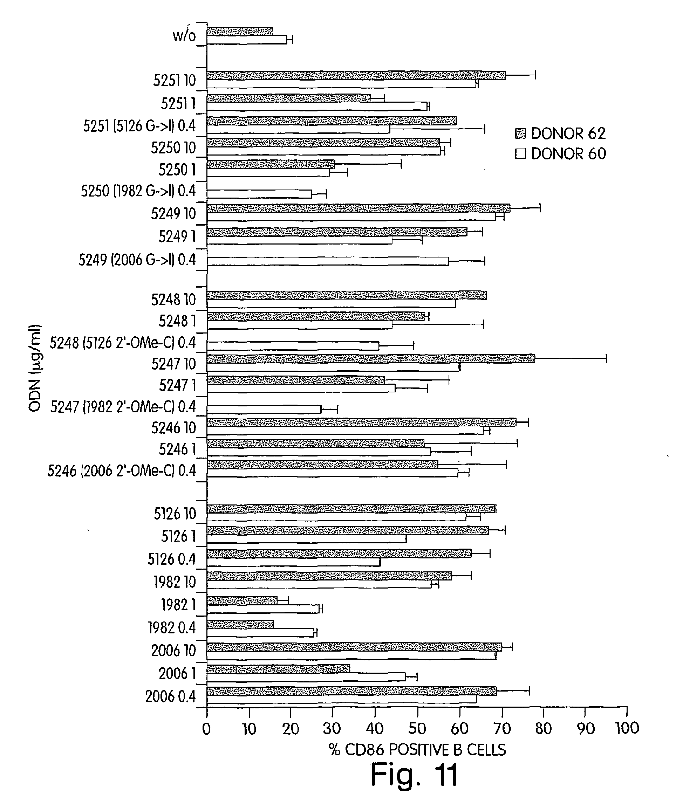

**Figure 11** is a graph depicting human B cell activation by CpI (G→I) ODN and by 2'-methoxy backbone-modified cytosine (2'-OMe-C) ODN. PBMC (2x10⁶ cells/ml) of donors 60 and 62 were incubated with 0.4 µg/ml, 1.0 µg/ml or 10.0 µg/ml of each ODN (2006, 1982, 5126, 5246, 5247, 5248, 5249, 5250 and 5251). Negative controls were incubated without any ODN (w/o). Expression of the activation marker CD86 on CD19+ B cells was measured by flow cytometry.

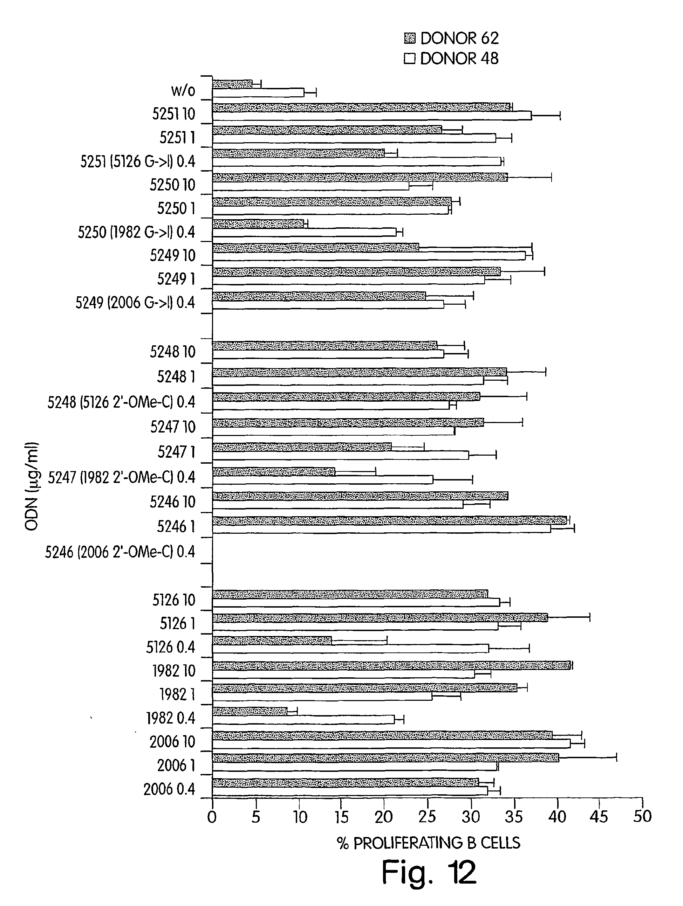

**Figure 12** is a graph depicting human B cell proliferation induced by CpI (G→I) ODN and by 2'-methoxy backbone-modified cytosine (2'-OMe-C) ODN. PBMC (2x10⁶ cells/ml) of donors 48 and 62 were stained with the dye carboxyfluorescein diacetate succinimidyl diester (CFSE) and incubated with 0.4 µg/ml, 1.0 µg/ml or 10.0 µg/ml of each ODN (2006, 1982, 5126, 5246, 5247, 5248, 5249, 5250 and 5251). Negative controls were incubated without any ODN (w/o). Proliferation of B cells was measured by flow cytometry as the percentage of CD19+ B cells with decreased brightness with the CFSE stain.

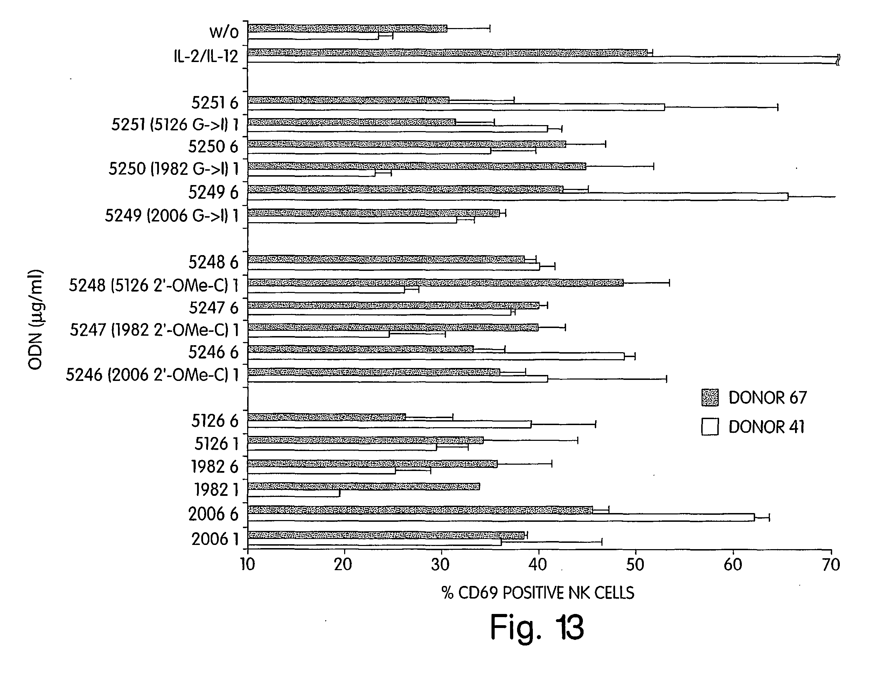

**Figure 13** is a graph depicting human NK cell activation by CpI (G→I) ODN and by 2'-methoxy backbone-modified cytosine (2'-OMe-C) ODN. PBMC (2x10⁶ cells/ml) were incubated with 1.0 µg/ml or 6.0 µg/ml of each ODN. Positive controls were incubated with IL-2 plus IL-12 (IL-2/IL-12), and negative controls were incubated without any ODN (w/o). Expression of CD69 on CD56+/CD3- NK cells was measured by flow cytometry. The IL-2/IL-12 positive control for Donor 41 activated 90% of CD69 positive NK cells.

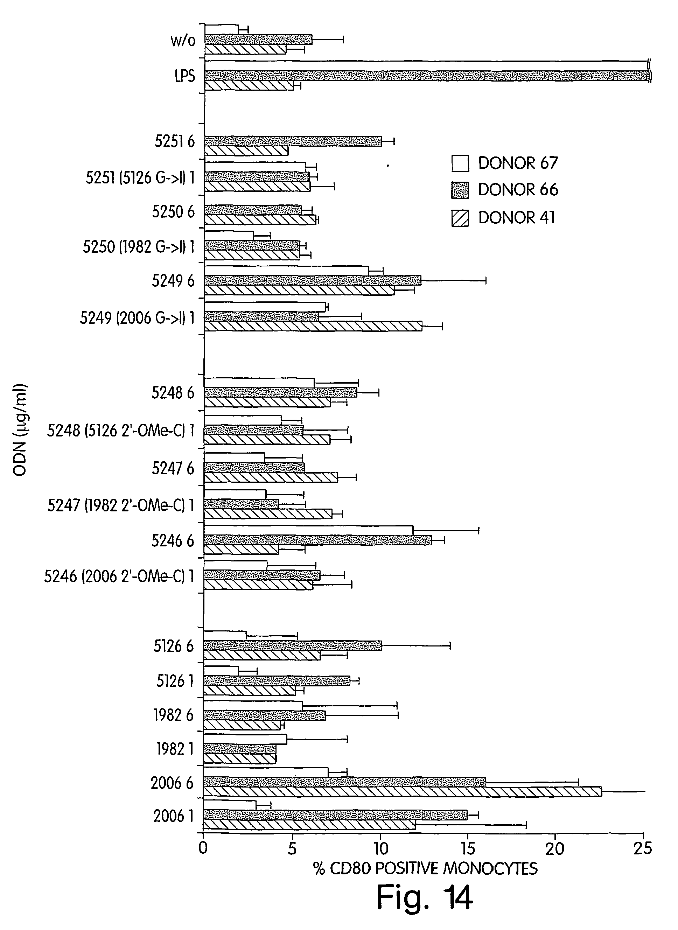

**Figure 14** is a graph depicting human peripheral blood monocyte activation by CpI (G→I) ODN and by 2'-methoxy backbone-modified cytosine (2'-OMe-C) ODN. PBMC (2x10⁶ cells/ml) from donors 67, 66, and 41 were incubated with 1.0 µg/ml and 6.0 µg/ml of each indicated ODN. Positive controls were incubated with µg/ml LPS, and negative controls were incubated without any ODN (w/o). Cells were stained for CD 14, CD 19 and CD80 to measure the expression of the cell surface marker CD80 on CD14+ monocytes excluding CD19+/CD14+ B cells by flow cytometry. LPS activated 31% and 48% of CD80+ monocytes from Donor 67 and Donor 66, respectively.

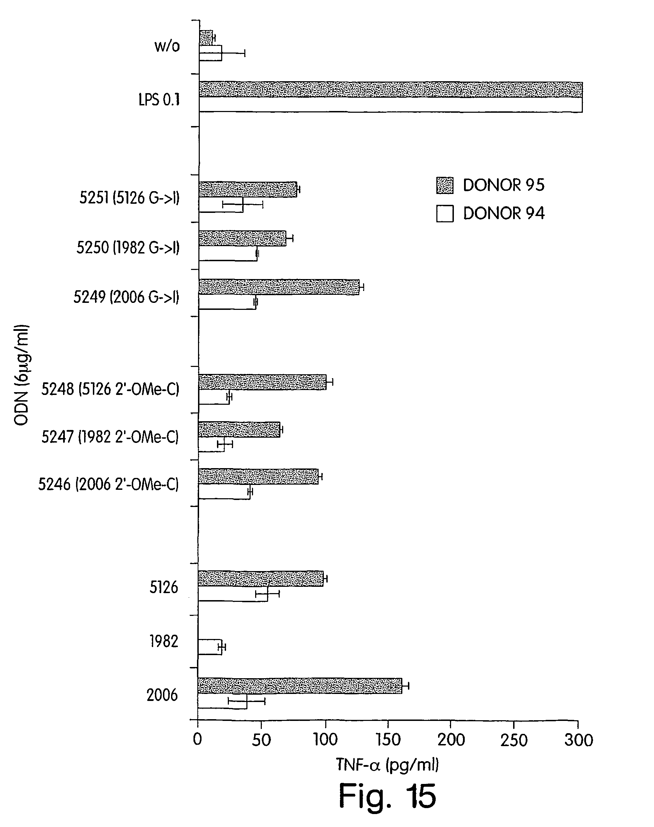

**Figure 15** is a graph depicting TNF-α secretion by human PBMC following cultivation with CpI (G→I) ODN and by 2'-methoxy backbone-modified cytosine (2'-OMe-C) ODN. PBMC (5x10⁶ cells/ml) of donors 94 and 95 were incubated with 6.0 µg/ml of the indicated ODN or LPS (0.1µg/ml). Negative controls were incubated without any ODN or LPS (w/o). TNF-α in the cell culture supernatants (pg/ml) was measured by ELISA. LPS induced 677 pg/ml and 1000 pg/ml TNF-α for PBMC from Donors 95 and 94, respectively.

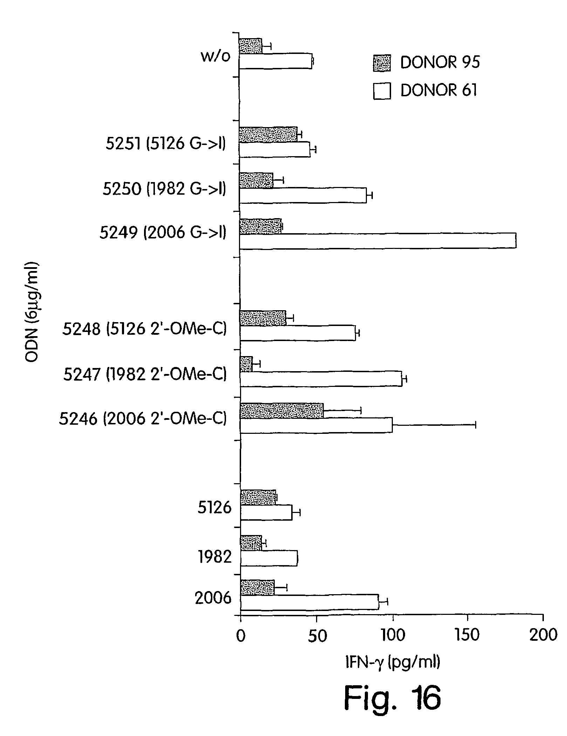

**Figure 16** is a graph depicting IFN-γ secretion by human PBMC following cultivation with CpI (G-I) ODN and by 2'-methoxy backbone-modified cytosine (2'-OMe-C) ODN. PBMC (5x10⁶ cells/ml) of donors 95 and 61 were incubated with 6.0 µg/ml of the indicated ODN. Negative controls were incubated without any ODN (w/o). IFN-γ in the cell culture supernatants (pg/ml) was measured by ELISA.

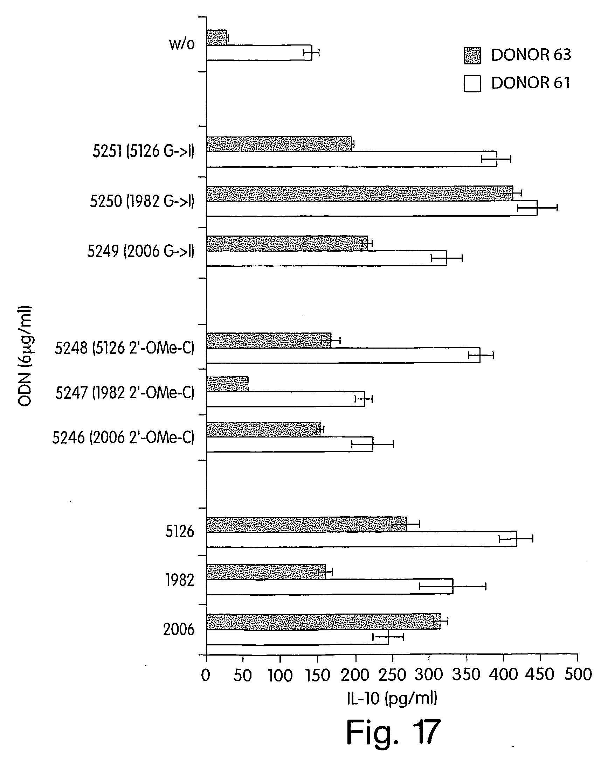

**Figure 17** is a graph depicting IL-10 secretion by human PBMC following cultivation with CpI (G→I) ODN and by 2'-methoxy backbone-modified cytosine (2'-OMe-C) ODN. PBMC (5x10⁶ cells/ml) of donors 63 and 61 were incubated with 6.0 µg/ml of the indicated ODN. Negative controls were incubated without any ODN (w/o). IL-10 in the cell culture supernatants (pg/ml) was measured by ELISA.

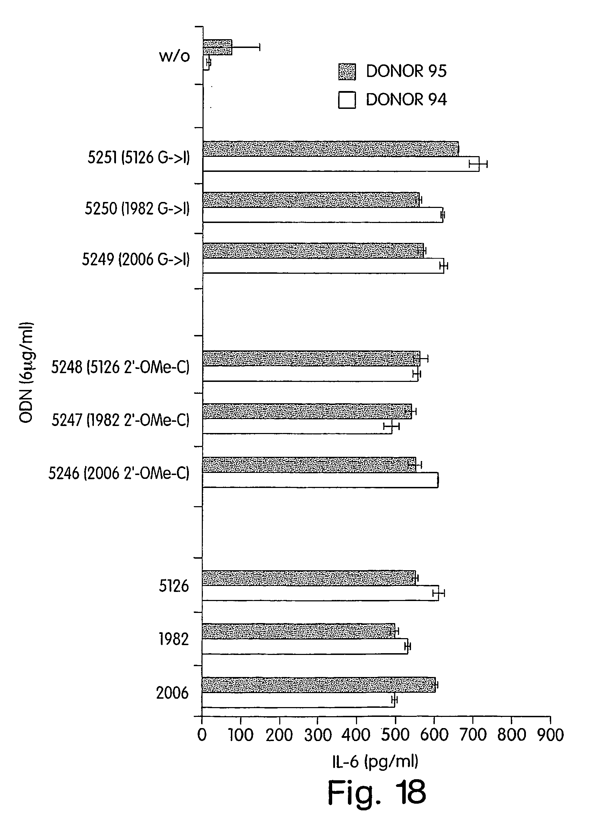

**Figure 18** is a graph depicting IL-6 secretion by human PBMC following cultivation with CpI (G→I) ODN and by 2'-methoxy backbone-modified cytosine (2'-OMe-C) ODN. PBMC (5x10⁶ cells/ml) of donors 95 and 94 were incubated with 6.0 µg/ml of the indicated ODN. Negative controls were incubated without any ODN (w/o). IL-6 in the cell culture supernatants (pg/ml) was measured by ELISA.

### DETAILED DESCRIPTION

It was surprisingly discovered according to the invention that certain CpG-like nucleic acids are immunostimulatory nucleic acids despite their lack of unmethylated CpG dinulceotides that previously had been reported to be crucial to the immunostimulatory effects of CpG nucleic acids. The CpG-like nucleic acids are useful in any application for which CpG nucleic acids are effective for inducing an activating, stimulating, or proliferative biological response.

Unmethylated CpG sequences, while relatively rare in human DNA, are commonly found in the DNA of infectious organisms such as bacteria. The human immune system has apparently evolved to recognize unmethylated CpG sequences as an early warning sign of infection and to initiate an immediate and powerful immune response against invading pathogens. Thus unmethylated CpG-containing nucleic acids, relying on this innate immune defense mechanism, can utilize a unique and natural pathway for immune therapy without causing adverse reactions frequently seen with other immune stimulatory agents. The effects of unmethylated CpG nucleic acids on immune modulation have been described extensively in published patent applications, such as PCT US95/01570; PCT/US97/19791; PCT/US98/03678; PCT/US98/10408; PCT/US98/04703; PCT/US99/07335; and PCT/US99/09863.

A "CpG nucleic acid" is a nucleic acid which includes at least one unmethylated CpG dinucleotide. A nucleic acid containing at least one unmethylated CpG dinucleotide is a nucleic acid molecule which contains an unmethylated cytosine in a cytosine-guanine dinucleotide sequence (i.e., "CpG DNA" or DNA containing a 5' cytosine followed by 3' guanine and linked by a phosphate bond) and activates the immune system. The CpG nucleic acids can be double-stranded or single-stranded. Generally, double-stranded molecules are more stable in vivo, while single-stranded molecules have increased immune activity. Thus in some aspects of the invention it is preferred that the nucleic acid be single-stranded and in other aspects it is preferred that the nucleic acid be double-stranded. The terms CpG nucleic acid or CpG oligonucleotide as used herein refer to an immunostimulatory CpG nucleic acid or immunostimulatory CpG oligonucleotide unless otherwise indicated. The entire immunostimulatory CpG nucleic acid can be unmethylated or portions may be unmethylated, but at least the C of the 5'-CG-3' dinucleotide must be unmethylated.

An "immunostimulatory nucleic acid" as used herein is any nucleic acid containing an immunostimulatory motif or backbone that induces an immune response. An immune response, as used herein, includes the stimulation of immune cells and of non-immune cells to secrete or express factors which participate in and/or characterize immune activation. This term thus includes, without limitation, stimulation of cytokine secretion by various types of cells including lymphocytes, professional antigen-presenting cells (APCs, including dendritic cells), and epithelial cells; stimulation of immunoglobulin secretion by B cells; and stimulation of cell surface molecule expression of costimulatory molecules and coreceptors on T cells, B cells, natural killer (NK) cells, monocytes, macrophages, and APCs.

An amount of an immunostimulatory nucleic acid effective to induce an immune response is an amount of an immunostimulatory nucleic acid that, when administered to a subject or contacted with cells of a subject, induces the stimulation of immune cells and/or non-immune cells to secrete or express factors which participate in and/or characterize immune activation. In certain embodiments the amount is effective for inducing cytokine secretion. Examples of cytokines are given below. Methods for determining cytokine induction and secretion are well known in the art and include enzyme-linked immunosorbent assay (ELISA), intracellular fluorescence-activated cell sorting (FACS), and bioassay. In certain other embodiments the amount is effective for stimulating NK cell activity. Methods for determining NK cell activity are well known in the art and include determination of target cell killing (cell lysis), cell surface expression of activation marker CD69 (e.g., by FACS), and secretion of interferon gamma (IFN-γ; e.g., by ELISA). Additional examples of specific cell surface markers of immune activation can include, without limitation, expression of CD11b, CD25, CD28, CD43, CD54, CD62L, CD71, CD80, CD86, CD95L, CD106, CD134, and CD134L.

The terms "nucleic acid" and "oligonucleotide" are used interchangeably to mean multiple nucleotides (i.e., molecules each comprising a sugar (e.g., ribose or deoxyribose) linked to a phosphate group and to an exchangeable organic base, which is either a substituted pyrimidine (e.g., cytosine (C), thymine (T) or uracil (U)) or a substituted purine (e.g., adenine (A) or guanine (G)). Also included are derivatives of C, including without limitation 2',3'-dideoxycitidine (ddC), 5-bromo-ddC, 3'-amino-2,3-ddC, 5-iodo-2'-deoxycytidine (dC), and derivatives of G, including without limitation 3'-azido-2',3'-dideoxyguanosine (ddG), 7-deaza-2'-deoxyguanosine (dG), 8-bromo-dG, 8-bromo-2'-dG, 06-methyl-2'-dG. As used herein, the terms "nucleic acid" and "oligonucleotide" refer to oligoribonucleotides as well as oligodeoxyribonucleotides (ODN). The terms shall also include polynucleosides (i.e., a polynucleotide minus the phosphate) and any other organic base-containing polymer. Nucleic acids include vectors, e.g., plasmids, as well as oligonucleotides. Nucleic acid molecules can be obtained from natural nucleic acid sources (e.g., genomic DNA or cDNA from prokaryotes including bacteria and from eukaryotes including yeast) and are referred to herein as "isolated," but are preferably synthetic (e.g., produced by oligonucleotide synthesis).

The invention provides in one aspect a CpG-like nucleic acid. As used herein, a "CpG-like nucleic acid" refers to an immunostimulatory nucleic acid having all the characteristics of a CpG nucleic acid as described herein, with at least one of the following exceptions: (1) the cytosine base of the C of the at least one CpG dinucleotide is 5' methylated (methylated CpG); (2) the G of the at least one CpG dinucleotide is replaced by an I (inosine; CpI nucleic acid); (3) the sugar of the C of the at least one CpG dinucleotide is modified to be a 2'-alkoxy cytosine; (4) the C of the at least one CpG dinucleotide is replaced by Z, where Z is selected from 2'-deoxyuridine (dU), 5-fluoro-2'-dU, and dSpacer, where dSpacer is a sugar moiety without a base; (5) the G of the at least one CpG dinucleotide is replaced by Y, where Y is selected from 2-aminopurine, xanthosine, N7-methyl-xanthosine, nebularine, and dSpacer, where dSpacer is a sugar moiety without a base. According to certain preferred embodiments the 2'-allcoxy cytosine is 2'-methoxy cytosine (2'-OMe-cytosine nucleic acid).

The CpG-like nucleic acids thus can incorporate certain modifications or substitutions involving either the bases or the sugar moieties of the backbone. Those with methylated CpG involve, in a preferred embodiment, methylation at the 5' position of the base cytosine. Those with CpI involve use of a particular base, inosine. Those with 2'-OMe-cytosine involve substitution of a methoxy group (OCH₃; OMe) for a hydroxy group (OH) at the 2' position of the sugar moiety linked to the base cytosine.

In some embodiments a CpG-like nucleic acid can be based on a previously known CpG nucleic acid. In other embodiments a CpG-like nucleic acid can be different from a previously known CpG nucleic acid. Also according to some preferred embodiments the CpG-like nucleic acid has a modified backbone, including a phosphate modified backbone (see below).

According to a preferred embodiment of this aspect of the invention, the CpG-like nucleic acid is at least 18 nucleotides long and is not an antisense nucleic acid. As used herein, an "antisense nucleic acid" refers to a nucleic acid sequence that (1) either has sequence complementarity to a structural gene sequence of the host, or is specifically hybridizable, under stringent conditions, with a nucleic acid sequence of the treated host, and (2) when complexed to the structural gene or nucleic acid sequence causes a loss of function of the structural gene or nucleic acid sequence. An antisense nucleic acid is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired, i.e., under physiological conditions in the case of in vivo assays or therapeutic treatment, and in the case of in vitro assays, under conditions in which the assays are performed. Mere complementarity to chromosomal DNA or messenger RNA may not define antisense, since not all sequences complementary to a particular gene or nucleic acid sequence cause a loss of function of the particular gene or nucleic add sequence. Typically an antisense nucleic acid will be able to hybridize, under stringent conditions, to a sequence in or corresponding to the coding region of a gene, including and particularly involving an intron. Alternatively, an antisense nucleic acid typically will be able to hybridize, under stringent conditions, to sequence in a regulatory element associated with a gene, e.g., a promoter or enhancer. Those skilled in the art can readily determine, without undue experimentation, whether a given CpG-like nucleic acid is an antisense nucleic acid.

As used herein, a "methylated CpG nucleic acid" refers to an immunostimulatory nucleic acid having all the characteristics of a CpG nucleic acid as described herein, with the exception that the C of the it least one CpG dinucleotide is methylated. This finding was quite unexpected. It has been widely reported that when the C of a CpG dinucleotide was replaced with a 5-methylcytosine, immunostimulatory activity was lost. Surprisingly, then, it was found that replacement of the C of a CpG dinucleotide with 5-methylcytosine did not cause this profound loss of activity. Such an immunostimulatory nucleotide acid may include at least one methylated CpG dinucleotide and at least one unmethylated CpG dinucleotide In an alternative aspect, the entire nucleic acid can be methylated, including all CpG dinucleotides present.

One aspect of this disclosure provides an immunostimulatory nucleic acid which is a methylated CpG nucleic acid represented by at least the formula:

5' X₁X₂CGX₃X₄ 3'

wherein C is methylated and X₁,X₂, X₃, and X₄ are nucleotides. In one version X₂ is adenine, guanine, cytosine, or thymine, In another version X₃ is adenine, guanine, cytosine, or thymine. In other versions X₂ is guanine, adenine, or thymine and X₃ is cytosine, adenine, or thymine.

In another version the immunostimulatory nucleic acid is an isolated methylated CpG nucleic acid represented by at least the formula:

5' N₁X₁X₂CGX₃X₄N₂ 3'

wherein C is methylated, X₁, X₂, X₃, and X₄ are nucleotides, and N₁ and N₂ are nucleic acid sequences composed of from about 0-25 nucleotides each. In one version X₁X₂ is a dinucleotide selected from the group consisting of: GpG, GpA, GpT, ApG, ApA, ApT, CpG, CpA, CpT, TpG, TpA, and TpT; and X₃X₄ is a dinucleotide selected from the group consisting of: ApG, ApA, ApT, ApC, CpG, CpA, CpC, TpG, TpA, TpT, and TpC. Preferably X₁X₂ is GpA or GpT and X₃X₄ is TpT. In other versions X₁ or X₂ or both are purines and X₃ or X₄ or both are pyrimidines or X₁X₂ is GpA and X₃ or X₄ or both are pyrimidines. In another version X₁X₂ is a dinucleotide selected from the group consisting of. TpA, ApA, ApC, ApG, and GpG. In yet another version X₃X₄ is a dinucleotide selected from the group consisting of ApG, ApA, ApC, TpG, TpA, TpT, and CpA. X₁X₂ in another version is a dinucleotide selected from the group consisting of: GpT,GpC, ApT, TpG, TpT,TpC, CpG, CpT, and CpC.

In another version the immunostimulatory nucleic acid has the sequence

5' TCNTX₁X₂CGX₃X₄ 3'

wherein the C of the CG dinucleotide is methylated, X₁, X₂, X₃, and X₄ are nucleotides, and N is a nucleic acid sequence composed of from about 0-25 nucleotides. The immunostimulatory nucleic acids of this disclosure in some version include X₁X₂ selected from the group consisting of GpG, GpA, GpT and ApA and X₃X₄ is selected from the group consisting of TpT, TpC, and CpT.

As used herein, a "CpI nucleic acid" refers to an immunostimulatory nucleic acid having all the characteristics of a CpG nucleic acid as described herem, with the exception that the G of the at least one CpG dinucleotide is replaced by an inosine (1). Inosine is a purine that represents a deaminated form of adenosine or guanosine in vertebrates. Stryer L, Biochemistry. 4th ed., WH Freeman. 1995. It was found according to this aspect of the invention that phosphorothioate ODN with inosine for guanosine exchanges are as effective as immunostimulatory nucleic acids as phosphorothioate CpG ODN. This finding was quite unexpected. Surprisingly, replacement of the G of a CpG dinucleotide with another purine, I, did not cause a loss of activity. Such an immunostimulatory nucleic acid may include at least one Cpl dinucleotide and at least one unmethylated or methylated CpG dinucleotide. In an alternative version, the immunostimulatory nucleic acid may have the same sequence as a CpG nucleic acid except that all the CpG dinucleotides are replaced by CpI dinucleotides. In a further alternative version, the immunostimulatory nucleic acid may have the same sequence as a CpG nucleic acid except that all the guanosine bases are replaced by inosine bases.

One version provides an immunostimulatory nucleic acid which is a CpI nucleic acid represented by at least the formula:

5' X₁X₂CIX₃X₄ 3'

wherein C is cytosine, I is inosine, and X₁, X₂, X₃, and X₄ are nucleotides. In certain versions the C of the CpI dinucleotide in the formula above is unmethylated. In certain other versions the C of the CpI dinucleotide in the formula above is methylated. In one version X₂ is adenine, guanine, inosine, cytosine, or thymine. In another versions X₃ is adenine, guanine, inosine, cytosine, or thymine. In other versions X₂ is guanine, adenine, or thymine and X₃ is cytosine, adenine, or thymine.

In another version the immunostimulatory nucleic acid is an isolated CpI nucleic acid represented by at least the formula:

5' N₁X₁X₂CIX₂X₃X₄N₂ 3'

wherein C is cytosine, I is inosine, X₁, X₂. X₃, and X₄ are nucleotides, and N₁ and N₂ are nucleic acid sequences composed of from about 0-25 nucleotides each. In certain versions the C of the CpI dinucleotide in the formula above is unmethylated. In one version X₁X₂ is a dinucleotide selected from the group consisting of: GpG, GpA, GpT, ApG, IpI, IpG, GpI, IpA, IpT, ApI, ApA, ApT, CpG, CpI, CpA, CpT, TpG, TpI, TpA, and TpT; and X₃X₄ is a dinucleotide selected from the group consisting of: ApG, ApI, ApA, ApT, APC, CpG, CpI, CpA, CpC, TpG, TpI, TpA, TpT, and TpC. Alternatively X₁X₂ is GpA, GpT, IpA or IpT, and X₃X₄ is TpT. In other versions X₁ or X₂ or both are purines and X₃ or X₄ or both are pyrimidines, or X₁X₂ is GpA or IpA and X₃ or X₄ or both are pyrimidines. In another version X₁X₂ is a dinucleotide selected from the group of: TpA, ApA, ApC, ApG, ApI, IPI; IpG, GpI, and GpG. In yet another version X₃X₄ is a dinucleotide selected from the group consisting of: ApG, ApI, ApA, ApC, TpG, TpI, TpA, TpT, and CpA. X₁X₂ in another version is a dinucleotide selected from the group consisting of: GpT, GpC, IpT, IpC, .ApT, TpG, TpI, TpT, TpC, CpG, CpI, CpT, and CpC.

In another version the immunostimulatory nucleic acid has the sequence

5' TCNTX₁X₂CIX₃X₄ 3'

wherein C is cytosine, I is inosine, X₁, X₂, X₃, and X₄ are nucleotides, and N is a nucleic acid sequence composed of from about 0-25 nucleotides. In certain versions the C of the CpI dinucleotide in the formula above is unmethylated. The immunostimulatory nucleic acids of this disclosure in some versions include X₁X₂ selected from the group consisting of GpG, GpA, GpT, IpI, IpA, IpT, IpG, GpI, and ApA and X₃X₄ is selected from the group consisting of TpT, TpC, and CpT.

As used herein, a "2'-alkoxy cytosine CpG nucleic acid" refers to an stimulatory nucleic acid having all the characteristics of a CpG nucleic acid as described herein, with the exception that the sugar moiety of the C of the at least one CpG dinucleotide is alkylated at the 2' position, thereby substituting for C a 2'-O-alkyl cytosine. In a preferred embodiment the sugar moiety of the C of the at least one CpG dinucleotide is methylated at the 2' position, thereby substituting for C a 2'-O-methyl cytosine (2'-OMe C). Such O-alkyl cytosine CpG nucleic acids may include at least one O-aDcyl cytosine CpG dinucleotide and at least one unmodified CpG dinucleotide. In an alternative embodiment, every cytosine of the CpG-like nucleic acid can be a 2'-alkoxy cytosine, including all CpG dinucleotides present.

The finding that the 2'-alkoxy cytosine CpG nucleic acids of the invention are immunostimulatory was quite unexpected. Previous studies have reported that replacement of specific individual or pairs of deoxynucleosides flanking a CpG dinucleotide with corresponding 2'-methoxy deoxynucleosides affects immunostimulatory potential of the olionucleotide sequence in a highly position-dependent manner. Zhao Q et aL (1999) Bioorg Med Chem Lett 9:3453-8; Zhao Q et al. (2000) Bioorg Med Chem Lett 10:1051-4. According to those reports, the closer the substituted nucleosides occur relative to the CpG dinucleotide, the weaker the immunostimulatory potential of the oligonucleotide as a whole. Surprisingly, then, it was found according to the instant invention that substitution of 2'-alkoxy cytosine for cytosine had no dramatic influence on the immunostimulatory activity of the oligonucleotide.

In certain preferred embodiments the invention provides an immunostimulatory nucleic acid which is a 2'-alkoxy cytosine CpG nucleic acid represented by at least the formula:

5' X₁X₂CGX₃X₄ 3'

wherein C is a nucleotide in which the nucleotide moiety is 2'-alkoxy cytidine and X₁, X₂, X₃, and X₄ are nucleotides. In one embodiment X₂ is adenine, guanine, cytosine, or thymine. In another embodiment X₃ is adenine, guanine, cytosine, or thymine. In other embodiments X₂ is guanine, adenine, or thymine and X₃ is cytosine, adenine, or thymine.

In other embodiments the immunostimulatory nucleic said is an isolated 2'-alkoxy cytosine CpG nucleic acid represented by at least the formula:

5' N₁X₁X₂CGX₃X₄N₂ 3'

wherein C is 2'-alkoxy cytosine, X₁, X₂, X₃, and X₄ are nucleotides, and N₁ and N₂ are nucleic acid sequences composed of from about 0-25 nucleotides each. In one embodiment X₁X₂ is a dinucleotide selected from the group consisting of: GpG, GpA, GpT, ApG, ApA, ApT, CpG, CpA, CpT, TpG, TpA, and TpT; and X₃X₄ is a dinucleotide selected from the group consisting of: ApG, ApA, ApT, ApC, CpG, CpA, CpC, TpG, TpA, TpT, and TpC. Preferably X₁X₂ is GpA or GpT and X₃X₄ is TpT. In other embodiments X₁ or X₂ or both are purines and X₃ or X₄ or both are pyrimidines or X₁X₂ is GpA and X₃ or X₄ or both are pyrimidines. In another preferred embodiment X₁X₂ is a dinucleotide selected from the group consisting of: TpA, ApA, ApC, ApG, and GpG. In yet another embodiment X₃X₄ is a dinucleotide selected from the group consisting of: ApG, ApA, ApC, TpG, TpA, TpT, and CpA. X₁X₂ in another embodiment is a dinucleotide selected from the group consisting of GpT, GpC, ApT, TpG, TpT, TpC, CpG, CpT, and CpC.

In other preferred embodiments the immunostimulatory nucleic acid has the sequence

5' TCN7X₁X₂CGX₃X₄ 3'

wherein the C of the CG dinucleotide is 2'-alkoxy cytosine, X₁, X₂, X₃, and X₄ are nucleotides, and N is a nucleic acid sequence composed of from about 0-25 nucleotides. The immunostimulatory nucleic acids of the invention in some embodiments include X₁X₂ selected from the group consisting of GpG, GpA, GpT, and ApA and X₃X₄ is selected from the group consisting of TpT, TpC, and CpT.

In yet further embodiments according to this aspect, the invention provides CpG-like nucleic acids which incorporate a combination of the types of modifications enumerated above. Thus, the invention also provides CpG-like nucleic acids incorporating a CpI dinucleotide and either a methylated C in the CpI dinucleotide or a 2'-alkoxy cytosine in the CpI dinucleotide. Likewise, the invention also embraces CpG-like nucleic acids incorporating both methylated cytosine in the CpG dinucleotide and a 2'-alkoxy cytosine in the CpI dinucleotide.

Another aspect of this disclosure provides CpG-like immunostimulatory nucleic acids having a sequence including at least the formula

5' X₁X₂ZYX₃X₄ 3'

wherein Z is selected from the group consisting of cytosine and dSpacer, Y is selected from the group consisting of inosine, 2-aminopurine, nebularine, and dSpacer; Z is not cytosine when Y is inosine; and X₁, X₂, X₃ and X₄ are nucleotide. Such CpG-like immunostimulatory nucleic acids are referred to herein as ZpY oligonucleotides

The term "dSpacer" as used herein refers to a sugar phosphate moiety that is like a nucleotide lacking a base. This entity can be incorporated into a nucleic acid backbone by 5'-to 3' type linkages that join nucleotides.

In versions where Z is cytosine, it can be methylated or unmethylated.

In some versions according to this aspect of the invention, the immunostimulatory nucleic acid has a sequence including at least the formula

5' TCNTX₁X₂ZYX₃X₄ 3'

wherein N is a nucleic acid sequence composed of from about 0-25 nucleotides.

In some versions according to this aspect of the disclosure, the immunostimulatory nucleic acid is an isolated nucleic acid.

In some embodiments the invention provides a pharmaceutical composition which includes at least an amount of the immunostimulatory nucleic acid effective to induce an immune response and a pharmaceutically acceptable carrier. The pharmaceutical composition according to this embodiment of the invention can be prepared by placing an amount of the immunostimulatory nucleic acid effective to induce an immune response in a pharmaceutically acceptable carrier. The pharmaceutically acceptable carrier can in some embodiments include a sustained-release device. Other medicaments and agents may be included in the pharmaceutical composition, including an antigen, an antibiotic (antiviral, antibacterial, antifilmangal, and antiparasitic agents), an ulcer medicament, an allergy or asthma medicament, a medicament for the treament of anemia, thrombocytopenia, or neutropenia, and a cytokine. In certain preferred embodiments which include a cytokine, the cytokine is selected from .among IL-2, IL-3, IL,4, IL-18, IFN-α, B-N-γ, TNF-α, Flt3 ligand, G-CSF, and GM-CSF.

The pharmaceutical compositions containing an immunostimulatory nucleic acid according to this aspect of the invention will in some embodiments include a plurality of immunostimultory nucleic acids having different sequences. In some embodiments the pharmaceutical composition can include a CpG nucleic acid having at least one unmethylated CpG motif.

In certain preferred embodiments the immunostimulatory nucleic acid has between 6 and 100 nucleotides, preferably between 8 and 40 nucleotides.

Also according to this aspect of the invention, in some embodiments wherein the immunostimulatory nucleic acid has a modified backbone. In certain preferred embodiments the the modified backbone is a phosphate modified backbone.

For facilitating uptake into cells, the immunostimulatory nucleic acids are preferably in the range of 6 to 100 bases in length. However, nucleic acids of any size greater than 6 nucleotides (even many kb long) are capable of inducing an immune response according to the invention if sufficient immunostimulatory motifs are present. Preferably the CpG-like nucleic acid is in the range of between 8 and 100 and in some embodiments between 8 and 40 or between 8 and 30 nucleotides in size.

The CpG-like nucleic acids can be formulated together with at least one other nucleic acid. In some instances the at least one other nucleic acid is an immunostimulatory nucleic acid, e.g., a CpG nucleic acid or another CpG-like nucleic acid. In other instances, the at least one other nucleic acid is a nucleic acid vector.

The CpG-like nucleic acids can be administered in conjunction with at least one other nucleic acid. In some instances the at least one other nucleic acid is an immunostimulatory nucleic acid, e.g., a CpG nucleic acid or another CpG-like nucleic acid. In other instances, the at least one other nucleic acid is a nucleic acid vector.

In the case when the immunostimulatory nucleic acid is administered in conjunction with a nucleic acid vector, under certain circumstances it is useful if the backbone of the immunostimulatory nucleic acid is a chimeric combination of phosphodiester and phosphorothioate (or other phosphate modification). A cell may have difficulty taking up a plasmid vector in the presence of completely phosphorothioate oligonucleotide. Thus when both a vector and an oligonucleotide are delivered to a subject, it is preferred that the oligonucleotide have a chimeric backbone, or, if the oligonucleotide has a completely phosphorothioate backbone, that the plasmid is associated with a vehicle that delivers it directly into the cell, thus avoiding the need for cellular uptake. Such vehicles are known in the art and include, for example, liposomes and gene guns.

In the case when more than one immunostimulatory nucleic acid is administered, either alone or in conjunction with a vector, the backbone of one immunostimulatory nucleic acid can be completely phosphorothioate and the backbone of another immunostimulatory nucleic acid completely phosphodiester. Thus, for example, a phosphorothioate ODN may be given together with a phosphodiester ODN.

For use in the instant invention, the immunostimulatory nucleic acids can be synthesized *de novo* using any of a number of procedures well known in the art. Such compounds are referred to as "synthetic nucleic acids." These methods of synthesis include, for example, the β-cyanoethyl phosphoramidite method (Beaucage SL and Caruthers MH (1981) Tetrahedron Lett 22:1859), and the nucleoside R-phosphonate method (Garegg et al. (1986) Tetrahedron Lett 27:4051-4054; Froehler et al. (1986) Nucl Acid Res 14:5399-5407; Garegg et al. (1986) Tetrahedron Lett 27:4055-4058; Gaffney et al. (1988) Tetrahedron Lett 29:2619-2622). These chemistries can be performed by a variety of automated oligonucleotide synthesizers available in the market. These nucleic acids are referred to as synthetic nucleic acids. Alternatively, immunostimulatory nucleic acids can be produced on a large scale in plasmids (see Sambrook et al., Molecular Cloning: A Laboratory Manual, Cold Spring Harbor Laboratory Press, New York, 1989) and separated into smaller pieces or administered whole. Nucleic acids can be prepared from natural nucleic acid sequences (e.g., genomic DNA or cDNA) using known techniques, such as those employing restriction enzymes, exonucleases or endonucleases. Nucleic acids prepared in this manner are referred to as isolated nucleic acids. The term "immunostimulatory nucleic acid" encompasses both synthetic and isolated immunostimulatory nucleic acids.

For use in vivo, nucleic acids are preferably relatively resistant to degradation (e.g., are stabilized). A "stabilized nucleic acid molecule" shall mean a nucleic acid molecule that is relatively resistant to in vivo degradation (e.g., via an exo- or endonuclease). Stabilization can be a function of length or secondary structure. Immunostimulatory nucleic acids that are tens to hundreds of kbs long are relatively resistant to in vivo degradation. For shorter immunostimulatory nucleic acids, secondary structure can stabilize and increase their effect. For example, if the 3' end of a nucleic acid has self-complementarity to an upstream region, so that it can fold back and form a sort of stem-loop structure, then the nucleic acid becomes stabilized and therefore exhibits more activity.

Alternatively, nucleic acid stabilization can be accomplished via backbone modifications. Preferred stabilized nucleic acids of the instant invention have a modified backbone. It has been demonstrated that modification of the nucleic acid backbone provides enhanced activity of the immunostimulatory nucleic acids when administered in vivo. One type of modified backbone is a phosphate backbone modification. Inclusion in immunostimulatory nucleic acids of at least two phosphorothioate linkages at or near the 5' end of the oligonucleotide and multiple (preferably five) phosphorothioate linkages at or near the 3' end, can in some circumstances provide maximal activity and protect the nucleic acid from degradation by intracellular exo- and endonucleases. Other phosphate-modified nucleic acids include phosphodiester-modified nucleic acids, combinations of phosphodiester and phosphorothioate nucleic acids, alklphosphonate and arylphosphonate, alklphosphorothioate and arylphosphorothioate, phosphorodithioate, and combinations thereof. Each of these combinations in CpG nucleic acids and their particular effects on immune cells is discussed in more detail in per Published Patent Applications PCT/US95/01570 and PCT/US97/19791. Although Applicants are not bound by the theory, it is believed that these phosphate-modified nucleic acids may show more stimulatory activity due to enhanced nuclease resistance, increased cellular uptake, increased protein binding, and/or altered intracellular localization.

Modified backbones such as phosphorothioates may be synthesized using automated techniques employing either phosphoramidate or H-phosphonate chemistries as described above. Aryl- and alkyl-phosphonates can be made, e.g., as described in U.S. Patent No. 4,469,863; and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described. Uhlmann E and Peyman A (1990) Chem Rev 90:544; Goodchild J (1990) Bioconjugate Chem 1:165.

Both phosphorothioate and phosphodiester nucleic acids containing immunostimulatory motifs are active in immune cells. However, based on the concentration needed to induce immunostimulatory nucleic acid-specific effects, the nuclease-resistant phosphorothioate backbone immunostimulatory nucleic acids are more potent. In certain in vitro assays, phosphorothioate CpG is two orders of magnitude more potent than phosphodiester CpG. Krieg AM et al. (1995) Nature 374:546-549.

Another class of backbone modifications include 2'-O-methylribonucleosides (2'-OMe). These types of substitutions are described extensively in the prior art and in particular with respect to their immunostimulating properties in Zhao Q et al. (1999) Bioorg Med Chem Lett 9:3453-8. Zhao et al. describes methods of preparing 2'-OMe modifications to nucleic acids.

The nucleic acid molecules of the invention may include naturally occurring or synthetic purine or pyrimidine heterocyclic bases as well as modified backbones. Purine or pyrimidine heterocyclic bases include, but are not limited to, adenine, guanine, cytosine, thymine, uracil, and inosine. Other representative heterocyclic bases are disclosed in U.S. Patent No. 3,687,808, issued to Merigan, et al. The term purine or pyrimidine or bases are used herein to refer to both naturally occurring and synthetic purines, pyrimidines or bases.

Other stabilized nucleic acids include: nonionic DNA analogs, such as alkyl- and aryl-phosphates (in which the charged phosphonate oxygen is replaced by an alkyl or aryl group), alkylphosphodiester and alkylphosphotriesters, in which the charged oxygen moiety is alkylated. Nucleic acids which contain diol, such as tetraethyleneglycol or hexaethyleneglycol, at either or both termini have also been shown to be substantially resistant to nuclease degradation.

According to further aspects of the invention, the CpG-like nucleic acids are useful for treating a subject that has or is at risk of developing certain diseases, including an infectious disease due to an infection with an infectious agent, an allergy or asthma where the allergen or predisposition to asthma is known or suspected, or a cancer in which a specific cancer antigen has been identified. The CpG-like nucleic acids can also be given without the antigen or allergen for shorter term protection against infection, cancer, allergy or asthma, and in this case repeated doses will allow longer-term protection.

The term "treating" is defined as administering to a subject a therapeutically effective amount of a compound (e.g., an oligonucleotide of the invention) that is sufficient to prevent the onset of, alleviate the symptoms of, or stop the progression of a disorder or disease being treated.

A "subject" according to the invention shall mean a human or other vertebrate animal including but not limited to a dog, cat, horse, cow, pig, sheep, goat, rabbit, guinea pig, rat, mouse, non-human primate (e.g., monkey), chicken, and fish (aquaculture species, e.g., salmon).

A "subject at risk" as used herein is a subject that has a predisposition for having a disease or that has any identified or suspected risk of exposure to an agent or condition associated with the development of a disease. Generally, the risk of developing a particular disease will be higher for the subject at risk than for other individuals who are considered not to be at risk.

With reference to an infectious disease, a "subject at risk of developing an infectious disease" as used herein is a subject that has any identified or suspected risk of exposure to an infection-causing pathogen. For instance, a subject at risk of developing an infection may be a subject that is living in or planning to travel to an area where a particular type of infectious agent is found. A subject at risk may be a subject that through lifestyle, occupation, or a medical procedure is potentially exposed to bodily fluids or materials which may contain infectious organisms or agents. Alternatively, a subject at risk may be a subject that through lifestyle, occupation, or medical procedures is exposed to infectious organisms or agents. A subject at risk of developing infection also may be a subject at risk of biowarfare, e.g., military personnel or those living in areas at risk of terrorist attack. Subjects at risk of developing infection also include general populations to which a medical agency recommends vaccination against a particular infectious organism or agent.

With reference to allergy and asthma, a subject at risk as used herein is a subject that has any identified or suspected risk of exposure to an allergy- or asthma-inducing antigen. For instance, a subject at risk of developing allergy or asthma may be a subject that is living in or planning to travel to an area where a particular type of antigen or allergen is found. A subject at risk of developing allergy or asthma may be a subject that through lifestyle or employment activities is exposed directly to the antigen or allergen. Subjects at risk of developing allergy and asthma also include general populations to which a medical agency recommends vaccination with a particular antigen. If the antigen is an allergen and the subject develops seasonal allergic responses to that particular antigen, e.g., during pollen season, then the subject may be at risk of developing allergy during the season of exposure to the antigen. A subject at risk of developing an allergy or asthma includes those subjects that have been identified as having an allergy or asthma but that don't have the active disease during the CpG-like nucleic acid treatment, as well as subjects that are considered to be at risk of developing these diseases because of genetic or environmental factors.

With reference to cancer, a "subject at risk of developing a cancer" is one that has an increased probability of having cancer. These subjects include, for instance, subjects having a genetic abnormality, the presence of which has been demonstrated to have a correlative relation to a higher likelihood of having a cancer, as well as subjects exposed to cancer-causing agents such as tobacco, asbestos, or other chemical toxins, or subjects that have previously been treated for cancer and are in apparent remission. When a subject at risk of developing a cancer is treated with an antigen specific for the type of cancer to which the subject is at risk of developing and a CpG-like nucleic acid, the subject may be able to kill the cancer cells as they develop. If a tumor begins to form in the subject, the subject will develop a specific immune response against the tumor antigen.

In addition to the use of the CpG-like nucleic acids for prophylactic treatment, the invention also encompasses the use of the CpG-like nucleic acids for the treatment of a subject having an infection, an allergy, asthma, or a cancer.

An infectious disease, as used herein, is a disease arising from the presence of a foreign microorganism or infectious pathogen in the body. A "subject that has an infectious disease" is a subject that has been exposed to an infectious pathogen and has acute or chronic detectable levels of the pathogen in the body. Infectious pathogens include viruses, bacteria, fungi, parasites, and other infectious agents. The CpG-like nucleic acids can be used with an antigen to mount an antigen-specific systemic or mucosal immune response that is capable of reducing the level of or eradicating the infectious pathogen.

A subject that has an allergy is a subject that has or is at risk of developing an allergic reaction in response to an allergen. An allergy refers to acquired hypersensitivity to a substance (allergen). Allergic conditions include but are not limited to eczema, urticaria (hives), allergic asthma, allergic rhinitis or coryza, hay fever, conjunctivitis, food allergies, and other atopic conditions.

Currently, allergic diseases are generally treated either symptomatically (see below) or definitively, the latter by the injection of small doses of antigen followed by subsequent increasing dosage of antigen. It is believed that this procedure induces tolerization to the allergen to prevent further allergic reactions. These methods, however, can take several years to be effective and are associated with the risk of side effects such as anaphylactic shock. The methods of the invention avoid these problems.

Allergies are generally characterized by IgE antibody generation against harmless allergens. The cytokines that are induced by systemic or mucosal administration of CpG-like nucleic acids are predominantly of a class called Th1 (examples are IL-12 and IFN-γ) and these induce both humoral (antibody) and cellular immune responses. The types of antibodies associated with a Th1 response are generally more protective because they have high neutralization and opsonization capabilities. The other major type of immune response, which is termed a Th2 immune response, is associated with the production of IL-4, IL-5 and IL-10 cytokines. Th2 responses involve predominately antibodies and these have less protective effect against infection and some Th2 isotypes (e.g., IgE) are associated with allergy. In general, it appears that allergic diseases are mediated by Th2 type immune responses while Th1 responses provide the best protection against infection, although excessive Th1 responses are associated with autoimmune disease. Based on the ability of the CpG-like nucleic acids to shift the immune response in a subject from a Th2 response (which is associated with production of IgE antibodies and allergy) to a Th1 response (which is protective against allergic reactions), an effective dose for inducing an immune response of a CpG-like nucleic acid can be administered to a subject to treat or prevent an allergy.

Thus, the CpG-like nucleic acids have significant therapeutic utility in the treatment of allergic and non-allergic conditions such as asthma. Th2 cytokines, especially IL-4 and IL-5, are elevated in the airways of asthmatic subjects. These cytokines promote important aspects of the asthmatic inflammatory response, including IgE isotope switching, eosinophil chemotaxis and activation, and mast cell degranulation. Th1 cytokines, especially IFN-γ and IL-12, can suppress the formation of Th2 clones and production of Th2 cytokines.

A subject that has asthma is a subject having a disorder of the respiratory system characterized by inflammation, narrowing of the airways, and increased reactivity of the airways to inhaled agents. Typically asthma is a chronic disease characterized by acute episodes of reversible, generalized airway narrowing. Thus a subject that has asthma need not require the subject currently be symptomatic, i.e., currently have an acute exacerbation of asthma. Methods of diagnosing asthma are well known in the art, and these can include measurement of reversible airway obstruction in response to challenge with a beta-adrenergic agonist, histamine, methacholine, or cold air. Asthma is frequently, although not exclusively associated with atopic or allergic symptoms.

A subject that has a cancer is a subject that has detectable cancerous cells. The cancer may be a malignant or non-malignant cancer. Cancers or tumors include but are not limited to biliary tract cancer; brain cancer; breast cancer; cervical cancer; choriocarcinoma; colon cancer; endometrial cancer; esophageal cancer; gastric cancer; intraepithelial neoplasms; lymphomas; liver cancer; lung cancer (e.g., small cell and non-small cell); melanoma; neuroblastoma; oral cancer; ovarian cancer; pancreas cancer; prostate cancer; rectal cancer; sarcomas; skin cancer; testicular cancer; thyroid cancer; and renal cancer, as well as other carcinomas and sarcomas. In one embodiment the cancer is hairy cell leukemia, chronic myelogenous leukemia, cutaneous T-cell leukemia, multiple myeloma, follicular lymphoma, malignant melanoma, squamous cell carcinoma, renal cell carcinoma, prostate carcinoma, bladder cell carcinoma, or colon carcinoma.

In some embodiments the invention can be used to treat cancer and tumors in non-human subjects. Cancer is one of the leading causes of death in companion animals (e.g., cats and dogs). Cancer usually strikes older animals which, in the case of house pets, have become integrated into the family. Forty-five percent of dogs older than 10 years of age are likely to succumb to cancer. The most common treatment options include surgery, chemotherapy and radiation therapy. Other treatment modalities which have been used with some success are laser therapy, cryotherapy, hyperthermia and immunotherapy. The choice of treatment depends on type of cancer and degree of dissemination. Unless the malignant growth is confined to a discrete area in the body, it is difficult to remove only malignant tissue without also affecting normal cells.

Malignant disorders commonly diagnosed in dogs and cats include but are not limited to adenosquamous carcinoma, brain tumor, bronchial gland tumor, bronchiolar adenocarcinoma, Burkitt's lymphoma, carcinoid lung tumor, Ewing's sarcoma, fibroma, fibrosarcoma, lymphosarcoma, mammary tumors, mastocytoma, melanoma, microglioma, myxochondroma, neuroblastoma, neurosarcoma, oral neoplasia, osteoclastoma, osteoma, osteosarcoma, papilloma, pulmonary sarcoma, retinoblastoma, rhabdomyosarcoma, and Wilms' tumor. Other neoplasias in dogs include adrenal gland carcinoma, basal cell tumor, chloroma (granulocytic sarcoma), corneal papilloma, corneal squamous cell carcinoma, cystadenoma, genital squamous cell carcinoma, hemangioendothelioma, hemangiopericytoma, hemangiosarcoma, histiocytoma, oral papillomatosis, pleural mesothelioma, seminoma, Sertoli cell tumor, stomach tumor, testicular tumor, thymoma, and transmissible venereal tumor. Additional malignancies diagnosed in cats include fibrosarcoma, follicular lymphoma, intestinal lymphosarcoma, and pulmonary squamous cell carcinoma. The ferret, an ever-more popular house pet, is known to develop gastric adenocarcinoma, gastric MALT lymphoma, insulinoma, lymphoma, neuroma, pancreatic islet cell tumor, and sarcoma.

Neoplasms affecting agricultural livestock include leukemia, hemangiopericytoma and bovine ocular neoplasia (in cattle); preputial fibrosarcoma, ulcerative squamous cell carcinoma, preputial carcinoma, connective tissue neoplasia and mastocytoma (in horses); hepatocellular carcinoma (in swine); lymphoma and pulmonary adenomatosis (in sheep); pulmonary sarcoma, lymphoma, Rous sarcoma, reticulendotheliosis, fibrosarcoma, nephroblastoma, B-cell lymphoma and lymphoid leukosis (in avian species); retinoblastoma, hepatic neoplasia, lymphosarcoma (lymphoblastic lymphoma), plasmacytoid leukemia and swimbladder sarcoma (in fish), caseous lumphadenitis (CLA): chronic, infectious, contagious disease of sheep and goats caused by the bacterium *Corynebacterium pseudotuberculosis,* and contagious lung tumor of sheep caused by jaagsiekte.

The CpG-like nucleic acids may also be formulated with or administered to a subject in conjunction with an antigen. An "antigen" as used herein is a molecule capable of provoking an immune response. Antigens include but are not limited to cells, cell extracts, proteins, polypeptides, peptides, polysaccharides, polysaccharide conjugates, peptide and non-peptide mimics of polysaccharides and other molecules, small molecules, lipids, glycolipids, carbohydrates, viruses and viral extracts and muticellular organisms such as parasites and allergens. The term antigen broadly includes any type of molecule which is recognized by a host immune system as being foreign. Antigens include but are not limited to cancer antigens, microbial antigens, and allergens.

The subject is exposed to the antigen. As used herein with reference to an antigen, the term "exposed to" refers to either the active step of contacting the subject with an antigen or the passive exposure of the subject to the antigen in vivo. Methods for the active exposure of a subject to an antigen are well-known in the art. In general, an antigen is administered directly to the subject by any means such as intravenous, intramuscular, oral, transdermal, mucosal, intranasal, intratracheal, or subcutaneous administration. The antigen can be administered systemically or locally. Methods for administering the antigen and the CpG-like nucleic acid are described in more detail below. A subject is passively exposed to an antigen if an antigen becomes available for exposure to the immune cells in the body. A subject may be passively exposed to an antigen, for instance, by entry of a foreign pathogen into the body or by the development of a tumor cell expressing a foreign antigen on its surface.

The methods in which a subject is passively exposed to an antigen can be particularly dependent on timing of administration of the CpG-like nucleic acid. For instance, in a subject at risk of developing an infectious disease or an allergic or asthmatic response, or a cancer, the subject may be administered the CpG-like nucleic acid on a regular basis when that risk is greatest, i.e., during allergy season or after exposure to a cancer-causing agent. Additionally the CpG-like nucleic acid may be administered to travelers before they travel to foreign lands where they are at risk of exposure to infectious agents. Likewise the CpG-like nucleic acid may be administered to soldiers or civilians at risk of exposure to biowarfare to induce a systemic or mucosal immune response to the antigen when and if the subject is exposed to it.

As used herein, the terms "cancer antigen" and "tumor antigen" are used interchangeably to refer to antigens which are differentially expressed by cancer cells and can thereby be exploited in order to target cancer cells. Cancer antigens are antigens which can potentially stimulate apparently tumor-specific immune responses. Some of these antigens are encoded, although not necessarily expressed, by normal cells. These antigens can be characterized as those which are normally silent (i.e., not expressed) in normal cells, those that are expressed only at certain stages of differentiation, and those that are temporally expressed such as embryonic and fetal antigens. Other cancer antigens are encoded by mutant cellular genes, such as oncogenes (e.g., activated ras oncogene), suppressor genes (e.g., mutant p53), and fusion proteins resulting from internal deletions or chromosomal translocations. Still other cancer antigens can be encoded by viral genes such as those carried on RNA and DNA tumor viruses.

A cancer antigen as used herein is a compound, such as a peptide or protein, associated with a tumor or cancer cell and which is capable of provoking an immune response when expressed on the surface of a cancer cell in the context of an MHC molecule. Preferably, the antigen is expressed at the cell surface of the cancer cell. Even more preferably, the antigen is one which is not expressed by normal cells, or at least not expressed to the same level as in cancer cells. Cancer antigens can be prepared from cancer cells either by preparing crude extracts of cancer cells, for example, as described in Cohen PA et al. (1994) Cancer Research 54:1055-8, by partially purifying the antigens, by recombinant technology, or by de novo synthesis of known antigens. Cancer antigens can be used in the form of immunogenic portions of a particular antigen or in some instances a whole cell or a tumor mass can be used as the antigen. Cancer antigens include but are not limited to antigens that are recombinantly expressed, an immunogenic portion of a tumor or cancer cell, or a whole tumor or cancer.

A microbial antigen as used herein is an antigen of a microorganism and includes but is not limited to antigens associated with viruses, bacteria, fungi, and parasites. Such antigens include the intact microorganism as well as natural isolates and fragments or derivatives thereof and also synthetic compounds which are identical to or similar to natural microorganism antigens and induce an immune response specific for that microorganism. A compound is similar to a natural microorganism antigen if it induces an immune response (humoral and/or cellular) to a natural microorganism antigen. Such antigens are used routinely in the art and are well known to those of ordinary skill in the art.

Examples of infectious viruses include but are not limited to: *Adenoviridae* (most adenoviruses); Arena viridae (hemorrhagic fever viruses); *Birnaviridae; Hepadnaviridae* (Hepatitis B virus); *Bungaviridae* (e.g., Hantaan viruses, bunga viruses, phleboviruses and Nairo viruses); *Calciviridae* (e.g., strains that cause gastroenteritis); *Coronaviridae* (e.g., coronaviruses); *Filoviridae* (e.g., ebola viruses); *Flaviridae* (e.g., dengue viruses, encephalitis viruses, yellow fever viruses); *Herpesviridae* (herpes simplex virus (HSV) 1 and 2, varicella zoster virus, cytomegalovirus (CMV), herpes virus; *Iridoviridae* (e.g., African swine fever virus); *Orthomyxoviridae* (e.g., influenza viruses); *Paramyxoviridae* (e.g., parainfluenza viruses, mumps virus, measles virus, respiratory syncytial virus); *Parvovirida* (parvoviruses); *Papovaviridae* (papilloma viruses, polyoma viruses); *Piconzaviridae* (e.g., polio viruses, hepatitis A virus; enteroviruses, human Coxsackie viruses, rhinoviruses, echoviruses); *Poxviridae* (variola viruses, vaccinia viruses, pox viruses); *Reoviridae* (e.g., reoviruses, orbiviurses and rotaviruses); *Retroviridae* (e.g., human immunodeficiency viruses, such as HIV-1 (also referred to as HTLV-III, LAV or HTLV-III/LAV, or HIV-III, and other isolates, such as HIV-LP; *Rhabdoviridae* (e.g., vesicular stomatitis viruses, rabies viruses); *Togaviridae* (e.g., equine encephalitis viruses, rubella viruses); and unclassified viruses (e.g., the etiological agents of spongiform encephalopathies, the agent of delta hepatitis (thought to be a defective satellite of hepatitis B virus), the agents of non-A, non-B hepatitis (class 1 = internally transmitted; class 2 = parenterally transmitted (i.e. Hepatitis C); Norwalk and related viruses, and astroviruses).

Examples of infectious, bacteria include but are not limited to: *Acinetobacter* spp., *Actinomyces israelli, Bacillus anthracis, Bacteroides* spp., *Bordetella pertussis, Borrelia burgdorferi, Brucella melitensis, pathogenic Campylobacter* spp., *Clostridium difficile; Clostridium perfringens, Clostridium tetani, Corynebacterium diphtheriae,* other *Corynebacterium* spp., *Enterobacter aerogenes, Enterococcus* spp.*, Erysipelothrix rhusiopathiae, Escherichia coli, Francisella tularensis, Fusobacterium nucleatum, Haemophihus influenzae, Helicobacter pylori, Klebsiella pneumoniae, Legionella pneumophilia, Leptospira* spp., *Listeria monocytogenes, Mycobacteria* spp. (e.g., *M. tuberculosis, M. avium, M. gordonae, M. intracellulare, and M. kansasii*), *Neisseria gonorrhoeae, Neisseria Meningitidis, Nocardia asteroides, Nocardia brasiliensis, Pasturella multocida, Peptostreptococcus* spp., *Proteus* spp., *Pseudomonas aeruginosa,* other *Pseudomonas* spp., *Rickettsia, Salmonella* spp., *Serratia* spp., *Shigella* spp., *Staphylococcus aureus, Streptobacillus moniliformis, Streptococcus* (anaerobic spp.), *Streptococcus* (viridans group); *Streptococcus agalactiae* (Group B Streptococcus), *Streptococcus bovis, Streptococcus faecalis, Streptococcus pneumoniae, Streptococcus pyogenes* (Group A Streptococcus), *Treponema pallidum, Treponema pertenue, Vibrio cholerae,* other *Vibrio* spp., and *Yersinia* spp.

Examples of infectious fungi include but are not limited to: *Aspergillus* spp., *Blastomyces dermatitidis, Candida albicans,* other *Candida* spp., *Coccidioides immitis, Cryptococcus neoformans, Histoplasma capsulatum* and *Rhizopus* spp.

Other infectious organisms include but are not limited to: *Babesia divergens, Babesia microti, Chlamydia trachomatis, Giardia* spp., *Leishmania braziliensis, Leishmania donovani, Leishmania major, Leishmania tropica, Plasmodium* spp. (e.g., *Plasmodium falciparum, Plasmodium knowlesi, Plasmodium malariae, Plasmodium ovale,* and *Plasmodium vivax), Toxoplasma gondii, Trichinella spiralis, Trypanosoma cruzi, Trypanosoma gambiense,* and *Trypanasoma rhodesiense.*

Other medically relevant microorganisms have been described extensively in the literature, e.g., see C.G.A Thomas, Medical Microbiology, Bailliere Tindall, Great Britain 1983.

Although many of the microbial antigens described above relate to human disorders, the invention is also useful for treating non-human vertebrates. Non-human vertebrates are also capable of developing infections which can be prevented or treated with the CpG-h-ke nucleic acids disclosed herein. For instance, in addition to the treatment of infectious human diseases, the methods of the invention are useful for treating infections of animals.

Many vaccines for the treatment of non-human vertebrates are disclosed in Bennett, K., Compendium of Veterinary Products, 3rd ed., North American Compendiums, Inc., 1995. As discussed above, antigens include infectious microbes such as viruses, bacteria, parasites, fungi, and fragments thereof, derived from natural sources or synthetically.

Infectious viruses of both human and non-human vertebrates, include retroviruses, RNA viruses and DNA viruses. The group of retroviruses includes both simple retroviruses and complex retroviruses. The simple retroviruses include the subgroups ofB-type retroviruses, C-type retroviruses and D-type retroviruses. An example of a B-type retrovirus is mouse mammary tumor virus (MMTV). The C-type retroviruses include subgroups C-type group A (including Rous sarcoma virus (RSV), avian leukemia virus (ALV), and avian myeloblastosis virus (AMV)) and C-type group B (including feline leukemia virus (FeLV), gibbon ape leukemia virus (GALV), spleen necrosis virus (SNV), reticuloendotheliosis virus (RV) and simian sarcoma virus (SSV)). The D-type retroviruses include Mason-Pfizer monkey virus (MPMV) and simian retrovirus type 1 (SRV-1). The complex retroviruses include the subgroups of lentiviruses, T-cell leukemia viruses and the foamy viruses. Lentiviruses include HIV-1, HIV-2, SIV, Visna virus, feline immunodeficiency virus (FIV), and equine infectious anemia virus (EIAV). The T-cell leukemia viruses include HTLV-1, HTLV-II, simian T-cell leukemia virus (STLV), and bovine leukemia virus (BLV). The foamy viruses include human foamy virus (HFV), simian foamy virus (SFV) and bovine foamy virus (BFV).

Examples of other RNA viruses that are antigens in vertebrate animals include, but are not limited to, members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, simian rotavirus, bovine or ovine rotavirus, avian rotavirus); the family Picomaviridae, including the genus Enterovirus (poliovirus, Coxsackie virus A and B, enteric cytopathic human orphan (ECHO) viruses, hepatitis A virus, Simian enteroviruses, Murine encephalomyelitis (ME) viruses, Poliovirus muris, Bovine enteroviruses, Porcine enteroviruses, the genus Cardiovirus (Encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus); the family Rhabdoviridae, including the genus Vesiculovirus (VSV), Chandipura virus, Flanders-Hart Park virus), the genus Lyssavirus (Rabies virus), fish Rhabdoviruses, and two probable Rhabdoviruses (Marburg virus and Ebola virus); the family Arenaviridae, including Lymphocytic choriomeningitis virus (LCM), Tacaribe virus complex, and Lassa virus; the family Coronoaviridae, including Infectious Bronchitis Virus (IBV), Hepatitis virus, Human enteric corona virus, and Feline infectious peritonitis (Feline coronavirus).

Illustrative DNA viruses that are antigens in vertebrate animals include, but are not limited to, the family Poxviridae, including the genus Orthopoxvirus (Variola major, Variola minor, Monkey pox Vaccinia, Cowpox, Buffalopox, Rabbitpox, Ectromelia), the genus Leporipoxvirus (Myxoma, Fibroma), the genus Avipoxvirus (Fowlpox, other avian poxvirus), the genus Capripoxvirus (sheeppox, goatpox), the genus Suipoxvirus (Swinepox), the genus Parapoxvirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the alpha-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesviruses (Human cytomegalovirus and cytomegaloviruses of swine and monkeys); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A,B,C,D,E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV-40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc). Finally, DNA viruses may include viruses which do not fit into the above families such as Kuru and Creutzfeldt-Jacob disease viruses and chronic infectious neuropathic agents (CHINA virus).

Each of the foregoing lists is illustrative, and is not intended to be limiting. In addition to the use of the CpG-like nucleic acids to induce an antigen-specific immune response in humans, the methods of the preferred embodiments are particularly well suited for treatment of birds such as hens, chickens, turkeys, ducks, geese, quail, and pheasant. Birds are prime targets for many types of infections.

Hatchling birds are exposed to pathogenic microorganisms shortly after birth. Although these birds are initially protected against pathogens by maternal derived antibodies, this protection is only temporary, and the bird's own immature immune system must begin to protect the bird against the pathogens. It is often desirable to prevent infection in young birds when they are most susceptible. It is also desirable to prevent infection in older birds, especially when the birds are housed in close quarters, leading to the rapid spread of disease. Thus, it is desirable to administer the CpG-like nucleic acid of the invention to birds to enhance an antigen-specific immune response when antigen is present.

An example of a common viral infection in chickens is chicken infectious anemia virus (CIAV). CIAV was first isolated in Japan in 1979 during an investigation of a Marek's disease vaccination break. Yuasa et al. (1979) Avian Dis 23:366-385. Since that time, CIAV has been detected in commercial poultry in all major poultry producing countries (van Bulow et al., in Diseases of Poultry, 9th edition, Iowa State University Press, 1991, pp.690-699).

CIAV infection results in a clinical disease characterized by anemia, hemorrhage and immunosuppression, in young susceptible chickens. Atrophy of the thymus and of the bone marrow and consistent lesions of CIAV-infected chickens are also characteristic of CIAV infection. Lymphocyte depletion in the thymus, and occasionally in the bursa of Fabricius, results in immunosuppression and increased susceptibility to secondary viral, bacterial, or fungal infections which then complicate the course of the disease. The immunosuppression may cause aggravated disease after infection with one or more of Marek's disease virus (MDV), infectious bursal disease virus, reticuloendotheliosis virus, adenovirus, or reovirus. It has been reported that pathogenesis of MDV is enhanced by CIAV (DeBoer et al., 1989, p.28, In: Proceedings of the 38th Western Poultry Diseases Conference, Tempe, Ariz.). Further, it has been reported that CIAV aggravates the signs of infectious bursal disease. Rosenberger et al. (1989) Avian Dis 33:707-713. Chickens develop an age resistance to experimentally induced disease due to CAA. This is essentially complete by the age of 2 weeks, but older birds are still susceptible to infection (Yuasa, N. et al., 1979 supra; Yuasa, N. et al., Avian Dis 24, 202-209, 1980). However, if chickens are dually infected with CAA and an immunosuppressive agent (IBDV, MDV, etc.), age resistance against the disease is delayed (Yuasa, N. et al., 1979 and 1980 supra; Bulow von V. et al. J Veterinary Medicine 33:93-116, 1986). Characteristics of CIAV that may potentiate disease transmission include high resistance to environmental inactivation and some common disinfectants. The economic impact of CIAV infection on the poultry industry is clear from the fact that 10% to 30% of infected birds in disease outbreaks die.

Vaccination of birds, like other vertebrate animals, can be performed at any age. Normally, vaccinations are performed at up to 12 weeks of age for a live microorganism and between 14-18 weeks for an inactivated microorganism or other type of vaccine. For vaccination *in ovo,* vaccination can be performed in the last quarter of embryo development. The vaccine may be administered subcutaneously, by spray, orally, intraocularly, intratracheally, nasally, or by other mucosal delivery methods described herein. Thus, the CpG-like nucleic acids of the invention can be administered to birds and other non-human vertebrates using routine vaccination schedules and the antigen can be administered after an appropriate time period as described herein.

Cattle and livestock are also susceptible to infection. Diseases which affect these animals can produce severe economic losses, especially amongst cattle. The methods of the invention can be used to protect against infection in livestock, such as cows, horses, pigs, sheep, and goats.

Cows can be infected by bovine viruses. Bovine viral diarrhea virus (BVDV) is a small enveloped positive-stranded RNA virus and is classified, along with hog cholera virus (HOCV) and sheep border disease virus (BDV), in the Pestivirus genus. Although Pestiviruses were previously classified in the Togaviridae family, some studies have suggested their reclassification within the Flaviviridae family along with the flavivirus and hepatitis C virus (HCV) groups (Francki, et al., 1991).

BVDV, which is an important pathogen of cattle, can be distinguished, based on cell culture analysis, into cytopathogenic (CP) and noncytopathogenic (NCP) biotypes. The NCP biotype is more widespread although both biotypes can be found in cattle. If a pregnant cow becomes infected with an NCP strain, the cow can give birth to a persistently infected and specifically immunotolerant calf that will spread virus during its lifetime. The persistently infected cattle can succumb to mucosal disease, and both biotypes can then be isolated from the animal. Clinical manifestations can include abortion, teratogenesis, and respiratory problems, mucosal disease and mild diarrhea. In addition, severe thrombocytopenia, associated with herd epidemics, that may result in the death of the animal has been described and strains associated with this disease seem more virulent than the classical BVDVs.

Equine herpes viruses (EHV) comprise a group of antigenically distinct biological agents which cause a variety of infections in horses ranging from subclinical to fatal disease. These include Equine herpesvirus-1 (EHV-1), a ubiquitous pathogen in horses. EHV-1 is associated with epidemics of abortion, respiratory tract disease, and central nervous system disorders. Primary infection of upper respiratory tract of young horses results in a febrile illness which lasts for 8 to 10 days. Immunologically experienced mares may be re-infected via the respiratory tract without disease becoming apparent, so that abortion usually occurs without warning. The neurological syndrome is associated with respiratory disease or abortion and can affect animals of either sex at any age, leading to lack of co-ordination, weakness and posterior paralysis. Telford EAR et al. (1992) Virology 189:304-316. Other EHVs include EHV-2, or equine cytomegalovirus, EHV-3, equine coital exanthema virus, and EHV-4, previously classified as EHV-1 subtype 2.

Sheep and goats can be infected by a variety of dangerous microorganisms including visna-maedi.

Primates such as monkeys, apes and macaques can be infected by simian immunodeficiency virus (SIV). Inactivated cell-virus and cell-free whole simian immunodeficiency vaccines have been reported to afford protection in macaques. Stott et al. (1990) Lancet 36:1538-1541; Desrosiers et al. (1989) Proc Natl Acad Sci USA 86:6353-6357; Murphey-Corb et al. (1989) Science 246:1293-1297; and Carlson et al. (1990) AIDS Res Human Retroviruses 6:1239-1246. A recombinant HIV gp120 vaccine has been reported to afford protection in chimpanzees. Berman et al. (1990) Nature 345:622-625.

Cats, both domestic and wild, are susceptible to infection with a variety of microorganisms. For instance, feline infectious peritonitis is a disease which occurs in both domestic and wild cats, such as lions, leopards, cheetahs, and jaguars. When it is desirable to prevent infection with this and other types of pathogenic organisms in cats, the methods of the invention can be used to vaccinate cats to protect them against infection.

Domestic cats may become infected with several retroviruses, including but not limited to feline leukemia virus (FeLV), feline sarcoma virus (FeSV), endogenous type Concomavirus (RD-114), and feline syncytia-forming virus (FeSFV). Of these, FeLV is the most significant pathogen, causing diverse symptoms, including lymphoreticular and myeloid neoplasms, anemias, immune mediated disorders, and an immunodeficiency syndrome which is similar to human acquired immune deficiency syndrome (AIDS). Recently, a particular replication-defective FeLV mutant, designated FeLV-AIDS, has been more particularly associated with immunosuppressive properties.

The discovery of feline T-lymphotropic lentivirus (also referred to as feline immunodeficiency virus, FIV) was first reported in Pedersen et al. (1987) Science 235:790-793. Characteristics of FIV have been reported in Yamamoto et al. (1988) Leukemia 2(12 Supp):204S-215S; Yamamoto et al. (1988) Am J Vet Res 49:1246-1258; and Ackley et al. (1990) J Virol 64:5652-5655. Cloning and sequence analysis of FIV have been reported in Olmsted et al. (1989) Proc Natl Acad Sci USA 86:2448-2452 and 86:4355-4360.

Feline infectious peritonitis (FIP) is a sporadic disease occurring unpredictably in domestic and wild Felidae. While FIP is primarily a disease of domestic cats, it has been diagnosed in lions, mountain lions, leopards, cheetahs, and the jaguar. Smaller wild cats that have been afflicted with FIP include the lynx and caracal, sand cat, and pallas cat. In domestic cats, the disease occurs predominantly in young animals, although cats of all ages are susceptible. A peak incidence occurs between 6 and 12 months of age. A decline in incidence is noted from 5 to 13 years of age, followed by an increased incidence in cats 14 to 15 years old.

Viral, bacterial, and parasitic diseases in fin-fish, shellfish or other aquatic life forms pose a serious problem for the aquaculture industry. Owing to the high density of animals in the hatchery tanks or enclosed marine farming areas, infectious diseases may eradicate a large proportion of the stock in, for example, a fin-fish, shellfish, or other aquatic life forms facility. Prevention of disease is a more desired remedy to these threats to fish than intervention once the disease is in progress. Vaccination of fish is the only preventative method which may offer long-term protection through immunity. Nucleic acid based vaccinations are described in US Patent No. 5,780,448 issued to Davis.

The fish immune system has many features similar to the mammalian immune system, such as the presence of B cells, T cells, lymphokines, complement, and immunoglobulins. Fish have lymphocyte subclasses with roles that appear similar in many respects to those of the B and T cells of mammals. Vaccines can be administered by immersion or orally.

Aquaculture species include but are not limited to fin-fish, shellfish, and other aquatic animals. Fin-fish include all vertebrate fish, which may be bony or cartilaginous fish, such as, for example, salmonids, carp, catfish, yellowtail, seabream, and seabass. Salmonids are a family of fin-fish which include trout (including rainbow trout), salmon, and Arctic char. Examples of shellfish include, but are not limited to, clams, lobster, shrimp, crab, and oysters. Other cultured aquatic animals include, but are not limited to eels, squid, and octopi.

Polypeptides of viral aquaculture pathogens include but are not limited to glycoprotein (G) or nucleoprotein (N) of viral hemorrhagic septicemia virus (VHSV); G or N proteins of infectious hematopoietic necrosis virus (IHNV); VP1, VP2, VP3 or N structural proteins of infectious pancreatic necrosis virus (IPNV); G protein of spring viremia of carp (SVC); and a membrane-associated protein, tegumin or capsid protein or glycoprotein of channel catfish virus (CCV).

Typical parasites infecting horses are *Gasterophilus* spp.; *Eimeria leuckarti; Giardia* spp.; *Tritrichomonas equi; Babesia* spp. (RBC's); *Theileria equi; Trypanosoma* spp.; *Klossiella equi;* and *Sarcocystis* spp.

Typical parasites infecting swine include *Eimeria bebliecki; Eimeria scabra; Isospora suis; Giardia* spp.; *Balantidium coli; Entamoeba histolytica; Toxoplasma gondii* and *Sarcocystis* spp.; and *Trichinella spiralis.*

The major parasites of dairy and beef cattle include *Eimeria* spp.; *Cryptosporidiu*m spp.; *Giardia* spp.; *Toxoplasma gondii; Babesia bovis* (RBC); *Babesia bigemina* (RBC); *Trypanosoma* spp. (plasma); *Theileria* spp. (RBC); *Theileria parva* (lymphocytes); *Tritrichomonas foetus;* and *Sarcocystis* spp.

The major parasites of raptors include *Trichomonas gallinae;* Coccidia (*Eimeria* spp.); *Plasmodium relictum; Leucocytozoon danilewskyi* (owls); *Haemoproteus* spp.; *Trypanosoma* spp.; *Histomonas; Cryptosporidium meleagridis; Cryptosporidium baileyi; Giardia; Eimeria; Toxoplasma.*

Typical parasites infecting sheep and goats include *Eimeria* spp.; *Cryptosporidium* spp.; *Giardia* spp.; *Toxoplasma gondii; Babesia* spp. (RBC); *Trypanosoma* spp. (plasma); *Theileria* spp. (RBC); and *Sarcocystis* spp.

Typical parasitic infections in poultry include coccidiosis caused by *Eimeria acervulina; E. necatrix; E. tenella; Isospora* spp. and *Eimeria truncata;* histomoniasis; caused by *Histomonas meleagridis* and *Histomonas gallinarum*; trichomoniasis caused by *Trichomonas gallinae;* and hexamitiasis caused by *Hexamita meleagridis.* Poultry can also be infected *Emeria maxima; Emeria meleagridis; Eimeria adenoeides; Eimeria meleagrimitis*; *Cryptosporidium; Eimeria brunetti; Emeria adenoeides; Leucocytozoon* spp.; *Plasmodium* spp.; *Hemoproteus meleagridis; Toxoplasma gondii;* and *Sarcocystis.*

The methods of the invention can also be applied to the treatment and/or prevention of parasitic infection in dogs, cats, birds, fish and ferrets. Typical parasites of birds include *Trichomonas gallinae; Eimeria* spp.; *Isospora* spp.; *Giardia; Cryptosporidium; Sarcocystis* spp.; *Toxoplasma gondii; Haemoproteus*/*Parahaemoproteus; Plasmodium* spp.; *Leucocytozoon*/*Akiba; Atoxoplasma;* and *Trypanosoma* spp. Typical parasites infecting dogs include *Trichinella spiralis; Isopora* spp.; *Sarcocystis* spp.; *Cryptosporidium* spp.; *Hammondia* spp.; *Giardia duodenalis (canis); Balantidium coli; Entanzoeba histolytica; Hepatozoon canis; Toxoplasma gondii; Trypanosoma cruzi; Babesia canis; Leishmania amastigotes;* and *Neospora caninum*.

Typical parasites infecting feline species include *Isospora* spp.; *Toxoplasma gondii; Sarcocystis* spp.; *Hammondia hammondi; Besnoitia* spp.; *Giardia* spp.; *Entamoeba histolytica; Hepatozoon canis; Cytauxzoon* spp.; *Cytauxzoon* spp.; and *Cytauxzoon* spp. (red cells; RE cells).

Typical parasites infecting fish include *Hexamita* spp.; *Eimeria* spp.; *Cryptobia* spp.; *Nosema* spp.; *Myxosoma* spp.; *Chilodonella* spp.; *Trichodina* spp.; *Plistophora* spp.; *Myxosoma Henneguya; Costia* spp.; *Ichthyophithirius* spp.; and *Oodinium* spp.

Typical parasites of wild mammals include *Giardia* spp. (carnivores; herbivores); *Isospora* spp. (carnivores); *Eimeria* spp. (carnivores; herbivores); *Theileria* spp. (herbivores); *Babesia* spp. (carnivores; herbivores); *Trypanosoma* spp. (carnivores; herbivores); *Schistosoma* spp. (herbivores); *Fasciola hepatica* (herbivores); *Fascioloides magna* (herbivores); *Fasciola gigantica* (herbivores); and *Trichinella spiralis* (carnivores; herbivores).

Parasitic infections in zoos can also pose serious problems. Typical parasites of the bovidae family (blesbok, antelope, banteng, eland, gaur, impala, klipspringer, kudu, gazelle) include Eimeria spp. Typical parasites in the pinnipedae family (seal, sea lion) include *Eimeria phocae.* Typical parasites in the camelidae family (camels, llamas) include *Eimeria* spp. Typical parasites of the giraffidae family (giraffes) include *Eimeria* spp. Typical parasites in the elephantidae family (African and Asian) include *Fasciola* spp. Typical parasites of lower primates (chimpanzees, orangutans, apes, baboons, macaques, monkeys) include *Giardia* spp.; *Balantidium coli; Entamoeba histolytica; Sarcocystis* spp.; *Toxoplasma gondii; Plasmodim* spp. (RBC); *Babesia* spp. (RBC); *Trypanosoma* spp. (plasma); and *Leishmania* spp. (macrophages).

Polypeptides of bacterial pathogens include but are not limited to an iron-regulated outer membrane protein (IROMP), an outer membrane protein (OMP), and an A-protein of *Aeromonis salmonicida* which causes furunculosis, p57 protein of *Renibacterium salmoninarum* which causes bacterial kidney disease (BKD), major surface associated antigen (msa), a surface expressed cytotoxin (mpr), a surface expressed hemolysin (ish), and a flagellar antigen of Yersiniosis; an extracellular protein (ECP), an iron-regulated outer membrane protein (IROMP), and a structural protein of Pasteurellosis; an OMP and a flagellar protein of *Vibrosis anguillarum* and *V. ordalii;* a flagellar protein, an OMP protein, aroA, and purA of *Edwardsiellosis ictaluri* and *E. tarda;* and surface antigen of *Ichthyophthirius;* and a structural and regulatory protein of *Cytophaga columnari;* and a structural and regulatory protein of *Rickettsia.*

Polypeptides of a parasitic pathogen include but are not limited to the surface antigens of *Ichthyophthirius.*

The CpG-like nucleic acids may also be formulated with or administered to a subject in conjunction with an allergen. An allergen refers to a substance (antigen) that can induce an allergic or asthmatic response in a susceptible subject. The list of allergens is enormous and can include pollens, insect venoms, animal dander, dust, fungal spores and drugs (e.g., penicillin). Examples of natural, animal and plant allergens include but are not limited to proteins specific to the following genuses: *Agropyron* (e.g., *Agropyron repens*); *Agrostis* (e.g., *Agrostis alba*); *Alder; Alnus (Alnus gultinoasa); Alternaria (Alternaria alternata); Ambrosia (Ambrosia artemiisfolia); Anthoxanthum* (e.g., *Anthoxanthum odoratum); Apis* (e.g., *Apis multiflorum*); *Arrhenatherum* (e.g., *Arrhenatherum elatius*); *Artemisia (Artemisia vulgaris); Avena* (e.g., *Avena sativa*); *Betula (Betula verrucosa); Blattella* (e.g., *Blattella germanica*); *Bromus* (e.g., *Bromus inermis*); *Canine (Canis familiaris); Chamaecyparis* (e.g., *Chamaecyparis obtusa); Cryptomeria (Cryptomeria japonica); Cupressus (e.g., Cupressus sempervirens, Cupressus arizonica,* and *Cupressus macrocarpa*); *Dactylis* (e.g., *Dactylis glomerata*); *Dermatophagoides* (e.g., *Dermatophagoides farinae*); *Felis (Felis domesticus); Festuca* (e.g., *Festuca elatior*); *Holcus* (e.g., *Holcus lanatus*); *Juniperus* (e.g., *Juniperus sabinoides, Juniperus virginiana, Juniperus communis,* and *Juniperus ashei*); *Lolium* (e.g., *Lolium perenne* or *Lolium multiflorum*); *Olea (Olea europa); Parietaria* (e.g., *Parietaria officinalis* or *Parietaria judaica*); *Paspalum* (e.g., *Paspalum notatum*); *Periplaneta* (e.g., *Peniplaneta americana*); *Phalaris* (e.g., *Phalaris arundinacea*); *Phleum* (e.g., *Phleum pratense*); *Plantago* (e.g., *Plantago lanceolata*); *Poa* (e.g., *Poa pratensis* or *Poa compressa*); *Quercus (Quercus alba); Secale* (e.g., *Secale cereale*); *Sorghum* (e.g., *Sorghum halepensis*); *Thuya* (e.g., *Thuya orientalis*); and *Triticum* (e.g., *Triticum aestivum*).

The antigen may be an antigen that is encoded by a nucleic acid vector or it may be not encoded in a nucleic acid vector. In the former case the nucleic acid vector is administered to the subject and the antigen is expressed in vivo. In the latter case the antigen may be administered directly to the subject. An antigen not encoded in a nucleic acid vector as used herein refers to any type of antigen that is not a nucleic acid. For instance, in some aspects of the invention the antigen not encoded in a nucleic acid vector is a polypeptide. Minor modifications of the primary amino acid sequences of polypeptide antigens may also result in a polypeptide which has substantially equivalent antigenic activity as compared to the unmodified counterpart polypeptide. Such modifications may be deliberate, as by site-directed mutagenesis, or may be spontaneous. All of the polypeptides produced by these modifications are included herein as long as antigenicity still exists. The polypeptide may be, for example, a viral polypeptide.

The term "substantially purified" as used herein refers to the state of a particular compund as being substantially free of other proteins, lipids, carbohydrates or other materials with which it is naturally associated. The term substantially purified as used herein as applied to a polypeptide thus refers to a polypeptide which is substantially free of other proteins, lipids, carbohydrates or other materials with which it is naturally associated. One skilled in the art can purify viral or bacterial polypeptides using standard techniques for protein purification. The substantially pure polypeptide will often yield a single major band on a non-reducing polyacrylamide gel. In the case of partially glycosylated polypeptides or those that have several start codons, there may be several bands on a non-reducing polyacrylamide gel, but these will form a distinctive pattern for that polypeptide. The purity of the viral or bacterial polypeptide can also be determined by amino-terminal amino acid sequence analysis. Other types of antigens not encoded by a nucleic acid vector such as polysaccharides, small molecules, mimics, etc., are described above and included within the invention.

The invention also utilizes polynucleotides encoding the antigenic polypeptides. It is envisioned that the antigen may be delivered to the subject in a nucleic acid molecule which encodes for the antigen such that the antigen must be expressed in vivo. Such antigens delivered to the subject in a nucleic acid vector are referred to as antigens encoded by a nucleic acid vector. The nucleic acid encoding the antigen is operatively linked to a gene expression sequence which directs the expression of the antigen nucleic acid within a eukaryotic cell. The gene expression sequence is any regulatory nucleotide sequence, such as a promoter sequence or promoter-enhancer combination, which facilitates the efficient transcription and translation of the antigen nucleic acid to which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, β-actin promoter, and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, the long terminal repeats (LTR) of Moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired.

The antigen nucleic acid is operatively linked to the gene expression sequence. As used herein, the antigen nucleic acid sequence and the gene expression sequence are said to be operably linked when they are covalently linked in such a way as to place the expression or transcription and/or translation of the antigen coding sequence under the influence or control of the gene expression sequence. Two DNA sequences are said to be operably linked if induction of a promoter in the 5' gene expression sequence results in the transcription of the antigen sequence and if the nature of the linkage between the two DNA sequences does not (1) result in the introduction of a frame-shift mutation, (2) interfere with the ability of the promoter region to direct the transcription of the antigen sequence, or (3) interfere with the ability of the corresponding RNA transcript to be translated into a protein. Thus, a gene expression sequence would be operably linked to an antigen nucleic acid sequence if the gene expression sequence were capable of effecting transcription of that antigen nucleic acid sequence such that the resulting transcript is translated into the desired protein or polypeptide.

The antigen nucleic acid of the invention may be delivered to the immune system alone or in association with a vector. In its broadest sense, a vector is any vehicle capable of facilitating the transfer of the antigen nucleic acid to the cells of the immune system so that the antigen can be expressed and presented on the surface of the immune cell. The vector generally transports the nucleic acid to the immune cells with reduced degradation relative to the extent of degradation that would result in the absence of the vector. The vector optionally includes the above-described gene expression sequence to enhance expression of the antigen nucleic acid in immune cells. In general, the vectors useful in the invention include, but are not limited to, plasmids, phagemids, viruses, other vehicles derived from viral or bacterial sources that have been manipulated by the insertion or incorporation of the antigen nucleic acid sequences. Viral vectors are a preferred type of vector and include, but are not limited to, nucleic acid sequences from the following viruses: retrovirus, such as Moloney murine leukemia virus, Harvey murine sarcoma virus, murine mammary tumor virus, and Rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named here but known in the art.

Preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, M., Gene Transfer and Expression, A Laboratory Manual, W.H. Freeman Co., New York (1990) and Murry, E.J., Methods in Molecular Biology, Vol. 7, Humana Press, Inc., Cliffton, New Jersey (1991).

A preferred virus for certain applications is the adeno-associated virus (AAV), a double-stranded DNA virus. The adeno-associated virus can be engineered to be replication-deficient and is capable of infecting a wide range of cell types and species. It further has advantages such as heat and lipid solvent stability; high transduction frequencies in cells of diverse lineages, including hematopoietic cells; and lack of superinfection inhibition, thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well-known to those of skill in the art. See, e.g., Sambrook et al., Molecular Cloning: A Laboratory Manual, Second Edition, Cold Spring Harbor Laboratory Press, 1989. In the last few years, plasmid vectors have been found to be particularly advantageous for delivering genes to cells in vivo because of their inability to replicate within and integrate into a host genome. These plasmids, however, having a promoter compatible with the host cell, can express a peptide from a gene operatively encoded within the plasmid. Some commonly used plasmids include pBR322, pUC18, pUC19, pRc/CMV, pRc/RSV, pcDNA3.1, pcDNA4, pZeoSV2, SV40, and pBlueScript. Other plasmids are well-known to those of ordinary skill in the art. Additionally, plasmids may be custom designed using restriction enzymes and ligation reactions to remove and add specific fragments of DNA.

It has recently been discovered that gene-carrying plasmids can be delivered to the immune system using bacteria. Modified forms of bacteria such as *Salmonella* can be transfected with the plasmid and used as delivery vehicles. The bacterial delivery vehicles can be administered to a host subject orally or by other administration means. The bacteria deliver the plasmid to immune cells, e.g., B cells and dendritic cells, likely by passing through the gut barrier. High levels of immune protection have been established using this methodology. Such methods of delivery are useful for the aspects of the invention utilizing systemic delivery of antigen, CpG-like nucleic acid and/or other therapeutic agent.

Similar to CpG nucleic acids, the CpG-like nucleic acids are useful as adjuvants for inducing a systemic immune response. Thus CpG-like nucleic acids can be delivered to a subject exposed to an antigen to produce an enhanced immune response to the antigen. Combinations of two or more different CpG-like nucleic acids are also useful as adjuvants for inducing a systemic immune response.

CpG-like nucleic acids can also be combined with other therapeutic agents such as adjuvants to enhance immune responses. The CpG-like nucleic acid and other therapeutic agent may be administered simultaneously or sequentially. When the other therapeutic agents are administered simultaneously, they can be administered in the same or separate formulations, but are administered at the same time. The other therapeutic agents are administered sequentially with one another and with CpG-like nucleic acid, when the administration of the other therapeutic agents and the CpG-like nucleic acid is temporally separated. The separation in time between the administration of these compounds may be a matter of minutes or it may be longer. Other therapeutic agents include but are not limited to adjuvants, cytokines, antibodies, antigens, etc.

CpG nucleic acids are adjuvants that can be combined with CpG-like nucleic acids of the invention to enhance immune responses. Other immunostimulatory nucleic acids besides CpG nucleic acids and CpG-like nucleic acids can also be combined with with CpG-like nucleic acids of the invention to enhance immune responses. These other immunostimulatory nucleic acids include poly-G nucleic acids and T-rich nucleic acids.

A number of references describe the immunostimulatory properties of poly-G nucleic acids. Pisetsky and Reich (1993) Mol Biol Reports 18:217-221; Krieger and Herz (1994) Ann Rev Biochem 63:601-637; Macaya et al. (1993) Proc Natl Acad Sci USA 90:3745-3749; Wyatt et al. (1994) Proc Natl Acad Sci USA 91:1356-1360; Rando and Hogan (1998) In: Applied Antisense Oligonucleotide Technology, eds. Krieg and Stein, p. 335-352; and Kimura et al. (1994) J Biochem 116:991-994.

The poly-G nucleic acid in preferred embodiments comprises one of the following formulas: 5' X₁X₂GGGX₃X₄ 3', wherein X₁, X₂, X₃, and X₄ are nucleotides, 5' GGGNGGG 3' or 5' GGGNGGGNGGG 3', wherein N represents between 0 and 20 nucleotides. In some embodiments at least one of X₃ and X₄ is a G and in other embodiments both of X₃ and X₄ are G's.

It was recently discovered by Dr. Arthur Krieg that T-rich nucleic acids are immunostimulatory. It was presented by Dr. Krieg at the International Workshop on "Immunobiology of Bacterial CpG-DNA" held in Upper Bavaria on September 26 - 29, 1999, that poly-T nucleic acids of 24 bases in length are immunostimulatory, whereas poly-C oligonucleotides of the same length are not immunostimulatory. As used herein, "T-rich nucleic acid" refers to a nucleic acid which includes at least one poly-T sequence and/or which has a nucleotide composition of greater than 25 percent thymine (T) nucleotide residues. A poly-T sequence includes at least four consecutive T nucleotides and does not require the presence of a CpG motif. A T-rich nucleic acid may optionally be free of unmethylated CpG dinucleotides or free of methylated CpG dinucleotides.

In addition to combining CpG-like nucleic acids with other CpG-like nucleic acids or other immunostimulatory nucleic acid adjuvants, the compositions of the invention may also be administered with non-nucleic acid adjuvants. A non-nucleic acid adjuvant is any molecule or compound except for the CpG-like nucleic acids described herein which can stimulate the humoral and/or cellular immune response. Non-nucleic acid adjuvants include, for instance, adjuvants that create a depot effect, immune-stimulating adjuvants, and adjuvants that create a depot effect and stimulate the immune system.

An adjuvant that creates a depot effect as used herein is an adjuvant that causes the antigen to be slowly released in the body, thus prolonging the exposure of immune cells to the antigen. This class of adjuvants includes but is not limited to alum (e.g., aluminum hydroxide, aluminum phosphate); or emulsion-based formulations including mineral oil, non-mineral oil, water-in-oil or oil-in-water-in oil emulsion, oil-in-water emulsions such as Seppic ISA series of Montanide adjuvants (e.g., Montanide ISA 720, AirLiquide, Paris, France); MF-59 (a squalene-in-water emulsion stabilized with Span 85 and Tween 80; Chiron Corporation, Emeryville, CA; and PROVAX (an oil-in-water emulsion containing a stabilizing detergent and a micelle-forming agent; IDEC, Pharmaceuticals Corporation, San Diego, CA).

An immune-stimulating adjuvant is an adjuvant that causes activation of a cell of the immune system. It may, for instance, cause an immune cell to produce and secrete cytokines. This class of adjuvants includes but is not limited to saponins purified from the bark of the *Q*. *saponaria* tree, such as QS21 (a glycolipid that elutes in the 21^{st} peak with HPLC fractionation; Aquila Biopharmaceuticals, Inc., Worcester, MA); poly[di(carboxylatophenoxy)phosphazene (PCPP polymer; Virus Research Institute, USA); derivatives of lipopolysaccharides such as monophosphoryl lipid A (MPL; Ribi ImmunoChem Research, Inc., Hamilton, MT), muramyl dipeptide (MDP; Ribi) and threonyl-muramyl dipeptide (t-MDP; Ribi); OM-174 (a glucosamine disaccharide related to lipid A; OM Pharma SA, Meyrin, Switzerland); and Leishmania elongation factor (a purified *Leishmania* protein; Corixa Corporation, Seattle, WA).

Adjuvants that create a depot effect and stimulate the immune system are those compounds which have both of the above-identified functions. This class of adjuvants includes but is not limited to ISCOMS (immunostimulating complexes which contain mixed saponins, lipids and form virus-sized particles with pores that can hold antigen; CSL, Melbourne, Australia); SB-AS2 (SmithKline Beecham adjuvant system #2 which is an oil-in-water emulsion containing MPL and QS21: SmithKline Beecham Biologicals [SBB], Rixensart, Belgium); SB-AS4 (SmithKline Beecham adjuvant system #4 which contains alum and MPL; SBB, Belgium); non-ionic block copolymers that form micelles such as CRL 1005 (these contain a linear chain of hydrophobic polyoxpropylene flanked by chains of polyoxyethylene; Vaxcel, Inc., Norcross, GA); and Syntex Adjuvant Formulation (SAF, an oil-in-water emulsion containing Tween 80 and a nonionic block copolymer; Syntex Chemicals, Inc., Boulder, CO).

The CpG-like nucleic acids are also useful as mucosal adjuvants. It has previously been discovered that both systemic and mucosal immunity are induced by mucosal delivery of CpG nucleic acids. The systemic immunity induced in response to CpG nucleic acids included both humoral and cell-mediated responses to specific antigens that were not capable of inducing systemic immunity when administered alone to the mucosa. Furthermore, both CpG nucleic acids and cholera toxin (CT, a mucosal adjuvant that induces a Th2-like response) induced cytotoxic T lymphocytes (CTL). This was surprising since with systemic immunization, the presence of Th2-like antibodies is normally associated with a lack of CTL (Schirmbeck et al.,1995). Based on the results presented herein it is expected that the CpG-like nucleic acids will function in a similar manner.

Additionally, the CpG-like nucleic acids induce a mucosal response at both local (e.g., lung) and remote (e.g., lower digestive tract) mucosal sites. Significant levels of IgA antibodies are induced at distant mucosal sites by the CpG-like nucleic acids. CT is generally considered to be a highly effective mucosal adjuvant. As has been previously reported (Snider DP (1995) Crit Rev Immunol 15:317-48), CT induces predominantly IgG1 isotype of antibodies, which are indicative of Th2-type response. In contrast, the CpG-like nucleic acids are more Th1 with predominantly IgG2a antibodies, especially after boost or when the two adjuvants are combined. Th1-type antibodies in general have better neutralizing capabilities, and furthermore, a Th2 response in the lung is highly undesirable because it is associated with asthma. Kay AB (1996) Ann NY Acad Sci 796:1-8; Hogg JC (1997) APMIS 105:735-45. Thus the use of CpG-like nucleic acids as a mucosal adjuvant has benefits that other mucosal adjuvants cannot achieve. The CpG-like nucleic acids of the invention also are useful as mucosal adjuvants for induction of both a systemic and a mucosal immune response.

Mucosal adjuvants referred to as non-nucleic acid mucosal adjuvants may also be administered with the CpG-like nucleic acids. A non-nucleic acid mucosal adjuvant as used herein is an adjuvant other than a CpG-like nucleic acid that is capable of inducing a mucosal immune response in a subject when administered to a mucosal surface in conjunction with an antigen. Mucosal adjuvants include but are not limited to bacterial toxins e.g., Cholera toxin (CT), CT derivatives including but not limited to CT B subunit (CTB) (Wu et al., 1998, Tochikubo et al., 1998); CTD53 (Val to Asp) (Fontana et al., 1995); CTK97 (Val to Lys) (Fontana et al., 1995); CTK104 (Tyr to Lys) (Fontana et al., 1995); CTD53/K63 (Val to Asp, Ser to Lys) (Fontana et al., 1995); CTH54 (Arg to His) (Fontana et al., 1995); CTN107 (His to Asn) (Fontana et al., 1995); CTE114 (Ser to Glu) (Fontana et al., 1995); CTE112K (Glu to Lys) (Yamamoto et al., 1997a); CTS61F (Ser to Phe) (Yamamoto et al., 1997a, 1997b); CTS106 (Pro to Lys) (Douce et al., 1997, Fontana et al., 1995); and CTK63 (Ser to Lys) (Douce et al., 1997, Fontana et al., 1995), zonula occludens toxin, zot, Escherichia coli heat-labile enterotoxin, labile toxin (LT), LT derivatives including but not limited to LT B subunit (LTB) (Verweij et al., 1998); LT7K (Arg to Lys) (Komase et al., 1998, Douce et al., 1995); LT61F (Ser to Phe) (Komase et al., 1998); LT112K (Glu to Lys) (Komase et al., 1998); LT118E (Gly to Glu) (Komase et al., 1998); LT146E (Arg to Glu) (Komase et al., 1998); LT192G (Arg to Gly) (Komase et al., 1998); LTK63 (Ser to Lys) (Marchetti et al., 1998, Douce et al., 1997, 1998, Di Tommaso et al., 1996); and LTR72 (Ala to Arg) (Giuliani et al., 1998), Pertussis toxin, PT. (Lycke et al., 1992, Spangler BD, 1992, Freytag and Clemments, 1999, Roberts et al., 1995, Wilson et al., 1995) including PT-9K/129G (Roberts et al., 1995, Cropley et al., 1995); toxin derivatives (see below) (Holmgren et al., 1993, Verweij et al., 1998, Rappuoli et al., 1995, Freytag and Clements, 1999); lipid A derivatives (e.g., monophosphoryl lipid A, MPL) (Sasaki et al., 1998, Vancott et al., 1998; muramyl dipeptide (MDP) derivatives (Fukushima et al., 1996, Ogawa et al., 1989, Michalek et al., 1983, Morisaki et al., 1983); bacterial outer membrane proteins (e.g., outer surface protein A (OspA) lipoprotein of *Borrelia burgdorferi,* outer membrane protein of *Neisseria meₗₗingitidis)* (Marinaro et al., 1999, Van de Verg et al., 1996); oil-in-water emulsions (e.g., MF59) (Barchfield et al., 1999, Verschoor et al., 1999, O'Hagan, 1998); aluminum salts (Isaka et al., 1998, 1999); and saponins (e.g., QS21, Aquila Biopharmaceuticals, Inc., Worcester, MA) (Sasaki et al., 1998, MacNeal et al., 1998), ISCOMS, MF-59 (a squalene-in-water emulsion stabilized with Span 85 and Tween 80; Chiron Corporation, Emeryville, CA); the Seppic ISA series of Montanide adjuvants (e.g., Montanide ISA 720; AirLiquide, Paris, France); PROVAX (an oil-in-water emulsion containing a stabilizing detergent and a micelle-forming agent; IDEC Pharmaceuticals Corporation, San Diego, CA); Syntext Adjuvant Formulation (SAF; Syntex Chemicals, Inc., Boulder, CO); poly[di(carboxylatophenoxy)phosphazene (PCPP polymer; Virus Research Institute, USA) and Leishmania elongation factor (Corixa Corporation, Seattle, WA).

Immune responses can also be induced or augmented by the co-administration or colinear expression of cytokines (Bueler and Mulligan, 1996; Chow et al., 1997; Geissler et al.,1997; Iwasaki et al.,1997; Kim et a1.,1997) or co-stimulatory molecules (e.g., B7-1 (CD80), B7-2 (CD86); Iwasaki et al.,1997; Tsuji et al.,1997) with the CpG-like nucleic acids. The CpG-like nucleic acids may also be formulated with or administered to a subject in conjunction with a cytokine. The cytokines can be administered directly with CpG-like nucleic acids or may be administered in the form of a nucleic acid vector that encodes the cytokine, such that the cytokine can be expressed in vivo. In one embodiment, the cytokine is administered in the form of a plasmid expression vector.

The term "cytokine" is used as a generic name for a diverse group of soluble proteins and peptides which act as humoral regulators at nanomolar to picomolar concentrations and which, either under normal or pathological conditions, modulate the functional activities of individual cells and tissues. These proteins also mediate interactions between cells directly and regulate processes taking place in the extracellular environment. Examples of cytokines include, but are not limited to interleukin (IL)-1, IL-2, IL-3, IL-4, IL-5, IL-6, IL-7, IL-10, IL-12, IL-15, IL-18, granulocyte-macrophage colony stimulating factor (GM-CSF), granulocyte colony stimulating factor (G-CSF), interferon-γ (IFN-γ), IFN-α, tumor necrosis factor (TNF)-α, transforming growth factor (TGF)-β, Flt3 ligand, and CD40 ligand. In certain preferred embodiments the cytokine that is formulated with or administered to a subject in conjunction with a CpG-like nucleic acid is selected from among IL-2, IL-3, IL-4, IL-18, IFN-α, IFN-γ, TNF-α, Flt3 ligand, G-CSF, and GM-CSF. In certain embodiments where the CpG-like nucleic acid is administered to a subject in an amount effective for inducing a cytokine as an aspect of inducing an immune response, the induced cytokine may include any of the following: IL-1β IL-2, IL-6, IL-12, IL-18, TNF-α, IFN-α, and IFN-γ.

Cytokines play a role in directing the T cell response. Helper (CD4+) T cells orchestrate the immune response of mammals through production of soluble factors that act on other immune system cells, including other T cells. Most mature CD4+ T helper cells express one of two cytokine profiles: Th1 or Th2. Th1 cytokines characteristically include IFN-γ, IL-12, and IL-2. The Th1 subset promotes delayed-type hypersensitivity, cell-mediated immunity, and immunoglobulin class switching to IgG₂ₐ. In contrast, the Th2 cytokine profile characteristically includes IL-4, IL-5, IL-10 and IL-13. The Th2 subset induces humoral immunity by activating B cells, promoting antibody production, and inducing class switching to IgG₁ and IgE. In some embodiments, it is preferred that the cytokine be a Th1 cytokine.

The nucleic acids of the invention are also useful for redirecting an immune response from a Th2 immune response to a Th1 immune response. Redirection of an immune response from a Th2 to a Th1 immune response can be assessed by measuring the levels of cytokines produced in response to the nucleic acid (e.g., by inducing monocytic cells and other cells to produce Th1 cytokines, including IL-12, IFN-γ and GM-CSF). The redirection of the immune response from a Th2 to a Th1 response is particularly useful for the treatment or prevention of asthma. For instance, an effective amount of a CpG-like nucleic acid for treating asthma can be that amount useful for redirecting a Th2 type of immune response that is associated with asthma to a Th1 type of response. Th2 cytokines, especially IL-4 and IL-5, are elevated in the airways of asthmatic subjects. These cytokines promote important aspects of the asthmatic inflammatory response, including IgE isotype switching, eosinophil chemotaxis, and mast cell activation and degranulation. Th1 cytokines, especially IFN-γ and IL-12, can suppress the formation of Th2 clones and production of Th2 cytokines.

The nucleic acids of the invention may be formulated with or administered to a subject with an anti-microbial agent. An anti-microbial agent, as used herein, refers to a naturally-occurring, semi-synthetic, or synthetic compound which is capable of killing or inhibiting infectious microorganisms. The type of anti-microbial agent useful according to the invention will depend upon the type of microorganism with which the subject is infected or at risk of becoming infected. Anti-microbial agents include but are not limited to antibacterial agents, antiviral agents, antifungal agents and antiparasitic agents.

One type of anti-microbial agent is an antibacterial agent. Antibacterial agents kill or inhibit the growth or function of bacteria. A large class of antibacterial agents is antibiotics. Antibiotics which are effective for killing or inhibiting a wide range of bacteria are referred to as broad-spectrum antibiotics. Other types of antibiotics are predominantly effective against the bacteria of the class Gram-positive or Gram-negative. These types of antibiotics are referred to as narrow-spectrum antibiotics. Other antibiotics which are effective against a single organism or disease and not against other types of bacteria, are referred to as limited-spectrum antibiotics.

Antibacterial agents are sometimes classified based on their primary mode of action. In general, antibacterial agents are cell wall synthesis inhibitors, cell membrane inhibitors, protein synthesis inhibitors, nucleic acid synthesis or functional inhibitors, and competitive inhibitors. Cell wall synthesis inhibitors inhibit a step in the process of cell wall synthesis, and in general in the synthesis of bacterial peptidoglycan. Cell wall synthesis inhibitors include β-lactam antibiotics, natural penicillins, semi-synthetic penicillins, ampicillin, clavulanic acid, cephalolsporins, and bacitracin.

The β-lactams are antibiotics containing a four-membered β-1actam ring which inhibits the last step of peptidoglycan synthesis. β-lactam antibiotics can be synthesized or natural. The natural antibiotics are generally produced by two groups of fungi, *Penicillium* and *Cephalosporium* molds. The β-lactam antibiotics produced by *Penicillium* are the natural penicillins, such as penicillin G or penicillin V. These are produced by fermentation of *Penicillium chrysogenum.* The natural penicillins have a narrow spectrum of activity and are generally effective against *Streptococcus,* G*onococcus,* and *Staphylococcus.* Other types of natural penicillins, which are also effective against Gram-positive bacteria, include penicillins F, X, K, and O.

Semi-synthetic penicillins are generally modifications of the molecule 6-aminopenicillanic acid produced by a mold. The 6-aminopenicillanic acid can be modified by addition of side chains which produce penicillins having broader spectrums of activity than natural penicillins or various other advantageous properties. Some types of semi-synthetic penicillins have broad spectrums against Gram-positive and Gram-negative bacteria, but are inactivated by penicillinase. These semi-synthetic penicillins include ampicillin, azlocillin, carbenicillin, mezlocillin, oxacillin, and piperacillin. Other types of semi-synthetic penicillins have narrower activities against Gram-positive bacteria, but have developed properties such that they are not inactivated by penicillinase. These include, for instance, dicloxacillin, methicillin, and nafcillin. Some of the broad-spectrum semi-synthetic penicillins can be used in combination with β-lactamase inhibitors, such as clavulanic acids and sulbactam. The p-lactamase inhibitors do not have anti-microbial action but they function to inhibit penicillinase, thus protecting the semi-synthetic penicillin from degradation.

One of the serious side effects associated with penicillins, both natural and semi-synthetic, is penicillin allergy. Penicillin allergies are very serious and can cause death rapidly. In a subject that is allergic to penicillin, the β-lactam molecule will attach to a serum protein which initiates an IgE-mediated inflammatory response. The inflammatory response leads to anaphylaxis and possibly death.

Another type of β-lactam antibiotic is the cephalolsporins. Cephalolsporins are produced by *Cephalolsporium* molds, and have a similar mode of action to penicillin. They are sensitive to degradation by bacterial β-lactamases and thus are not always effective alone. Cephalolsporins, however, are resistant to penicillinase. They are effective against a variety of Gram-positive and Gram-negative bacteria. Cephalolsporins include, but are not limited to, cefaclor, cefamandole, cefazolin, cefetamet, cefixime, cefoperazone, cefotaxime, cefoxitin, cefsulodin, ceftazidine, ceftiaxone, cefuroxine, cephalexin, cephalothin, cephapirin, and moxalactam.

Bacitracin is another class of antibiotics which inhibit cell wall synthesis. These antibiotics, produced by *Bacillus* species, prevent cell wall growth by inhibiting the release of muropeptide subunits or peptidoglycan from the molecule that delivers the subunit to the outside of the membrane. Although bacitracin is effective against Gram-positive bacteria, its use is limited in general to topical administration because of its high toxicity. Since lower effective doses of bacitracin can be used when the compound is administered with the immunostimulatory nucleic acids of the invention, this compound can be used systemically and the toxicity reduced.

Carbapenems are another broad spectrum β-lactam antibiotic, which is capable of inhibiting cell wall synthesis. Examples of carbapenems include, but are not limited to, imipenems. Monobactams are also broad spectrum β-lactam antibiotics, and they include aztreonam. An antibiotic produced by *Streptomyces,* vancomycin, is also effective against Gram-positive bacteria by inhibiting cell membrane synthesis.

Another class of antibacterial agents is the antibacterial agents that are cell membrane inhibitors. These compounds disorganize the structure or inhibit the function of bacterial membranes. Alteration of the cytoplasmic membrane of bacteria results in leakage of cellular materials from the cell. Compounds that inhibit or interfere with the cell membrane cause death of the cell because the integrity of the cytoplasmic and outer membranes is vital to bacteria. One problem with antibacterial agents that are cell membrane inhibitors is that they can produce effects in eukaryotic cells as well as bacteria because of the similarities in phospholipids in bacterial and eukaryotic membranes. Thus these compounds are rarely specific enough to permit these compounds to be used systemically and prevent the use of high doses for local administration.

One clinically useful antibacterial agent that is a cell membrane inhibitor is Polymyxin, produced by *Bacillus polymyxis.* Polymyxins interfere with membrane function by binding to membrane phospholipids. Polymyxin is effective mainly against Gram-negative bacteria and is generally used in severe *Pseudomonas* infections or *Pseudomonas* infections that are resistant to less toxic antibiotics. It is also used in some limited instances topically. Its limited use is due to the severe side effects associated with systemic administration, such as damage to the kidneys and other organs.

Other cell membrane inhibitors include Amphotericin B and Nystatin produced by the bacterium *Streptomyces* which are also antifungal agents, used predominantly in the treatment of systemic fungal infections and *Candida* yeast infections, respectively. Imidazoles, produced by the bacterium *Streptomyces,* are another class of antibiotic that is a cell membrane inhibitor. Imidazoles are used as antibacterial agents as well as antifungal agents, e.g., used for treatment of yeast infections, dermatophytic infections, and systemic fungal infections. Imidazoles include but are not limited to clotrimazole, fluconazole, itraconazole, ketoconazole, and miconazole.

Many antibacterial agents are protein synthesis inhibitors. These compounds prevent bacteria from synthesizing structural proteins and enzymes and thus cause inhibition of bacterial cell growth or function or cell death. In general these compounds interfere with the processes of transcription or translation. Antibacterial agents that block transcription include but are not limited to Rifampins, produced by the bacterium *Streptomyces,* and Ethambutol, a synthetic chemical. Rifampins, which inhibit the enzyme RNA polymerase, have a broad spectrum o activity and are effective against Gram-positive and Gram-negative bacteria as well as *Mycobacterium tuberculosis.* Ethambutol is effective against *Mycobacterium tuberculosis.*

Antibacterial agents which block translation interfere with bacterial ribosomes to prevent mRNA from being translated into proteins. In general this class of compounds includes but is not limited to tetracyclines, chloramphenicol, the macrolides (e.g., azithromycin, erythromycin) and the aminoglycosides (e.g., amikacin, gentamicin, kanamycin, streptomycin, tobramycin).

Some of these compounds bind irreversibly to the 30 S ribosomal subunit and cause a misreading of the mRNA, e.g., the aminoglycosides. The aminoglycosides are a class of antibiotics which are produced by the bacterium *Streptomyces,* such as, for instance amikacin, gentamicin, kanamycin, streptomycin, and tobramycin. Aminoglycosides have been used against a wide variety of bacterial infections caused by Gram-positive and Gram-negative bacteria. Streptomycin has been used extensively as a primary drug in the treatment of tuberculosis. Gentamicin is used against many strains of Gram-positive and Gram-negative bacteria, including *Pseudomonas* infections, especially in combination with tobramycin. Kanamycin is used against many Gram-positive bacteria, including penicillin-resistant *Staphylococci.* One side effect of aminoglycosides that has limited their use clinically is that at dosages which are essential for efficacy, prolonged use has been shown to impair kidney function and cause damage to the auditory nerves leading to deafness.

Another type of translation inhibitor antibacterial agent is the tetracyclines. The tetracyclines bind reversibly to the 30 S ribosomal subunit and interfere with the binding of charged tRNA to the bacterial ribosome. The tetracyclines are a class of antibiotics, produced by the bacterium *Streptomyces,* that are broad-spectrum and are effective against a variety of Gram-positive and Gram-negative bacteria. Examples of tetracyclines include chlortetracycline, doxycycline, minocycline, and tetracycline. They are important for the treatment of many types of bacteria but are particularly important in the treatment of Lyme disease *(Borrelia burgdorferi).*

As a result of their low toxicity and minimal direct side effects, the tetracyclines have been overused and misused by the medical community, leading to problems. For instance, their overuse has led to widespread development of resistance. When used in combination with the immunostimulatory nucleic acids of the invention, these problems can be minimized and tetracyclines can be effectively used for the broad spectrum treatment of many bacteria.

Antibacterial agents such as the macrolides bind reversibly to the 50 S ribosomal subunit and inhibits elongation of the protein by peptidyl transferase or prevents the release of uncharged tRNA from the bacterial ribosome or both. The macrolides contain large lactone rings linked through glycoside bonds with amino sugars. These compounds include azithromycin, clarithromycin, clindamycin, erythromycin, lincomycin, oleandomycin, and roxithromycin. Erythromycin is active against most Gram-positive bacteria, *Neisseria, Legionella* and *Haemophilus,* but not against the *Enterobacteriacéae.* Lincomycin and clindamycin, which block peptide bond formation during protein synthesis, are used against Gram-positive bacteria.

Another type of translation inhibitor is chloramphenicol. Chloramphenicol binds the 70 S ribosome, inhibiting the bacterial enzyme peptidyl transferase and thereby preventing the growth of the polypeptide chain during protein synthesis. Chloramphenicol can be prepared from *Streptomyces* or produced entirely by chemical synthesis. One serious side effect associated with chloramphenicol is aplastic anemia. Aplastic anemia develops at doses of chloramphenicol which are effective for treating bacteria in a small proportion (1/50,000) of patients. Once a highly prescribed antibiotic, chloramphenicol is now seldom uses as a result of the deaths from anemia. Because of its effectiveness it is still used in life-threatening situations (e.g., typhoid fever). By combining chloramphenicol with the immunostimulatory nucleic acids these compounds can again be used as antibacterial agents because the immunostimulatory agents allow a lower dose of the chloramphenicol to be used, a dose that does not produce side effects.

Some antibacterial agents disrupt nucleic acid synthesis or function, e.g., bind to DNA or RNA so that their messages cannot be read. These include but are not limited to quinolones and co-trimoxazole, both synthetic chemicals, and rifamycins, a natural or semi-synthetic chemical. The quinolones block bacterial DNA replication by inhibiting the DNA gyrase, the enzyme needed by bacteria to produce their circular DNA. They are broad spectrum and examples include ciprofloxacin, enoxacin, nalidixic acid, norfloxacin, ofloxacin, and temafloxacin. Nalidixic acid is a bactericidal agent that binds to the DNA gyrase enzyme (topoisomerase) which is essential for DNA replication and allows supercoils to be relaxed and reformed, inhibiting DNA gyrase activity. The main use of nalidixic acid is in treatment of lower urinary tract infections (UTI) because it is effective against several types of Gram-negative bacteria such as *E. coli, Enterobacter aerogenes, K. pneumoniae,* and *Proteus* species which are common causes of UTI. Co-trimoxazole is a combination of sulfamethoxazole and trimethoprim, which blocks the bacterial synthesis of folic acid needed to make DNA nucleotides. Rifampicin is a derivative of rifamycin that is active against Gram-positive bacteria (including *Mycobacterium tuberculosis* and meningitis caused by *Neisseria meningitidis*) and some Gram-negative bacteria. Rifampicin binds to the beta subunit of the polymerase and blocks the addition of the first nucleotide which is necessary to activate the polymerase, thereby blocking mRNA synthesis.

Another class of antibacterial agents is compounds that function as competitive inhibitors of bacterial enzymes. The competitive inhibitors are mostly all structurally similar to a bacterial growth factor and compete for binding but do not perform the metabolic function in the cell. These compounds include sulfonamides and chemically modified forms of sulfanilamide which have even higher and broader antibacterial activity. The sulfonamides (e.g., gantrisin and trimethoprim) are useful for the treatment of *Streptococcus pneumoniae,* beta-hemolytic *Streptococci* and *E. coli,* and have been used in the treatment of uncomplicated UTI caused by *E. coli,* and in the treatment of meningococcal meningitis.

Antiviral agents are compounds which prevent infection of cells by viruses or replication of the virus within the cell. There are many fewer antiviral drugs than antibacterial drugs because the process of viral replication is so closely related to DNA replication within the host cell, that non-specific antiviral agents would often be toxic to the host. There are several stages within the process of viral infection which can be blocked or inhibited by antiviral agents. These stages include attachment of the virus to the host cell (immunoglobulin or binding peptides), uncoating of the virus (e.g., amantadine), synthesis or translation of viral mRNA (e.g., interferon), replication of viral RNA or DNA (e.g., nucleoside analogues), maturation of new virus proteins (e.g., protease inhibitors), and budding and release of the virus.

Nucleotide analogues are synthetic compounds which are similar to nucleotides, but which have an incomplete or abnormal deoxyribose or ribose group. Once the nucleotide analogues are in the cell, they are phosphorylated, producing the triphosphate form which competes with normal nucleotides for incorporation into the viral DNA or RNA. Once the triphosphate form of the nucleotide analogue is incorporated into the growing nucleic acid chain, it causes irreversible association with the viral polymerase and thus chain termination. Nucleotide analogues include, but are not limited to, acyclovir (used for the treatment of herpes simplex virus and varicella-zoster virus), gancyclovir (useful for the treatment of cytomegalovirus), idoxuridine, ribavirin (useful for the treatment of respiratory syncitial virus), dideoxyinosine, dideoxycytidine, and zidovudine (azidothymidine).

The interferons are cytokines which are secreted by virus-infected cells as well as immune cells. The interferons function by binding to specific receptors on cells adjacent to the infected cells, causing the change in the cell which protects it from infection by the virus. α and β-interferon also induce the expression of Class I and Class II MHC molecules on the surface of infected cells, resulting in increased antigen presentation for host immune cell recognition. α and β-interferons are available as recombinant forms and have been used for the treatment of chronic hepatitis B and C infection. At the dosages which are effective for antiviral therapy, interferons have severe side effects such as fever, malaise and weight loss.

Immunoglobulin therapy is used for the prevention of viral infection. Immunoglobulin therapy for viral infections is different from immunoglobulin therapy for bacterial infections because, rather than being antigen-specific, the immunoglobulin therapy functions by binding to extracellular virions and preventing them from attaching to and entering cells which are susceptible to the viral infection. The therapy is useful for the prevention of viral infection for the period of time that the antibodies are present in the host. In general there are two types of immunoglobulin therapies, normal immune globulin therapy and hyper-immune globulin therapy. Normal immune globulin therapy utilizes a antibody product which is prepared from the serum of normal blood donors and pooled. This pooled product contains low titers of antibody to a wide range of human viruses, such as hepatitis A, parvovirus, enterovirus (especially in neonates). Hyper-immune globulin therapy utilizes antibodies which are prepared from the serum of individuals who have high titers of an antibody to a particular virus. Those antibodies are then used against a specific virus. Examples of hyper-immune globulins include zoster immune globulin (useful for the prevention of varicella in immunocompromised children and neonates), human rabies immunoglobulin (useful in the post-exposure prophylaxis of a subject bitten by a rabid animal), hepatitis B immune globulin (useful in the prevention of hepatitis B virus, especially in a subject exposed to the virus), and RSV immune globulin (useful in the treatment of respiratory syncitial virus infections).

Another type of immunoglobulin therapy is passive immunization. This involves the administration of antibodies or antibody fragments to viral surface proteins. Two types of vaccines which are available for passive immunization of hepatitis B include serum-derived hepatitis B antibodies and recombinant hepatitis B antibodies. Both are prepared from hepatitis B surface antigen (HBsAg). The antibodies are administered in three doses to subjects at high risk of infection with hepatitis B virus, such as health care workers, sexual partners of chronic carriers, and infants.

Antifungal agents are useful for the treatment and prevention of infective fungi. Antifungal agents are sometimes classified by their mechanism of action. Some antifungal agents function as cell wall inhibitors by inhibiting glucose synthase. These include, but are not limited to, basiungin/ECB. Other antifungal agents function by destabilizing membrane integrity. These include, but are not limited to, imidazoles, such as clotrimazole, fluconazole, itraconazole, ketoconazole, miconazole, sertaconzole, and voriconacole, as well as FK 463, amphotericin B, BAY 38-9502, MK 991, pradimicin, UK 292, butenafine, and terbinafine. Other antifungal agents function by breaking down chitin (e.g., chitinase) or immunosuppression (501 cream). Some examples of commercially-available antifungal agents are shown in Table 1.

**Table 1. Antifungal Agents**

| **Company** | **Brand Name** | **Generic Name** | **Indication** | **Mechanism of Action** |
|---|---|---|---|---|
| Pharmacia & Upjohn | PNU 196443 | PNU 196443 | Anti Fungal | n/k |
| Lilly | LY 303366 | Basiungin/ECB | Fungal Infections | Cell wall inhibitor, glucose synthase inhibitor |
| Bayer | Canesten | Clotrimazole | Fungal Infections | Membrane integrity destabilizer |
| Fujisawa | FK 463 | FK 463 | Fungal Infections | Membrane integrity destabilizer |
| Mylan | Sertaconazole | Sertaconazole | Fungal Infections | Membrane integrity destabilizer |
| Genzyme | Chitinase | Chitinase | Fungal infections. Systemic | Chitin Breakdown |
| Liposome | Abelcet | Amphotericin B, Liposomal | Fungal infections. Systemic | Membrane integrity destabilizer |
| Sequus | Amphotec | Amphotericin B, Liposomal | Fungal Infections, Systemic | Membrane integrity destabilizer |
| Bayer | BAY 38-9502 | BAY 38-9502 | Fungal Infections, Systemic | Membrane integrity destabilizer |
| Pfizer | Diflucan | Fluconazole | Fungal Infections, Systemic | Membrane integrity destabilizer |
| Johnson & Johnson | Sporanox | Itraconazole | Fungal Infections, Systemic | Membrane integrity destabilizer |
| Sepracor | Itraconzole (2R, 4S) | Itraconzole (2R, 4S) | Fungal Infections, Systemic | Membrane integrity destabilizer |
| Johnson & Johnson | Nizoral | Ketoconazole | Fungal Infections, Systemic | Membrane integrity destabilizer |
| Johnson & Johnson | Monistat | Miconazole | Fungal Infections, Systemic | Membrane integrity destabilizer |
| Merck | MK 991 | MK 991 | Fungal Infections, Systemic | Membrane integrity destabilizer |
| Bristol Myers Sq'b | Pradimicin | Pradimicin | Fungal Infections, Systemic | Membrane integrity destabilizer |
| Pfizer | UK-292, 663 | UK-292, 663 | Fungal Infections, Systemic | Membrane integrity destabilizer |
| Pfizer | Voriconazole | Voriconazole | Fungal infections, Systemic | Membrane integrity destabilizer |
| Mylan | 501 Cream | 501 Cream | Inflammatory Fungal Conditions | Immunosuppression |
| Mylan | Mentax | Butenafine | Nail Fungus | Membrane integrity destabilizer |
| Schering Plough | Anti Fungal | Anti Fungal | Opportunistic Infections | Membrane integrity destabilizer |
| Alza | Mycelex Troche | Clotrimazole | Oral Thrush | Membrane integrity destabilizer |
| Novartis | Lamisil | Terbinafine | Systemic Fungal Infections, Onychomycosis | Membrane integrity destabilizer |

Examples of antiparasitic agents, also referred to as parasiticides useful for human administration, include but are not limited to albendazole, amphotericin B, benznidazole, bithionol, chloroquine HCl, chloroquine phosphate, clindamycin, dehydroemetine, diethylcarbamazine, diloxanide furoate, doxycycline, eflomithine, furazolidaone, glucocorticoids, halofantrine, iodoquinol, ivermectin, mebendazole, mefloquine, meglumine antimoniate, melarsoprol, metrifonate, metronidazole, niclosamide, nifurtimox, oxamniquine, paromomycin, pentamidine isethionate, piperazine, praziquantel, primaquine phosphate, proguanil, pyrantel pamoate, pyrimethamine-sulfadoxine, pyrimethamine-sulfonamides, quinacrine HCl, quinine sulfate, quinidine gluconate, spiramycin, stibogluconate sodium (sodium antimony gluconate), suramin, tetracycline, thiabendazole, tinidazole, trimethroprim-sulfamethoxazole, and tryparsamide, some of which are used alone or in combination with others.

Parasiticides used in non-human subjects include albendazole, amprolium; decoquinate, benzimidazoles such as fenbendazole, clorsulon, dichlorvos, diethylcarbamazine, doramectic, epsiprantel, febantel, fenbendazole, hygromycin B thiacetarsemide sodium, iprinomectin, ivermectin, lasalocid, levamisole, melarsomine, metronidazole, milbemycin oxime, monensin sulfadimethoxine, moxidectin, N-butyl chloride, oxfendazole, oxibendazole, piperazine, praziquantel, pyrantel pamoate, pyrantel tartrate, sulfamethazine, sulfaquinoxaline, thiabendazole, and toluene. Parasiticides used in horses include mebendazole, oxfendazole, febantel, pyrantel, dichlorvos, trichlorfon, ivermectin, piperazine; for *S. westeri:* ivermectin, benzimiddazoles such as thiabendazole, cambendazole, oxibendazole and fenbendazole. Useful parasiticides in dogs include milbemycin oxine, ivermectin, pyrantel pamoate and the combination of ivermectin and pyrantel. The treatment of parasites in swine can include the use of levamisole, piperazine, pyrantel, thiabendazole, dichlorvos and fenbendazole. In sheep and goats anthelminthic agents include levamisole or ivermectin. Caparsolate has shown some efficacy in the treatment of *D. immitis* (heartworm) in cats.

The CpG-like nucleic acids may also be formulated with or administered to a subject in conjunction with an anti-asthma or anti-allergy therapy. In certain embodiments, an asthma/allergy medicament is a medicament selected from the group consisting of phophodiesterase (PDE)-4 inhibitor, Bronchodilator/beta-2 agonist, K+ channel opener, VLA-4 antagonist, Neurokin antagonist, TXA2 synthesis inhibitor, Xanthanine, Arachidonic acid antagonist, 5 lipoxygenase inhibitor, Thromboxin A2 receptor antagonist, Thromboxane A2 antagonist, Inhibitor of 5-lipox activation protein, and Protease inhibitor, but is not so limited. In some important embodiments, the asthma/allergy medicament is a Bronchodilator/beta-2 agonist selected from the group consisting of salmeterol, salbutamol, terbutaline, D2522/formoterol, fenoterol, and orciprenaline.

In another embodiment, the asthma/allergy medicament is a medicament selected from the group consisting of Anti-histamines and Prostaglandin inducers. In one embodiment, the anti-histamine is selected from the group consisting of astemizole, azelastine, betatastine, buclizine, cetirizine, cetirizine analogues, CS 560, desloratadine, ebastine, epinastine, fexofenadine, HSR 609, levocabastine, loratidine, mizolastine, norastemizole, terfenadine, and tranilast. In another embodiment, the Prostaglandin inducer is S-5751.

In yet another embodiment, the asthma/allergy medicament is selected from the group consisting of Steroids and Immunomodulators. The immunomodulators may be selected from the group consisting of anti-inflammatory agents, leukotriene antagonists, IL-4 muteins, Soluble IL-4 receptors, Immunosuppressants, anti-IL-4 antibodies, IL-4 antagonists, anti-IL-5 antibodies, soluble IL-13 receptor-Fc fusion proteins, anti-IL-9 antibodies, CCR3 antagonists, CCR5 antagonists, VLA-4 inhibitors, and Downregulators of IgE, but are not so limited. In one embodiment, the downregulator of IgE is an anti-IgE.

In another embodiment, the Steroid is selected from the group consisting of belomethasone, budesonide, fluticasone, methylprednisolone, prednisone, and triamcinolone. In still a further embodiment, the Immunosuppressant is a Tolerizing peptide vaccine.

The CpG-like nucleic acids may also be formulated with or administered to a subject in conjunction with an anti-cancer therapy. Anti-cancer therapies include cancer medicaments, radiation and surgical procedures. As used herein, a "cancer medicament" refers to a pharmaceutical agent which is administered to a subject for the purpose of treating a cancer. As used herein, "treating cancer" includes preventing the development of a cancer, reducing the symptoms of cancer, and/or inhibiting the growth of an established cancer. In other aspects, the cancer medicament is administered to a subject at risk of developing a cancer for the purpose of reducing the risk of developing the cancer. Various types of medicaments for the treatment of cancer are described herein. For the purpose of this specification, cancer medicaments are classified as chemotherapeutic agents, immunotherapeutic agents, radiotherapeutic agents, cancer vaccines, hormone therapy, and biological response modifiers.

Additionally, the methods of the invention are intended to embrace the use of more than one cancer medicament along with the CpG-like nucleic acids. As an example, where appropriate, the CpG-like nucleic acids may be administered with a both a chemotherapeutic agent and an immunotherapeutic agent. Alternatively, the cancer medicament may embrace an immunotherapeutic agent and a cancer vaccine, or a chemotherapeutic agent and a cancer vaccine, or a chemotherapeutic agent, an immunotherapeutic agent and a cancer vaccine all administered to one subject for the purpose of treating a subject having a cancer or at risk of developing a cancer.

Cancer medicaments function in a variety of ways. Some cancer medicaments work by targeting physiological mechanisms that are specific to tumor cells. Examples include the targeting of specific genes (and their gene products, i.e., proteins primarily) which are mutated in cancers. Such genes include but are not limited to oncogenes (e.g., Ras, Her2, bcl-2), tumor suppressor genes (e.g., EGF, p53, Rb), and cell cycle targets (e.g., CDK4, p21, telomerase). Cancer medicaments can alternately target signal transduction pathways and molecular mechanisms which are altered in cancer cells. Targeting of cancer cells via the epitopes expressed on their cell surface is accomplished through the use of monoclonal antibodies and derivatives and analogs of monoclonal antibodies. This latter type of cancer medicament is generally referred to herein as a form of immunotherapy. Immunotherapies also include cytokines such as IFNα, IL-2, and IL-12.

Other cancer medicaments target cells other than cancer cells. For example, some medicaments prime the immune system to attack tumor cells (i.e., cancer vaccines). Still other medicaments, called angiogenesis inhibitors, function by attacking the blood supply of solid tumors. Since the most malignant cancers are able to metastasize (i.e., exit the primary tumor site and seed a nonadjacent tissue, thereby forming a secondary tumor), medicaments that impede this metastasis are also useful in the treatment of cancer. Angiogenic mediators include basic fibroblast growth factor (b-FGF), vascular endothelial growth factor (VEGF), angiopoietins, angiostatin, endostatin, tumor necrosis factor (TNF)-α, TNP-470, thrombospondin-1, platelet factor 4, carboxyamido-triazole (CAI), and certain members of the integrin family of proteins. One category of this type of medicament is a metalloproteinase inhibitor, which inhibits the enzymes used by the cancer cells to exit the primary tumor site and extravasate into another tissue.

Some cancer cells are antigenic and thus can be targeted by the immune system. In one aspect, the combined administration of CpG-like nucleic acids and cancer medicaments, particularly those which are classified as cancer immunotherapies, is useful for stimulating a specific immune response against a cancer antigen.

The theory of immune surveillance is that a prime function of the immune system is to detect and eliminate neoplastic cells before a tumor forms. A basic principle of this theory is that cancer, cells are antigenically different from normal cells and thus elicit immune reactions that are similar to those that cause rejection of immunologically incompatible ("non-self') allografts. Studies have confirmed that tumor cells differ, either qualitatively or quantitatively, in their expression of antigens. For example, "tumor-specific antigens" are antigens that are specifically associated with tumor cells but not normal cells. Examples of tumor-specific antigens are viral antigens in tumors induced by DNA or RNA viruses, as well as neo-antigens arising from abnormal gene splicing. "Tumor-associated" antigens are present in both tumor cells and normal cells but are present in a different quantity or a different form in tumor cells. Examples of such antigens are oncofetal antigens (e.g., carcinoembryonic antigen), differentiation antigens (e.g., T and Tn antigens), and oncogene products (e.g., HER/neu).

Different types of cells that can kill tumor targets in vitro and in vivo have been identified: natural killer cells (NK cells), cytolytic T lymphocytes (CTLs), lymphokine-activated killer cells (LAKs), and activated macrophages. NK cells can kill tumor cells without having been previously sensitized to specific antigens, and the activity does not require the presence of class I antigens encoded by the major histocompatibility complex (MHC) on target cells. NK cells are thought to participate in the control of nascent tumors and in the control of metastatic growth. In contrast to NK cells, CTLs can kill tumor cells only after they have been sensitized to tumor antigens and when the target antigen is expressed on the tumor cells that also express MHC class I. CTLs are thought to be effector cells in the rejection of transplanted tumors and of tumors caused by DNA viruses. LAK cells are a subset of null lymphocytes distinct from the NK and CTL populations. Activated macrophages can kill tumor cells in a manner that is neither antigen-dependent nor MHC-restricted once activated. Activated macrophages are thought to decrease the growth rate of the tumors they infiltrate. *In vitro* assays have identified other immune mechanisms such as antibody-dependent, cell-mediated cytotoxic (ADCC) reactions and lysis by antibody plus complement. However, these immune effector mechanisms are thought to be less important than the function ofNK, CTLs, LAK, and macrophages in vivo (for a review see Piessens, W.F., and David, J., "Tumor Immunology", In: Scientific American Medicine, Vol. 2, Scientific American Books, N.Y.; pp. 1-13, 1996).

The goal of immunotherapy is to augment a patient's immune response to an established tumor. One method of immunotherapy includes the use of adjuvants. Adjuvant substances derived from microorganisms, such as Bacillus Calmette-Guérin (BCG), heighten the immune response and enhance resistance to tumors in animals.

Immunotherapeutic agents are medicaments which derive from antibodies or antibody fragments which specifically bind or recognize a cancer antigen. Preferably the antibody or antibody fragment is derived from a monoclonal antibody that is specific for a tumor cell or a tumor cell antigen. Monoclonal antibodies are well known in the art, as are a variety of molecules derived from monoclonal antibodies, including chimeric, humanized, and bispecific monoclonal antibodies, Fab fragments, (Fab')₂ fragments, Fv fragments, single-chain Fv molecules, etc. As described above, a cancer antigen as used herein is broadly defined as an antigen expressed by a cancer cell. Preferably, the antigen is expressed at the cell surface of the cancer cell. Even more preferably, the antigen is one which is not expressed by normal cells, or at least not expressed to the same level as in cancer cells. Antibody-based immunotherapies may function by binding to the cell surface of a cancer cell and thereby stimulate the endogenous immune system to attack the cancer cell. Another way in which antibody-based therapy functions is as a delivery system for the specific targeting of toxic substances to cancer cells. In this regard, antibodies are usually conjugated to toxins such as ricin (e.g., from castor beans), calicheamicin and maytansinoids, to radioactive isotopes such as Iodine-131 (¹³¹I) and Yttrium-90 (⁹⁰Y), to chemotherapeutic agents (as described herein), or to biological response modifiers. In this way, the toxic substances can be concentrated in the region of the cancer and non-specific toxicity to normal cells can be minimized. In addition to the use of antibodies which are specific for cancer antigens, antibodies which bind to vasculature, such as those which bind to endothelial cells, are also useful in the invention. This is because solid tumors generally are dependent upon newly formed blood vessels to survive, and thus most tumors are capable of recruiting and stimulating the growth of new blood vessels. As a result, one strategy of many cancer medicaments is to attack the blood vessels feeding a tumor and/or the connective tissues (or stroma) supporting such blood vessels.

The use of CpG-like nucleic acids in conjunction with immunotherapeutic agents such as monoclonal antibodies is able to increase long-term survival through a number of mechanisms including significant enhancement of ADCC (as discussed above), activation of NK cells, and an increase in IFN-α levels. IFN-α is believed to induce activation of NK cells. The nucleic acids when used in combination with monoclonal antibodies serve to reduce the dose of the antibody required to achieve a biological result.

Examples of cancer immunotherapies which are currently being used or which are in development are listed in Table 2.

**Table 2. Cancer Immunotherapies in Development or on the Market**

| **MARKETER** | **BRAND NAME (GENERIC NAME)** | **INDICATION** |
|---|---|---|
| Altarex, Canada | Ovarex (B43.13, anti-idiotypic CA125, mouse MAb) | Ovarian |
| Aronex Pharmaceuticals, Inc. | ATRAGEN® | Acute promyelocytic leukemia |
| Aronex Pharmaceuticals, Inc. | ATRAGEN® | Kaposi's sarcoma |
| Aronex Pharmaceuticals, Inc. | ATRAGEN® | non-Hodgkin's lymphoma |
| Center of Molecular Immunology | ior c5 (murine MAb colorectal) for radioimmunotherapy | Colorectal |
| Center of Molecular Immunology | ior egf/r3 (anti EGF-R humanized Ab) | Radioimmunotherapy |
| Center of Molecular Immunology | ior t6 (anti CD6, murine MAb) CTCL | Cancer |
| Centocor/Ajinomoto | Panorex® (17-1A) (chimeric murine monoclonal antibody) | Pancreatic, lung, breast, ovary |
| Centocor/Glaxo/Ajinomoto | Panorex® (17-1A) (murine monoclonal antibody) | Adjuvant therapy for colorectal (Dukes-C) |
| Coulter Pharma (Clinical results have been positive, but the drug has been associated with significant bone marrow toxicity) | Bexxar (anti-CD20 Mab labeled with ¹³¹I) | non-Hodgkin's lymphoma |
| Creative BioMolecules/Chiron | BABS (biosynthetic antibody binding site) Proteins | Breast cancer |
| Cytogen | OV103 (Yttrium-90 labelled antibody) | Ovarian |
| Cytogen | OV 103 (Yttrium-90 labelled antibody) | Prostate |
| Cytogen Corp. | Quadramet (CYT-424) radiotherapeutic agent | Bone metastases |
| Genentech | Anti-VEGF, RhuMAb (inhibits angiogenesis) | Lung, breast, prostate, colorectal |
| Genentech/Hoffmann-La Roche | Herceptin, anti-Her2 hMAb | Breast/ovarian |
| Genetics Institute/AHP | GNI-250 Mab | Colorectal |
| Glaxo Wellcome plc | 3622W94 MAb that binds to EGP40 (17-1A) pancarcinoma antigen on adenocarcinomas | non-small cell lung, prostate (adjuvant) |
| IDEC | IDEC-Y2B8 (murine, anti-CD20 MAb labeled with Yttrium-90) | non-Hodgkin's lymhoma |
| IDEC | MELIMMLTNE-1 (murine monoclonal antibody therapeutic vaccine) | Melanoma |
| IDEC | MELIMMUNE-2 (murine monoclonal antibody therapeutic vaccine) | Melanoma |
| IDEC Pharmaceuticals Corp./Genentech | Rituxan™ (MAb against CD20) pan-B Ab in combination with chemotherapy | B cell lymphoma |
| IDEC/Genentech, Inc./Hoffmann-LaRoche (first monoclonal antibody licensed for the treatment of cancer in the U.S.) | Rituxan™ (rituximab, Mabthera) (IDEC-C2B8, chimeric murine/human anti-CD20 MAb) | non-Hodgkin's lymphoma |
| ImClone Systems | BEC2 (anti-idiotypic MAb, mimics the GD₃ epitope) | Melanoma |
| ImClone Systems | BEC2 (anti-idiotypic MAb, mimics the GD₃ epitope) (with BCG) | Small cell lung |
| ImClone Systems | C225 (chimeric anti-EGFr monoclonal antibody) + adriamycin | Prostate |
| ImClone Systems | C225 (chimeric anti-EGFr monoclonal antibody) + taxol | Breast |
| ImClone Systems | C225 (chimeric anti-EGFr monoclonal antibody) + cisplatin or radiation | Head & neck, non-small cell lung cancer |
| ImClone Systems | C225 (chimeric monoclonal antibody to epidermal growth factor receptor (EGFr)) | Renal cell |
| ImClone Systems (licensed from RPR) | C225 (chimeric anti-EGFr monoclonal antibody) + doxorubicin | Prostate |
| ImClone Systems/Chugai | FLK-2 (monoclonal antibody to fetal liver kinase-2 (FLK-2)) | Tumor-associated angiogenesis |
| Immunex/AHP | CMA 676 (monoclonal antibody conjugate) | Acute myelogenous leukemia |
| ImmunoGen, Inc. | Humanized MAb/small-drug conjugate | Small-cell lung |
| Immunomedics | LymphoCide (humanized LL2 antibody) | non-Hodgkin's B-cell lymphoma |
| Immunomedics, Inc. | CEACIDE™ (I-131) | Colorectal and other |
| Immunomedics, Inc. | ImmuRAIT-CEA | Colorectal |
| LeukoSite/Ilex Oncology | LDP-03, huMAb to the leukocyte antigen CAMPATH | Chronic lymphocytic leukemia (CLL) |
| Medarex | MDX-11 (complement activating receptor (CAR) monoclonal antibody) | Acute myelogenous leukemia (AML) |
| Medarex | MDX-11 (complement activating receptor (CAR) monoclonal antibody) | Ex vivo bone marrow purging in acute myelogenous leukemia (AML) |
| Medarex | MDX-210 (humanized anti-HER-2 bispecific antibody) | Cancer |
| Medarex | MDX-22 (humanized bispecific antibody, MAb-conjugates) (complement cascade activators) | Acute myleoid leukemia |
| Medarex | MDX-220 (bispecific for tumors that express TAG-72) | Lung, colon, prostate, ovarian, endometrial, pancreatic and gastric |
| Medarex, Inc. | MDX-260 bispecific, targets GD-2 | Melanoma, glioma, neuroblastoma |
| Medarex/Merck KgaA | MDX-447 (humanized anti-EGF receptor bispecific antibody) | EGF receptor cancers (head & neck, prostate, lung, bladder, cervical, ovarian) |
| Medarex/Novartis | MDX-210 (humanized anti-HER-2 bispecific antibody) | Breast, ovarian |
| Medarex/Novartis | MDX-210 (humanized anti-HER-2 bispecific antibody) | Prostate, non-small cell lung, pancreatic, breast |
| Medarex/Novartis | MDX-210 (humanized anti-HER-2 bispecific antibody) | Renal and colon |
| Medarex/Novartis | MDX-210 (humanized anti-HER-2 bispecific antibody) | Prostate |
| Medarex/Novartis | MDX-210 (humanized anti-HER-2 bispecific antibody) | Comb. Therapy with G-CSF for various cancers, esp. breast |
| Merck KgaA | EMD-72000 (chimeric-EGF antagonist) | Cancer |
| NeoRx | Pretarget™ radioactive antibodies | non-Hodgkin's B cell lymphoma |
| Novopharm | Gliomab-H (Monoclonals - Humanized Abs) | Brain, melanomas, neuroblastomas |
| Novopharm Biotech, Inc. | 4B5 anti-idiotype Ab | Melanoma, small-cell lung |
| Novopharm Biotech, Inc. | Monopharm-C | Colon, lung, pancreatic |
| Novopharm Biotech, Inc. | NovoMAb-G2 (pancarcinoma specific Ab) | Cancer |
| Procyon Biopharma, Inc. | ANA Ab | Cancer |
| Protein Design Labs | SMART 1D10 Ab | B-cell lymphoma |
| Protein Design Labs | SMART M195 Ab, humanized | Acute myleoid leukemia |
| Protein Design Labs | SMART M195 Ab, humanized | Acute promyelocytic leukemia |
| Protein Design Labs | Zenapax (SMART Anti-Tac (IL-2 receptor) Ab, humanized) | Leukemia, lymphoma |
| Protein Design Labs/Novartis | SMART ABL 364 Ab | Breast, lung, colon |
| Techniclone Corporation/ Cambridge Antibody Technology | TNT (chimeric MAb to histone antigens) | Brain |
| Techniclone Corporation/Cambridge Antibody Technology | ¹³¹I LYM-1 (Oncolym™) | non-Hodgkin's lymphoma |
| Techniclone International/ Cambridge Antibody Technology | TNT (chimeric MAb to histone antigens) | Brain |
| Techniclone International/Alpha Therapeutics | Oncolym (Lym-1 monoclonal antibody linked to ¹³¹I) | non-Hodgkin's lymphoma |

Cancer vaccines are medicaments which are intended to stimulate an endogenous immune response against cancer cells. Currently produced vaccines predominantly activate the humoral immune system (i.e., the antibody-dependent immune response). Other vaccines currently in development are focused on activating the cell-mediated immune system including cytotoxic T lymphocytes which are capable of killing tumor cells. Cancer vaccines generally enhance the presentation of cancer antigens to both APCs (e.g., macrophages and dendritic cells) and/or to other immune cells such as T cells, B cells, and NK cells.

Although cancer vaccines may take one of several forms, as discussed below, their purpose is to deliver cancer antigens and/or cancer-associated antigens to APCs in order to facilitate the endogenous processing of such antigens by APC and the ultimate presentation of antigen presentation on the cell surface in the context ofMHC class I molecules. One form of cancer vaccine is a whole-cell vaccine which is a preparation of cancer cells which have been removed from a subject, treated ex vivo and then reintroduced as whole cells in the subject. Lysates of tumor cells can also be used as cancer vaccines to elicit an immune response. Another form cancer vaccine is a peptide vaccine which uses cancer-specific or cancer-associated small proteins to activate T cells. Cancer-associated proteins are proteins which are not exclusively expressed by cancer cells (i.e., other normal cells may still express these antigens). However, the expression of cancer-associated antigens is generally consistently upregulated with cancers of a particular type.

Yet another form of cancer vaccine is a dendritic cell vaccine which includes whole dendritic cells which have been exposed to a cancer antigen or a cancer-associated antigen in vitro. Lysates or membrane fractions of dendritic cells may also be used as cancer vaccines. Dendritic cell vaccines are able to activate APCs directly. Other cancer vaccines include ganglioside vaccines, heat-shock protein vaccines, viral and bacterial vaccines, and nucleic acid vaccines.

The use of CpG-like nucleic acids in conjunction with cancer vaccines provides an improved antigen-specific humoral and cell-mediated immune response, in addition to activating NK cells and endogenous dendritic cells, and increasing IFN-α, levels. This enhancement allows a vaccine with a reduced antigen dose to be used to achieve the same beneficial effect as would be achieved using a higher antigen dose without the CpG-like nucleic acid. In some instances, cancer vaccines may be used along with adjuvants, such as those described above.

Other vaccines take the form of dendritic cells which have been exposed to cancer antigens in vitro, have processed the antigens and are able to express the cancer antigens at their cell surface in the context of MHC molecules for effective antigen presentation to other immune system cells.

The CpG-like nucleic acids are used in one aspect of the invention in conjunction with cancer vaccines which are dendritic cell-based. A dendritic cell is a professional antigen-presenting cell. Dendritic cells form a link between the innate and the acquired immune system by presenting antigens and through their expression of pattern recognition receptors which detect microbial molecules like lipopolysaccharide (LPS) in their local environment. Dendritic cells efficiently internalize, process, and present soluble specific antigen to which they are exposed. The process of internalizing and presenting antigen causes rapid upregulation of the expression of major histocompatibility complex (MHC) and costimulatory molecules, the production of cytokines, and migration toward lymphatic organs where they are believed to be involved in the activation of T cells.

Table 3 lists a variety of cancer vaccines which are either currently being used or are in development.

**Table 3. Cancer Vaccines in Development or on the Market**

| **MARKETER** | **BRAND NAME (GENERIC NAME)** | **INDICATION** |
|---|---|---|
| Center of Molecular Immunology | EGF | Cancer |
| Center of Molecular Immunology | | Ganglioside cancer vaccine |
| Center of Molecular Immunology | Anti-idiotypic | Cancer vaccine |
| ImClone Systems/Memorial Sloan-Kettering Cancer Center | gp75 antigen | Melanoma |
| ImClone Systems/Memorial Sloan-Kettering Cancer Center | Anti-idiotypic Abs | Cancer vaccines |
| Progenies Pharmaceuticals, Inc. | GMK melanoma vaccine | Melanoma |
| Progenies Pharmaceuticals, Inc, | MGV ganglioside conjugate vaccine | Lymphoma, colorectal, lung |
| Corixa | Her2/neu | Breast, ovarian |
| AltaRex | Ovarex | Ovarian |
| AVAX Technologies Inc. | M-Vax, autologous whole cell | Melanoma |
| AVAX Technologies Inc. | O-Vax, autologous whole cell | Ovarian |
| AVAX Technologies Inc. | L-Vax, autologous whole cell | Leukemia-AML |
| Biomira Inc./Chiron | Theratope, STn-KLH | Breast, Colorectal |
| Biomira Inc. | BLP25, MUC-1 peptide vaccine encapsulated in liposomal delivery system | Lung |
| Biomira Inc. | BLP25, MUC-1 peptide vaccine encapsulated in liposomal delivery system + Liposomal IL-2 | Lung |
| Biomira Inc. | Liposomal idiotypic vaccine | Lymphoma B-cell malignancies |
| Ribi Immunochem | Melacine, cell lysate | Melanoma |
| Corixa | Peptide antigens, microsphere delivery sysem and LeIF adjuvant | Breast |
| Corixa | Peptide antigens, microsphere delivery sysem and LeIF adjuvant | Prostate |
| Corixa | Peptide antigens, microsphere delivery sysem and LeIF adjuvant | Ovarian |
| Corixa | Peptide antigens, microsphere delivery sysem and LeIF adjuvant | Lymphoma |
| Corixa | Peptide antigens, microsphere delivery sysem and LeIF adjuvant | Lung |
| Virus Research Institute | Toxin/antigen recombinant delivery system | All cancers |
| Apollon Inc. | Genevax-TCR | T-cell lymphoma |
| Bavarian Nordic Research Institute A/S | MVA-based (vaccinia virus) vaccine | Melanoma |
| BioChem Pharma/BioChem Vaccine | PACIS, BCG vaccine | Bladder |
| Cantab Pharmaceuticals | TA-HPV | Cervical |
| Cantab Pharmaceuticals | TA-CIN | Cervical |
| Cantab Pharmaceuticals | DISC-Virus, immunotherapy | Cancer |
| Pasteur Merieux Connaught | ImmuCyst®/TheraCys® - BCG Immunotherapeutic (Bacillus Calmette-Guerin/Connaught), for intravesical treatment of superficial bladder cancer | Bladder |

As used herein, chemotherapeutic agents (and, equivalently, chemotherapy agents) embrace all other forms of cancer medicaments which do not fall into the categories of immunotherapeutic agents or cancer vaccines. Chemotherapeutic agents as used herein encompass both chemical and biological agents. These agents function to inhibit a cellular activity upon which the cancer cell is dependent for continued survival. Categories of chemotherapeutic agents include alkylating/alkaloid agents, antimetabolites, hormones or hormone analogs, and miscellaneous antineoplastic drugs. Most if not all of these agents are directly toxic to cancer cells and do not require immune stimulation. Combination chemotherapy and immunostimulatory nucleic acid administration increases the maximum tolerable dose of chemotherapy

A "subject undergoing or at risk of undergoing chemotherapy" is a subject under active treament with a chemotherapy agent or a subject that has or is at risk of developing an indication for treatment with a chemotherapy agent. A subject undergoing chemotherapy includes a subect that is currently receiving or has just recently received a dose or course of chemotherapy. Such a subject can have or be at risk of developing an immunodeficiency. A subject at risk of undergoing chemotherapy includes a subj ect that is about to receive a first or subsequent dose or round of chemotherapy. Such a subject can have an immunodeficiency or, more commonly, is at risk of developing an immunodeficiency.

An immunodeficiency can involve a reduced number of, or a reduced capacity to generate, fully functional immune cells of at least one class, e.g., CD4+ T cells, B cells, NK cells, monocytes, macrophages. A subject that has an immunodeficiency is a subject with reduced capacity to mount an effective immune response. Such subjects may have, for example, an immune system that is immature; an immune system that is suppressed in association with exposure to certain pharmacological agents or irradiation; or an immune system that is suppressed in association with a chromosomal defect, a hereditary or inborn metabolic defect or enzyme deficiency, an antibody deficiency, a defect in the ability of T cells to process and/or present antigen, a nutritional deficiency, an infection that directly affects cells the immune system (e.g., HIV), or a neoplasm. These and other examples of conditions that cause a subject to be immunocompromised can be found in Harrison's Principles of Internal Medicine, 14th Ed., Fauci AS et al., eds., McGraw-Hill, New York, 1998.

Thus other types of chemotherapeutic agents which can be used according to the invention include Aminoglutethimide, Amsacrine (m-AMSA), Asparaginase, Azacitidine, Busulfan, Carboplatin, Chlorombucil, Cisplatin, Cyclophosphamide, Cytarabine HCl, Dactinomycin, Daunorubicin HCl, Erythropoietin, Estramustine phosphate sodium, Etoposide (VP 16-213), Floxuridine, Fluorouracil (5-FU), Flutamide, Hexamethylmelamine (HMM), Hydroxyurea (hydroxycarbamide), Ifosfamide, Leuprolide acetate (LHRH-releasing factor analogue), Lomustine (CCNU), Mechlorethamine HCl (nitrogen mustard), Mercaptopurine, Mesna, Mitoguazone (methyl-GAG; methyl glyoxal bis-guanylhydrazone; MGBG), Mitotane (o,p'-DDD), Mitoxantrone HCl, Octreotide, Paclitaxel, Pentostatin (2'deoxycoformycin), Plicamycin, Procarbazine HCl, Semustine (methyl-CCNU), Streptozotocin, Tamoxifen citrate, Teniposide (VM-26), Thioguanine, Thiotepa, Vinblastine sulfate, and Vindesine sulfate.

Chemotherapeutic agents which are currently in development or in use in a clinical setting are shown in Table 4.

**Table 4. Cancer Drugs in Development or on the Market**

| **MARKETER** | **BRAND NAME** | **GENERIC NAME** | **INDICATION** |
|---|---|---|---|
| Abbott | TNP 470/AGM 1470 | Fragyline | Anti-Angiogenesis in Cancer |
| Agouron | AG2037 | AG2037 | Solid Tumors |
| Agouron | AG3340 | AG3340 | Solid Tumors/Macular Degeneration |
| Agouron | Cdk4/cdk2 inhibitors | cdk4/cdk2 inhibitors | Solid Tumors |
| Agouron | PARP inhibitors | PARP Inhibitors | Solid Tumors |
| Agouron | VEGF/b-FGF Inhibitors | VEGF/b-FGF Inhibitors | Solid Tumors |
| AHP | Novantrone | Mitoxantrone | Cancer |
| Asta Medica | Ifex/Mesnex | Ifosamide | Solid Tumors |
| BASF | LU 103793 | Dolastain | Oncology |
| BASF | LU 79553 | Bis-Naphtalimide | Oncology |
| Bayer | BAY 12-9566 | BAY 12-9566 | Cancer |
| Biochem Pharma | BCH-4556 | BCH-4556 | Solid Tumors |
| Bristol-Meyers Squibb | Ifex/Mesnex | Ifosamide | Solid Tumors |
| Bristol-Myers Squibb | BMS-182751 | Oral Platinum | Cancer (Lung, Ovarian) |
| Bristol-Myers Squibb | Paraplatin | Carboplatin | Solid Tumors |
| Bristol-Myers Squibb | Plantinol | Cisplatin, Stealth | Solid Tumors |
| Bristol-Myers Squibb | Plantinol | Cisplatin | Solid Tumors |
| Bristol-Myers Squibb | RAS Famesyl Transferase Inhibitor | RAS FamesylTransferase Inhibitor | Cancer |
| Bristol-Myers Squibb | Taxane Analog | Taxane Analog | Taxol follow up |
| Bristol-Myers Squibb | Taxol | Paclitaxel | Breast Cancer Advanced, Ovarian Cancer Advanced, NSCLC |
| Bristol-Myers Squibb | UFT (Tegafur/Uracil) | UFT (Tegafur/LJracil) | Cancer Oral |
| Bristol-Myers Squibb | UFT (Tegafur/Uracil) | UFT (Tegafur/Uracil) | Solid Tumors |
| Bristol-Myers Squibb | Vepeside | Etoposide | Solid Tumors Melanoma |
| Bristol-Myers Squibb | Vumon | Teniposide | Solid Tumors |
| British Biotech | Batimastat | Batimastat (BB94) | Pterygium |
| British Biotech | Marimastat | Marimastat (BB 2516) | Solid Tumors |
| Celltech | CDP 845 | Aggrecanase Inhibitor | Solid Tumors / Breast Cancer |
| Chiron | DepoCyt | DepoCyt | Neoplastic Meningitis |
| Chiroscience | D2163 | D2163 | Solid Tumors / Metastases |
| Chiroscience | D4809 | Dexifosamide | Solid Tumors |
| Chugai | Picibanil | OK-432 | Antimalignant Tumor |
| Chugai | Taxotere | Docetaxel | Solid Tumors, Breast Cancer |
| Daiichi | DX8951f | DX8951 | Anti-Cancer |
| Daiichi | Lemonal DP 2202 | Lemonal DP 2202 | Anti-Cancer |
| Eisai | E 7070 | E 7070 | Solid Tumors |
| Fujisawa | FK 317 | FK 317 | Anticancer Antibiotic |
| Genentech | Anti-VEGF | Anti-VEGF | Prostate / Breast / Colorectal / NSCL Cancer |
| Glaxo Wellcome | Eniluracil/776C85 | 5FU Enhancer | Cancer, Refractory Solid & Colorectal Cancer |
| Glaxo Wellcome | Prodrug of guanine arabinside | prodrug of arabinside | T Cell Leukemia/Lymphoma & B Cell Neoplasm |
| Hoechst | HMR 1275 | Flavopiridol | Cancer |
| IDEC Pharma | 9-AC | 9-AC | Solid Tumors |
| Immunex | Novantrone | Mitoxantrone | Pain related to hormone-refractory prostate cancer. |
| Ivax | Cyclopax | Paclitaxel, Oral | Breast/Ovarian Cancer |
| Ivax | Paxene | Paclitaxel | Kaposi Sarcoma |
| Johnson & Johnson | Ergamisol | Levamisole | Cancer Therapy |
| Johnson & Johnson | Ergamisol | Levamisole | Colon Cancer |
| Johnson & Johnson | Leustain | Cladribine | Hairy Cell Leukaemia |
| Lilly | Gemzar | Gemcitabine | Pancreatic Cancer, Non Small Cell Lung Cancer, Breast, Bladder and Ovarian |
| Lilly | LY 264618/Lometexol | Lometexol | Cancer Solid Tumors |
| Lilly | MTA/LY 231514 | MTA/LY 231514 | Cancer Solid Tumors |
| Liposome | Evacet | Doxorubicin, Liposomal | Breast Cancer |
| Medeva | Valstar | Valrubicin | Bladder Cancer - Refractory in situ carcinoma |
| Medeva | Valstar | Valrubicin | Bladder Cancer - Papillary Cancer |
| Merck | Famesyl Transferase Inhibitor | Famesyl Transferase Inhibitor | Cancer (Solid tumors -pancrease, colon, lung, breast) |
| Novartis | ISI 641 | ISI 641 | Solid Tumors |
| Novartis | MMI 270 | MMI 270 | Cancer |
| Novartis | ODN 698 | ODN 698 | Solid Tumors |
| Novartis | PKC 412 | PKC 412 | Multi-Drug Resistant Cancer |
| Novartis | Valspodar | PSC 833 | Myeloid Leukemia/Ovarian Cancer |
| Nycomed Amersham | AD 32/valrubicin | Valrubicin | Bladder Cancer-Refractory , In situ Carcinoma |
| Nycomed Amersham | Metastron | Strontium Derivative | Bone Cancer (adjunt therapy, Pain) |
| Nycomed Amersham | Seeds/I-125 Rapid St | Lodine Seeds | Prostate Cancer |
| Nycomed Amersham | Yewtaxan | Paclitaxel | Breast Cancer Advanced, Ovarian Cancer Advanced |
| Pfizer | CP-358, 774 | EGFR | Cancer |
| Pfizer | CP-609, 754 | RAS Oncogene Inhibitor | Cancer |
| Pfizer | PFE | MMP | Cancer, angiogenesis |
| Pfizer | PFE | Tyrosine Kinase | Cancer, angiogenesis |
| Pharmacia & Upjohn | Adriamycin | Doxorubicin | Breast Cancer, Leukemia |
| Pharmacia & Upjohn | Camptosar | Irinotecan | Colorectal Cancer, Cervical Cancer |
| Pharmacia & Upjohn | Pharmorubicin | Epirubicin | Lung/Breast Cancer |
| Rhone Poulenc | Campto | Irinotecan | Colorectal Cancer, Cervical Cancer |
| Rhone Poulenc | Gliadel Wafer | Carmustaine + Polifepr Osan | Brain Tumor |
| Rhone Poulenc | Oral Taxoid | Oral Taxoid | Broad Cancer |
| Rhone Poulenc | Taxotere | Docetaxel | Solid Tumors, Breast Cancer |
| Roche | Furtulon | Doxifluridine | Breast Cancer, Colorectal Cancer, Gastric Cancer |
| Roche | Xeloda | Capecitabine | Breast Cancer, Colorectal Cancer |
| Sankyo | CS-682 | CS-682 | Solid Tumors |
| Sankyo | Krestin | Krestin | Solid Tumors |
| Schering AG | Angiogenesis inhibitor | Angiogenesis Inhibitor | Cancer / Cardio |
| Schering AG | Fludara | Fludarabine | Leukaemia |
| Schering Plough | Temodal | Temozolomide | Brain Tumours |
| Schering Plough | Temodal | Temozolonide | Brain Tumours |
| Scotia | Glamolec | LiGLA (lithium-gamma linolenate) | Cancer, pancreatic, breast, colon |
| Scotia | Meglamine GLA | Meglamine GLA | Bladder Cancer |
| Sequus | Caelyx | Doxorubicin, Liposomal | KS/Cancer |
| Sequus | Doxil | Doxorubicin, Liposomal | KS/Cancer |
| Sequus | SPI-077 | Cisplatin, Stealth | Cancer |
| Shering Plough | Caetyx | Doxorubicin-Liposome | Ovarian/Breast Cancer |
| Smithkline Beecham | Hycamtin | Topotecan | Metastatic Ovarian Cancer |
| Takeda | TNP 470/AGM 1470 | Fragyline | Anti-Angiogenesis in Cancer |
| Takeda | TNP-470 | n/k | Malignant Tumor |
| Tanube Seiyaku | TA 2516 | Marimistat | Solid Tumors |
| Vertex | Incel | VY-710 | Solid Tumors - IV Solid Tumors - Oral |
| Vertex | VX-853 | VX-853 | |
| Warner Lambert | CI-994 | CI-994 | Cancer, Solid Tumors/Leukemia |
| Warner Lambert | Metaret | Suramin | Prostate |
| Warner Lambert | PD 183805 | PD 183805 | |
| Warner Lambert | Undisclosed Cancer (b) | Undisclosed Cancer (b) | Cancer |
| Yamanouchi | YM 116 | YM116 | Prostate Cancer |
| Zeneca | Tomudex | Ralitrexed | Colorectal Cancer, Lung Cancer, Breast Cancer |
| Zeneca | ZD 0101 (inj) | ZD 0101 | Solid Tumors |
| Zeneca | ZD 1839 | ZD 1839 | Non Small Cell Lung Cancer, Pancreatic Cancer |
| Zeneca | ZD 9331 | ZD 9331 | Solid Tumors, Advanced Colorectal |

In one embodiment, the methods of the invention use CpG-like nucleic acids as a replacement to the use of IFN-α therapy in the treatment of cancer. In other embodiments the methods of the invention use CpG-like nucleic acids as a supplement to the use of IFN-α therapy in the treatment of cancer. Currently, some treatment protocols call for the use of IFN-α. Since IFN-α is produced following the administration of some immunostimulatory nucleic acids, these nucleic acids can be used to generate IFN-α endogenously.

In certain embodiments the invention also provides methods for treating a subject that has or is at risk of developing anemia, thrombocytopenia, or neutropenia. The methods involve administering to a subject that has or is at risk of developing anemia, thrombocytopenia, or neutropenia a CpG-like nucleic acid in an amount effective for enhancing erythropoiesis, thrombopoiesis, or neutrophil proliferation, respectively. In other words, according to these methods, the administration of CpG-like nucleic acid to the subject promotes the generation of new erythrocytes (red blood cells), platelets, or neutrophils, respectively.

Anemia is a deficiency of circulating red blood cells (erythrocytes). A "subj ect at risk of developing anemia" as used herein is a subject who has any risk of exposure to an agent associated with suppression of formation of erythrocytes or their progenitors, risk of loss of erythrocytes associated with surgery or injury, or risk of developing anemia by some other mechanism involving diminished production, accelerated destruction, and/or sequestration of erythrocytes, e.g., autoimmune destruction of erythrocytes, thalassemia, and renal insufficiency. A "subject that has anemia" is a subject that has a reduced number of circulating erythrocytes.

Thrombocytopenia is a deficiency of circulating platelets. A "subject at risk of developing thrombocytopenia" as used herein is a subject who has any risk of exposure to an agent associated with suppression of formation of platelets or their progenitors, risk of loss of platelets associated with surgery or injury, or risk of developing thrombocytopenia by some other mechanism involving diminished production, accelerated destruction, and/or sequestration of platelets, e.g., autoimmune destruction of platelets. A "subject that has thrombocytopenia" is a subject that has a reduced number of circulating platelets.

Neutropenia is a deficiency of circulating neutrophils. A "subject at risk of developing neutropenia" as used herein is a subject who has any risk of exposure to an agent associated with suppression of formation of neutrophils (also known as granulocytes, polymorphonuclear leukocytes, PMNs) or their progenitors, risk of loss of neutrophils associated with infection or peripheral pooling or periphearal destruction following exposure to drugs that serve as immune haptens (e.g., α-methyl dopa), or risk of developing or neutropenia by some other mechanism involving diminished production, accelerated destruction, and/or sequestration of neutrophils, e.g., autoimmune destruction of neutrophils. A "subject that has neutropenia" is a subject that has a reduced number of circulating neutrophils.

The CpG-like nucleic acids are useful according to these embodiments of the invention as a prophylactic for the treatment of a subject at risk of developing anemia, thrombocytopenia, or neutropenia. For example, the CpG-like nucleic acids can be administered to a subject where it is anticipated that the subject will be exposed to conditions associated with development of anemia, thrombocytopenia, or neutropenia. Alternatively, the CpG-like nucleic acids can be administered to a subject where it is known or suspected that the subject is predisposed to develop anemia, thrombocytopenia, or neutropenia.

In addition to the use of the CpG-like nucleic acid and the anemia, thrombocytopenia, or neutropenia medicament for prophylactic treatment, the invention also encompasses the use of the combination of drugs for the treatment of a subject having anemia, thrombocytopenia, or neutropenia.

Anemia, thrombocytopenia, and neutropenia are frequently defined in terms of laboratory measurements indicating a reduced hematocrit (volume percent), a reduced platelet count (per mm³), and a reduced neutrophil count (per mm³), respectively. Methods of determining these values are well known in the art, including automated as well as manual methods. The lower limits of normal for hematocrits and platelet counts in healthy nonpregnant humans is somewhat variable, depending on the age and sex of the subject, method of determination, and the norms for the laboratory performing the mesurements. Generally, however, an adult human subject is said to have anemia when the hematocrit is less than about 37-40%. Likewise, generally an adult human subject is said to have thrombocytopenia when the platelet count is below about 100,000 per mm³. Anemia is also frequently reported in terms of a reduced hemoglobin (g/dL) or red blood cell count (per mm³). Typical lower limits of normal values for these in healthy adult humans are 12-13 g/dL and about 4.1 x 10⁶ per mm³, respectively. Generally an adult human subject is said to have neutropenia when the neutrophil count falls below 1000 per mm³. Corresponding values for all these parameters can differ but are readily ascertainable for other species.

Anemia, thrombocytopenia, and neutropenia are also frequently associated with clinical signs and symptoms in relation to their degree of severity. Anemia may be manifested as pallor, generalized fatigue or weakness, reduced exercise tolerance, shortness of breath with exertion, rapid heart rate, irregular heart rhythm, chest pain (angina), congestive heart failure, and headache. Thrombocytopenia is typically manifested in terms of spontaneous or uncontrolled bleeding, petechiae, and easy bruising. Neutropenia is associated with infections, including notably infections from endogenous microbial flora, and lack of inflammation.

The CpG-like nucleic acids may also be formulated with or administered to a subject in conjunction with an anemia medicament. An "anemia medicament" as used herein is a composition of matter which reduces the symptoms related to anemia, prevents the development of anemia, or treats existing anemia. In certain embodiments the anemia medicament is selected from the group consisting of recombinant erythropoietin (EPO), recombinant granulocyte-macrophage colony-stimulating factor (GM-CSF), recombinant granulocyte colony-stimulating factor (G-CSF), recombinant IL-11, ferrous iron, ferric iron, vitamin B12, vitamin B6, vitamin C, vitamin D, calcitriol, alphacalcidol, folate, androgen, and carnitine. In a preferred embodiment the anemia medicament is recombinant EPO. Commonly used anemia drugs which are currently on the market or in development include recombinant human EPO (EPOGEN; PROCRIT), preparations of iron (ferrous and ferric, CHROMAGEN; FEOSOL; INFED; IROSPAN; NEPHRO-FER; NEPHRO-VITE; NIFEREX; NU-IRON; SLOW FE), vitamin B12, vitamin B6, folic acid (CHROMAGEN; FERRO-FOLIC; NEPHRO-FER; NIFEREX), ascorbic acid, certain metabolites of vitamin D (calcitriol and alphacalcidol; CALCIJEX; ROCALTROL), androgens, anabolic steroids (ANADROL), carnitine, recombinant IL- 11 (NEUMEGA), and G-CSF (NEUPOGEN). In a preferred embodiment the anenia medicament is recombinant EPO.

The CpG-like nucleic acids may also be formulated with or administered to a subject in conjunction with a thrombocytopenia medicament. A "thrombocytopenia medicament" as used herein is a composition of matter which reduces the symptoms related to thrombocytopenia, prevents the development of thrombocytopenia, or treats existing thrombocytopenia. In certain embodiments the thrombocytopenia medicament is selected from the group consisting of a glucocorticoid, recombinant thrombopoietin (TPO), recombinant megakaryocyte growth and development factor (MGDF), pegylated recombinant MGDF, lisophylline, recombinant IL-1, recombinant IL-3, recombinant IL-6, and recombinant IL-11. In a preferred embodiment the thrombocytopenia medicament is recombinant TPO. Drugs in common usage or development for the treatment of thrombocytopenia include glucocorticoids (prednisolone; prednisone; methylprednisolone; SOLUMEDROL), recombinant TPO, recombinant MGDF, pegylated recombinant MGDF, lisophylline, recombinant IL-1, recombinant IL-3, recombinant IL-6, recombinant IL- 11 (NEUMEGA), and recombinant G-CSF (NEUPOGEN). In a preferred embodiment the thrombocytopenia medicament is recombinant TPO.

The CpG-like nucleic acids may also be formulated with or administered to a subject in conjunction with a neutropenia medicament. A "neutropenia medicament" as used herein is a composition of matter which reduces the symptoms related to neutropenia, prevents the development of neutropenia, or treats existing neutropenia. In certain embodiments the neutropenia medicament is selected from the group consisting of a glucocorticoid, recombinant G-CSF, recombinant GM-CSF, recombinant macrophage colony-stimulating factor (M-CSF), recombinant IL-1, recombinant IL-3, recombinant IL-6, immunoglobulin, androgens, recombinant IFN-γ, small molecule G-CSF mimetics, G-CSF receptor antagonists, IL-3 receptor antagonists, and uteroferrin. In a preferred embodiment the neutropenia medicament is recombinant G-CSF. Drugs in common usage or development for the treatment of neutropenia include glucocorticoids (prednisolone; prednisone; methylprednisolone; SOLUMEDROL), recombinant G-CSF (NEUPOGEN), recombinant GM-CSF (LEUKINE), recombinant M-CSF, recombinant IL-1, recombinant IL-3, recombinant IL-6, immunoglobulin G (SANDOGLOBULIN, IVEEGAM, GAMMAR-P, GAMIMIJNE N, GAMMAGARD S/D), androgens, recombinant IFN-γ (ACTIMMUNE), small molecule G-CSF mimetics, G-CSF receptor antagonists, IL-3 receptor antagonists, and uteroferrin. In a preferred embodiment the neutropenia medicament is recombinant G-CSF. Antibiotics are frequently adminstered in association with neutropenia medicaments to treat or reduce the risk of infection.

The anemia, thrombocytopenia, or neutropenia medicaments useful in combination with the CpG-like nucleic acids include steroids, inducers of steroids, and immunomodulators.

The steroids include, but are not limited to, systemically administered corticosteroids including methylprednisolone, prednisolone and prednisone, cortisone, and hydrocortisone. Inducers of steroids include, but are not limited to adrenocorticotropic hormone (ACTH).

Corticosteroids inhibit cytokine production, adhesion protein activation, and inflammatory cell migration and activation. The side effects associated with systemic corticosteroids include, for instance, reversible abnormalities in glucose metabolism, increased appetite, fluid retention, weight gain, mood alteration, hypertension, peptic ulcer, and asceptic necrosis of bone. Some side effects associated with longer term use include adrenal axis suppression, growth suppression, dermal thinning, hypertension, diabetes mellitus, Cushing's syndrome, cataracts, muscle weakness, and in rare instances, impaired immune function. It is recommended that these types of compounds be used at their lowest effective dose.

The invention also includes in yet other aspects methods for inducing antigen non-specific innate immune activation and broad-spectrum resistance to infectious challenge using the CpG-like nucleic acids. The term "antigen non-specific innate immune activation" as used herein refers to the activation of immune cells other than B cells and for instance can include the activation ofNK cells, T cells or other immune cells that can respond in an antigen-independent fashion, or some combination of these cells. A broad spectrum resistance to infectious challenge is induced because the immune cells are in active form and are primed to respond to any invading compound or microorganism. The cells do not have to be specifically primed against a particular antigen. This is particularly useful in biowarfare, and the other circumstances described above such as travelers.

CpG-like nucleic acids are effective in non-rodent vertebrates. Different CpG-like nucleic acids can cause optimal immune stimulation depending on the type of subject and the sequence of the CpG-like nucleic acid. Many vertebrates have been found according to the invention to be responsive to the same class of CpG-like nucleic acids, sometimes referred to as human-specific CpG-like nucleic acids. Rodents, however, respond to different nucleic acids. Thus a CpG-like nucleic acid causing optimal stimulation in humans may not generally cause optimal stimulation in a mouse, and vice versa. A CpG-like nucleic acid causing optimal stimulation in humans often does, however, cause optimal stimulation in other animals such as cows, horses, sheep, etc. One of skill in the art can identify the optimal nucleic acid sequences useful for a particular species of interest using routine assays described herein and/or known in the art, using the guidance supplied herein.

The CpG-like nucleic acids may be directly administered to the subject or may be administered in conjunction with a nucleic acid delivery complex. A "nucleic acid delivery complex" shall mean a nucleic acid molecule associated with (e.g., ionically or covalently bound to; or encapsulated within) a targeting means (e.g., a molecule that results in higher affinity binding to target cell (e.g., B cell surfaces and/or increased cellular uptake by target cells). Examples of nucleic acid delivery complexes include nucleic acids associated with a sterol (e.g., cholesterol), a lipid (e.g., a cationic lipid, virosome or liposome), or a target cell-specific binding agent (e.g., a ligand recognized by target cell-specific receptor). Preferred complexes may be sufficiently stable in vivo to prevent significant uncoupling prior to internalization by the target cell. However, the complex can be cleavable under appropriate conditions within the cell so that the nucleic acid is released in a functional form. In certain embodiments the CpG-like nucleic acid can be conjugated with an antigen through a covalent bond.

Delivery vehicles or delivery devices for delivering antigen and nucleic acids to surfaces have been described. The CpG-like nucleic acid and/or the antigen and/or other therapeutics may be administered alone (e.g., in saline or buffer) or using any delivery vehicles known in the art. For instance the following delivery vehicles have been described: Cochleates (Gould-Fogerite et al., 1994, 1996); Emulsomes (Vancott et al., 1998, Lowell et al., 1997); ISCOMs (Mowat et al., 1993, Carlsson et al., 1991, Hu et., 1998, Morein et al., 1999); Liposomes (Childers et al., 1999, Michalek et al., 1989, 1992, de Haan 1995a, 1995b); Live bacterial vectors (e.g., *Salmonella, Escherichia coli, Bacillus calmatte-guerin, Shigella, Lactobacillus*) (Hone et al., 1996, Pouwels et al., 1998, Chatfield et al., 1993, Stover et al., 1991, Nugent et al., 1998); Live viral vectors (e.g., Vaccinia, adenovirus, Herpes Simplex) (Gallichan et al., 1993, 1995, Moss et al., 1996, Nugent et al., 1998, Flexner et al., 1988, Morrow et al., 1999); Microspheres (Gupta et al., 1998, Jones et al., 1996, Maloy et al., 1994, Moore et al., 1995, O'Hagan et al., 1994, Eldridge et al., 1989); Nucleic acid vaccines (Fynan et al., 1993, Kuklin et al., 1997, Sasaki et al., 1998, Okada et al., 1997, Ishii et al., 1997); Polymers (e.g., carboxymethylcellulose, chitosan) (Hamajima et al., 1998, Jabbal-Gill et al., 1998); Polymer rings (Wyatt et al., 1998); Proteosomes (Vancott et al., 1998, Lowell et al., 1988, 1996, 1997); Sodium Fluoride (Hashi et al., 1998); Transgenic plants (Tacket et al., 1998, Mason et al., 1998, Haq et al., 1995); Virosomes (Gluck et al., 1992, Mengiardi et al., 1995, Cryz et al., 1998); Virus-like particles (Jiang et al., 1999, Leibl et al., 1998). Other delivery vehicles are known in the art and some additional examples are provided above in the discussion of vectors.

The language "effective amount" of a nucleic acid molecule refers to the amount necessary or sufficient to realize a desired biologic effect. For example, an effective amount of a nucleic acid containing at least one CpG-like nucleic acid for treating an immune system deficiency could be that amount necessary to eliminate a tumor, cancer, or bacterial, viral or fungal or parasitic infection. An effective amount for use as a vaccine adjuvant could be that amount useful for boosting a subject's immune response to a vaccine. An effective amount for treating asthma can be that amount useful for redirecting a Th2 type of immune response that is associated with asthma to a Th1 type of response. The effective amount for any particular application can vary depending on such factors as the disease or condition being treated, the particular nucleic acid being administered (e.g., the number of unmethylated CpG motifs or their location in the nucleic acid), the size of the subject, or the severity of the disease or condition. One of ordinary skill in the art can empirically determine the effective amount of a particular oligonucleotide without necessitating undue experimentation. For any compound described herein a therapeutically effective amount can be initially determined from in vivo animal models or in vitro by comparison with oligonucleotides known to produce an immune response.

The phrase "therapeutically effective amount" means that amount of a compound which prevents the onset of, alleviates the symptoms of, or stops the progression of a disorder or disease being treated.

Subject doses of the compounds described herein for mucosal or local delivery typically range from about 0.1 µg to 10 mg per administration, which depending on the application could be given daily, weekly, or monthly and any other amount of time therebetween. More typically mucosal or local doses range from about 10 µg to 5 mg per administration, and most typically from about 100 µg to 1 mg, with 2 - 4 administrations being spaced days or weeks apart. More typically, immune stimulant doses range from 1 µg to 10 mg per administration, and most typically 10 µg to 1 mg, with daily or weekly administrations. Subject doses of the compounds described herein for parenteral delivery for the purpose of inducing an antigen-specific immune response, wherein the compounds are delivered with an antigen but not another therapeutic agent, are typically 5 to 10,000 times higher than the effective mucosal dose for vaccine adjuvant or immune stimulant applications, and more typically 10 to 1,000 times higher, and most typically 20 to 100 times higher. Doses of the compounds described herein for parenteral delivery for the purpose of inducing an innate immune response or for increasing ADCC or for inducing an antigen-specific immune response when the CpG-like nucleic acids are administered in combination with other therapeutic agents or in specialized delivery vehicles typically range from about 0.1 µg to 10 mg per administration, which depending on the application could be given daily, weekly, or monthly and any other amount of time therebetween. More typically parenteral doses for these purposes range from about 10 µg to 5 mg per administration, and most typically from about 100 µg to 1 mg, with 2 - 4 administrations being spaced days or weeks apart. In some embodiments, however, parenteral doses for these purposes may be used in a range of 5 to 10,000 times higher than the typical doses described above.

For any compound described herein the therapeutically effective amount can be initially determined from animal models. A therapeutically effective dose can also be determined from human data for CpG oligonucleotides which have been tested in humans (human clinical trials have been initiated) and for compounds which are known to exhibit similar pharmacological activities, such as other mucosal adjuvants, e.g., labile toxin (LT) and other antigens for vaccination purposes, for the mucosal or local administration. Higher doses are required for parenteral administration. The applied dose can be adjusted based on the relative bioavailability and potency of the administered compound. Adjusting the dose to achieve maximal efficacy based on the methods described above and other methods as are well-known in the art is well within the capabilities of the ordinarily skilled artisan.

The formulations of the invention are administered in pharmaceutically acceptable solutions, which may routinely contain pharmaceutically acceptable concentrations of salt, buffering agents, preservatives, compatible carriers, adjuvants, and optionally other therapeutic ingredients.

For use in therapy, an effective amount of the CpG-like nucleic acid can be administered to a subject by any mode that delivers the nucleic acid to the desired surface, e.g., mucosal, systemic. Administering the pharmaceutical composition of the present invention may be accomplished by any means known to the skilled artisan.

Preferred routes of administration include but are not limited to enteral (oral and any route involving absorption from the gastrointestinal tract), parenteral (intravenous, intramuscular, subcutaneous, intradermal, intraperitoneal, intrathecal, direct injection), mucosal (including oral, nasal, rectal, vaginal, transdermal, buccal, sublingual, pulmonary, ocular), and topical.

For oral administration, the compounds (i.e., CpG-like nucleic acids, antigens and other therapeutic agents) can be formulated readily by combining the active compound(s) with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as cross-linked polyvinyl pyrrolidone, agar, or alginic acid or a salt thereof such as sodium alginate. Optionally the oral formulations may also be formulated in saline or buffers for neutralizing internal acid conditions or may be administered without any carriers.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

For administration by inhalation, the compounds for use according to the present invention may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the compound and a suitable powder base such as lactose or starch.

The compounds, when it is desirable to deliver them systemically, may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, e.g., sterile pyrogen-free water, before use.

The compounds may also be formulated in rectal or vaginal compositions such as suppositories or retention enemas, e.g., containing conventional suppository bases such as cocoa butter or other glycerides.

In addition to the formulations described previously, the compounds may also be formulated as a depot preparation. Such long-acting formulations may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer R (1990) Sciellce 249:1527-1533.

The CpG-like nucleic acids and antigens may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: acetic, benzene sulphonic, citric, formic, hydrobromic, hydrochloric, maleic, malonic, methane sulphonic, naphthalene-2-sulphonic, nitric, phosphoric, p-toluene sulphonic, salicylic, succinic, sulfuric, and tartaric. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The pharmaceutical compositions of the invention contain an effective amount of a CpG-like nucleic acid and optionally antigens and/or other therapeutic agents optionally included in a pharmaceutically acceptable carrier. The term "pharmaceutically acceptable carrier" means one or more compatible solid or liquid filler, diluents or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The CpG-like nucleic acids useful in the invention may be delivered in mixtures with additional adjuvant(s), other therapeutics, or antigen(s). A mixture may consist of several adjuvants in addition to the CpG-like nucleic acid or several antigens or other therapeutics.

The CpG-like nucleic acids useful in the invention may be formulated in an amount effective to induce an immune response together with at least one additional adjuvant, other therapeutic agent, or antigen. Examples of adjuvants and of antigens are provided above. With regard to other therapeutic agents which may be formulated with the CpG-like nucleic acids, these include, for example, an anti-cancer medicament, antiviral agent, antibacterial agent, antifungal agent, antiparasitic agent, ulcer medicament, allergy medicament, asthma medicament, anemia medicament, thrombocytopenia medicament, neutropenia medicament, or a cytokine. Examples of each of these other therapeutic agents are provided herein. Preferred cytokines in this context include IL-2, IL-3, IL-4, IL-18, IFN-α, IFN-γ, TNF-α, Flt3 ligand, G-CSF, and GM-CSF. Ulcer medicaments are agents useful in the treament of gastrointestinal ulcers. Ulcer medicaments include antacids, anticholinergic agents, cytoprotective agents, ulcer coating/adherent agents, histamine H₂ receptor antagonists, prostaglandins, and proton pump inhibitors. Non-limiting examples of specific ulcer medicaments include MAALOX, MYLANTA, ROBINUL, CYTOTEC, CARAFATE, AXID, FAMOTIDINE, PEPCID, TAGAMENT, ZANTAC, PRILOSEC, PROTONIX, and PREY ACID,

A variety of administration routes are available. The particular mode selected will depend, of course, upon the particular adjuvants or antigen selected, the particular condition being treated and the dosage required for therapeutic efficacy. The methods of this invention, generally speaking, may be practiced using any mode of administration that is medically acceptable, meaning any mode that produces effective levels of an immune response without causing clinically unacceptable adverse effects. Preferred modes of administration are discussed above.

The compositions may conveniently be presented in unit dosage form and may be prepared by any of the methods well known in the art of pharmacy. The methods may include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product. Liquid dose units are vials or ampoules. Solid dose units are tablets, capsules, lozenges, troches, powders, and suppositories. For treatment of a patient, depending on activity of the compound, manner of administration, purpose of the immunization (i.e., prophylactic or therapeutic), nature and severity of the disorder, age and body weight of the patient, different doses may be necessary. The administration of a given dose can be carried out both by single administration in the form of an individual dose unit or else several smaller dose units. Multiple administration of doses at specific intervals of weeks or months apart is usual for boosting the antigen-specific responses.

Other delivery systems can include time-release, delayed release or sustained release delivery systems. Such systems can avoid repeated administrations of the compounds, increasing convenience to the subject and the physician. Many types of release delivery systems are available and known to those of ordinary skill in the art. They include polymer-based systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono-, di-, and tri-glycerides; hydrogel release systems; silastic systems; peptide-based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

The present invention is further illustrated by the following Examples, which in no way should be construed as further limiting.

### EXAMPLES

### Materials and Methods

*Oligonucleotides.* Phosphorothioate-modified ODN (PS ODN) were purchased from Hybridon Speciality Products (Milford, MA, USA) and ARK Scientific GmbH (Darmstadt, Germany). The sequences used were (all shown 5' to 3' from left to right):

| | | |
|---|---|---|
| 1758 (G3139 Genta) | tctcccagcgtgcgccat | SEQ ID NO:1 |
| 1812 (methylated 1758) | tztzzzagzgtgzgzzat | SEQ ID NO:2 |
| 1982 (non-CpG) | tccaggacttctctcaggtt | SEQ ID NO:3 |
| 2006 | tcgtcgttttgtcgttttgtcgtt | SEQ ID NO:4 |
| 2117 (methylated 2006) | tzgtzgttttgtzgttttgtzgtt | SEQ ID NO:5 |
| 2137 (GpC 2006) | tgctgcttttgtgcttttgtgctt | SEQ ID NO:6 |
| 2186 (DSP30) | tcgtcgctgtctccgcttcttcttgcc | SEQ ID NO:7 |
| 5107 (methylated 2186) | tzgtzgztgtztzzgzttzttzttgzz | SEQ ID NO:8 |
| 5114 (Formivirsen 13312 ISIS) | gcgtttgctcttcttcttgcg | SEQ ID NO:9 |
| 5116 (2302 ISIS) | gcccaagctggcatccgtca | SEQ ID NO:10 |
| 5122 | gtgctcgaggatgcgcttcgc | SEQ ID NO:11 |
| 5126 (non-CpG) | ggttcttttggtccttgtct | SEQ ID NO:12 |
| 5154 (methylated 5114) | gzgtttgztzttzttzttgzg | SEQ ID NO:13 |
| 5155 (methylated 5116) | gzzzaagztggzatzzgtza | SEQ ID NO:14 |
| 5246 (2'-methoxy C 2006 PS) | tcgtcgttttgtcgttttgtcgtt | SEQ ID NO:15 |
| 5247 (2'-methoxy C 1982 PS) | tccaggacttctctcaggtt | SEQ ID NO: 16 |
| 5248 (2'-methoxy C 5126 PS) | ggttcttttggtccttgtct | SEQ ID NO: 17 |
| 5249 (G to I 2006) | tcitcittttitcittttitcitt | SEQ ID NO:18 |
| 5250 (G to I 1982) | tccaiiacttctctcaiitt | SEQ ID NO:19 |
| 5251 (G to I 5126) | iittcttttiitccttitct | SEQ ID NO:20 |
| 5279 (Cp2 2006) | tc2tc2tttt2tc2tttt2.tc2tt | SEQ ID NO:21 |
| 5280 (CpdSp 2006) | tcdtcdttttgtcdttttgtcdtt | SEQ ID NO:22 |
| 5281 (dSppG 2006) | tdgtdgttttgtdgttttgtdgtt | SEQ ID NO:23 |
| 5282 (dSppdSp 2006) | tddtddttttgtddttttgtddtt | SEQ ID NO:24 |
| 5283 (CpdSp 5122) | gtgctcdaggatgcdcttcdc | SEQ ID NO:25 |
| 5284 (dSppG 5122) | gtgctdgaggatgdgcttdgc | SEQ ID NO:26 |
| 5285 (dSppdSp 5122) | gtgctddaggatgddcttddc | SEQ ID NO:27 |
| 5286 (Cp7 2006) | tc7tc7ttttgtc7ttttgtc7tt | SEQ ID NO:28 |
| 5290 (CpW 2006) | tcwtcwttttgtcwttttgtcwtt | SEQ ID NO:29 |
| 5331 (CpI2006) | tcitcittttgtcittttgtcitt | SEQ ID NO:30 |
| 5384 (UpG 2006) | tugtugttttgtugttttgtugtt | SEQ ID NO:31 |
| 5385 (GpG 2006) | tggtggttttgtggttttgtggtt | SEQ ID NO:32 |
| 5386 (CpC 2006) | tcctccttttgtccttttgtcctt | SEQ ID NO:33 |
| 5387 (GpI 2006) | tgitgittttgtgittttgtgitt | SEQ ID NO:34 |
| 5388 (2pG 2006) | t2gt2gttttgt2gttttgt2gtt | SEQ ID NO:35 |
| 5389 (CpM 2006) | tcmtcmttttgtcmttttgtcmtt | SEQ ID NO:36 |

In the methylated ODN 2117, 1812, 5107, 5154, and 5155, z = 5-methyleytidine. ODN 5246-5248 are phosphorothioate backbone ODN incorporating 2'-methoxy modification at the deoxyribose sugar of only the cytosine residues (2'-methoxy C; 2'-OMe-C). In ODN 5280 - 5285, d=dSpacer. In ODN 5249, 5250, 5251, 5331, and 5387, i = inosine. In ODN 5389, m=2,6-diaminopurine. In ODN 5384, u=uracil. In ODN 5290, w=nebularine. In ODN 5279 and 5388, 2=2-aminopurine. IN ODN 5286, 7=7-deaza-guanosine. Some of these ODN are presented in table form below to highlight the relationships between reference and modified ODN (Table 5).

**Table 5. Reference and CpG-like Nucleic Acids**

| REFERENCE ODN | | METHYLATED | | INOSINE | | 2'-OME-C | |
|---|---|---|---|---|---|---|---|
| 2006 | 5'-tcgtcgttttgtcgttttgtcgtt-3' | 2117 | 5'-tzgtzgttttgtzgttttgtzgtt-3' | 5249 | 5'-tcitcittttitcittttitcitt-3' | 5246 | 5'-tcgtcgttttgtcgttttgtcgtt-3' |
| 2137 | 5'-tgctgcttttgtgcttttgtgctt-3' | | | | | | |
| 1758 | 5'-tctcccagcgtgcgccat-3' | 1812 | 5'-tztzzzagzgtgzgzzat-3' | | | | |
| 2186 | 5'-tcgtcgctgtctccgcttcttcttgcc-3' | 5107 | 5'-tzgtzgztgtztzzgzttzttzttgzz-3' | | | | |
| 5114 | 5'-gcgtttgctcttcttcttgcg-3' | 5154 | 5'-gzgtttgztzttzttzttgzg-3' | | | | |
| 5116 | 5'-gcccaagctggcatccgtca-3' | 5155 | 5'-gzzzaagztggzatzzgtza-3' | | | | |
| 1982 | 5'-tccaggacttctctcaggtt-3' | | | 5250 | 5'-tccaiiacttctctcaiitt-3' | 5247 | 5'-tccaggacttctctcaggtt-3' |
| 5126 | 5'-ggttcttttggtccttgtct-3' | | | 5251 | 5'-iittcttttiitccttitct-3' | 5248 | 5'-ggttcttttggtccttgtct-3' |

| | | | | | | | |
|---|---|---|---|---|---|---|---|
| All backbones are phosphorothioate; z = 5-methylcytidine; i = inosine | | | | | | | |

It has been previously reported that 2'-methoxy modifications at the deoxyribose sugar decrease immunostimulation, but enhance the immunostimulatory effect for ODN with 2' modifications at specific sites. Henry S et al. (2000) J Pharmacol Exp Ther 292:468-79; Zhao Q et al. (1999) Bioorg Med Chem Lett 9:3453-8; Zhao Q et al. (2000) Bioorg Med Chem Lett 10:1051-4. Immune stimulation was markedly decreased with oligonucleotides containing the 2'-methoxyethoxy modifications, such that administration of these modified oligonucleotides to mice did not produce splenomegaly even at 50 mg/kg dose. Henry S et al. (2000) J Pharmacol Exp Ther 292:468-79. Substitution of deoxynucleosides in the flanking region of CpG-containing phosphorothioate oligodeoxynucleotides with 2'-O-methylribonucleosides resulted in significant decreases or increases in their immunostimulatory activities. Zhao Q et al. (1999) Bioorg Med Chem Lett 9:3453-8. Immunostimulatory activity of a PS-oligo containing a CpG motif can be modulated by substitution of a single deoxynucleoside at specific sites with either 2'-O-methylribonucleoside or 3'-O-methylribonucleoside in the flanking region to CpG motif. Zhao Q et al. (2000) Bioorg Med Chem Lett 10:1051-4.

ODN were tested for endotoxin (LPS) content using the Limulus amoebocyte lysate (LAL) assay or LAL kinetic assay (BioWhittaker, Belgium; lower detection limit 0.1 endotoxin units (EU)/ml or 0.005EU/ml, respectively) or a method recently published by Hartmann et al. Hartmann G et al. (1999) Gene Therapy 6:893-903. For all assays ODN were diluted in TE buffer and stored at -20°C. All dilutions were conducted using pyrogen-free reagents.

*Cell preparation and cell culture.* Human PBMC were isolated from peripheral blood of healthy volunteers, obtained by the German Red Cross (Ratingen, Germany), by Ficoll-Paque density centrifugation (Histopaque-1077, Sigma, Germany) as described. Hartmann G et al. (1999) Gene Therapy 6:893-903. Cells were suspended in RPMI 1640 culture medium supplemented with 10% (v/v) heat-inactivated fetal calf serum (FCS, Sigma), 1.5mM L-glutamine, 100U/ml penicillin and 100µg/ml streptomycin (all from Life Technologies, Germany). All compounds were purchased endotoxin-tested. For the B cell, NK cell and monocyte activation assays PBMC were cultured in complete medium at a concentration of 2x10⁶ cells/ml in 200µl volumes in 96-well round-bottom plates in a humidified incubator at 37°C. Different ODNs (see above), LPS (Sigma), IL-2, or IL-12 (R&D Systems, USA) were used as stimuli. At the indicated time points, cells were harvested for flow cytometry.

*Flow cytometry.* Monoclonal antibodies (mAbs) used for staining of surface antigens were specific for: CD3, CD14, CD19, CD25, CD40, CD54, CD56, CD69, CD80 and CD86 (all obtained from Pharmingen/Becton Dickinson, Germany). For monocytes Fc receptors were blocked using human IgG (Myltenyi, Germany) as previously described. Bauer M et al. (1999) Immunology 97:699-705. Flow cytometric data of at least 1000 cells of a specified subpopulation (B cells, monocytes, NK cells, NKT cells or T cells) were acquired on a FACSCalibur (Becton Dickinson) cell sorter. Data were analyzed using the program CellQuest (Becton Dickinson).

*B cell proliferation assay.* Proliferating CD19-positive B cells were identified by decreased carboxyfluorescein diacetate succinimidyl diester (CFSE) content using flow cytometry according to a recently published protocol. Hartmann G et al. (2000) J Immunol 164:944-53.

*ELISA.* Except where noted otherwise, PBMC (3-5x10⁶ cells/ml) were cultured with the specified concentrations of ODN or LPS for 24h (IL-6, TNF-α, IFN-γ, and IL-10) or 8h (IL-1β) in 48-well plates in a humidified atmosphere at 37°C. Supernatants were collected and cytokines were measured using OPTeia ELISA kits (Pharmingen) for IL-6, TNF-α, and IFN-γ or an Eli-pair ELISA assay (Hoelzel, Germany) for IL-1β and IL-10 according to the manufacturers' protocols.

*TLR9 Assay.* Cells used for this assay were stable transfectants (293 HEK, human embryo kidney cells) expressing the human TLR9 receptor and containing a genomic NFκB-luciferase cassette. Cells were incubated with ODNs at 1.0 µg/ml, 6.0 µg/ml and 12.0 µg/ml for 16h. Each data point was obtained in triplicate. Cells were lysed and assayed for luciferase activity (LucLite-system, Packard). Stimulation indices were calculated in reference to luciferase activity of medium without addition of ODN. Activity of ODNs is given as % activity of the activity measured with ODN 2006, which was set 100%. Activity of all ODNs tested was calculated as mean value of all experiments and all concentrations.

### Example 1. Response of of human B cells to methylated CpG oligonucleotides.

A first set of experiments examined different ODN, methylated and unmethylated, for their stimulatory effect on human B cells. PBMC of several healthy male or female volunteer blood donors were incubated for 48h in the presence of 0.4 µg/ml, 1.0 µg/ml or 5.0 µg/ml of the following ODN: 2006 and its methylated counterpart 2117; 1758 (antisense ODN G3139, Genta) and its methylated counterpart 1812; 2186 (DSP30, Liang H et al. (1996) J Clin Invest 98:1119-29) and its methylated counterpart S 107. Negative controls were similarly incubated for 48h in the absence of added ODN. PBMC were then stained with mAb to CD19 (B cell marker) and CD86 (B7-2, B cell activation marker), and CD86 expression on CD19+ human B cells was measured by flow cytometry. Results are shown in **Fig. 1****.**

**Fig. 1** shows that, with the exception of ODN 1812, all methylated ODN exhibited B cell stimulatory potential almost to the same degree as the corresponding unmethylated sequences. ODN 2006 at all concentrations induced similar strong activation ofB cells, in contrast to its methylated form ODN 2117, which showed a concentration-dependent increase of stimulation from 0.4 µg/ml to 5µg/ml, In some experiments ODN 2117 was even only marginally less stimulatory than 2006. Equivalent results were obtained for ODN 2186 with the methylated ODN 5107 having stimulatory potential. Only ODN 1812 did not induce B cell activation at these concentrations. In another set of experiments an increase of CD86 expression by ODN 1812 was only detected at very high ODN concentrations (i.e., 50µg/ml and higher), although the degree of stimulation never reached that observed with ODN 1758.

This unexpected high degree of human B cell stimulation induced by methylated CpG oligonucleotides was further investigated and confirmed in studies of two other methylated sequences, the stimulatory antisense oligonucleotides 5114 (13312, Formivirsen ISIS) (methylated: 5154) and 5116 (2302 ISIS) (methylated: 5155).

As shown in **Fig. 2****,** both antisense ODN 5114 and ODN 5116 exhibited significant stimulatory capacity on human B cells, although varying between different experiments. In addition, the corresponding methylated forms 5154 and 5155 also induced stimulation, although to a lesser extent than 5114 and 5116. These results were in accord with the results obtained in **Fig. 1** and demonstrated that methylated immunostimulatory CpG ODN indeed compare to the unmethylated sequences in their capacity to induce activation of human B cells.

To exclude the possibility that the observed results were due to mistakes during preparations or incomplete modification of the methylated ODNs, several ODNs (2117) obtained from the same or from different manufacturer were tested for their activity on human PBMC. The results of these studies clearly demonstrated that all preparations had comparable activity on human B cells.

This effect also could not be attributed to contamination of the ODN with bacterial endotoxins as human B cells were shown to be only poor responders to LPS.

Further experiments were performed to determine whether these methylated ODN were also able to enhance the expression of other molecules on the surface of B cells. Analogous to the experiments just described, PBMC of several healthy male or female volunteer blood donors were incubated for 48h in the presence of 0.4 µg/ml, 1.0 µg/ml or 10.0 µg/ml of the following ODN: 2006; its methylated counterpart 2117; and 2137, the GpC counterpart of 2006. Negative controls were similarly incubated for 48h in the absence of added ODN. After 48h of incubation PBMC were stained with mAb to CD19 (B cell marker) and CD80 (B7-1, B cell activation marker), CD25 (IL-2Rα), or CD54 (adhesion molecule ICAM-1). CD25, CD54, and CD80 expression on CD19+ human B cells was measured by flow cytometry. Results for CD80 and CD25 are shown in **Fig. 3****.**

**Fig. 3** demonstrates that methylated oligonucleotides, in addition to enhancing the expression of the activation marker CD86, induced expression of other markers such as CD80 and CD25 on the surface of human B cells. These experiments also demonstrated that methylated oligonucleotides induced the expression of CD54 on the surface of human B cells. In contrast, the GpC ODN 2137 was less stimulatory than both ODN 2006 and ODN 2117, consistent with a rank order of activation potency of 2006 > 2117 > 2137 (unmethylated CpG > methylated CpG > GpC)..

In B cell proliferation assays, ODN 2117 (methylated 2006) and ODN 5107 (methylated 2186) were in addition shown to induce proliferation of human B cells comparable to the results shown above.

### Example 2. Response of human monocytes to methylated CpG oligonucleotides.

The complex effects of CpG DNA are not solely due to their activation of peripheral blood B cells. It was recently demonstrated that these ODN also induce the secretion of a wide variety of cytokines by interacting with other cells, e.g., monocytes, macrophages, and dendritic cells. Krieg AM (1999) Biochim Biophys Acta 1489:107-16; Kranzer K et al. (2000) Immunology 99:170-8; Hartmann G et al. (1999) Proc Natl Acad Sci USA 96:9305-10; Hartmann G et al. (1999) Gene Therapy 6:893-903. In order to investigate the role of methylated ODN in mediating activation of human monocytes, PBMC (2×10⁶ cells/ml) of several blood donors were incubated for 24h with 6µg/ml ODN 2006, ODN 2117 (methylated 2006), ODN 2137 (GpC 2006), or 1µg/ml LPS. Negative controls were similarly incubated for 24h in the absence of added ODN or LPS. Cells were stained (after Fc-receptor blockade) with mAb to CD14 and CD80, and CD80 expression on CD14+ monocytes was determined by flow cytometry. **Fig. 4** shows two representative results from five separate experiments for the comparison of ODN 2006, 2117, and 2137.

Similar to the results presented in Example 1 for B cells, methylated ODN 2117 was found to induce the stimulation of human monocytes. ODN 2006 showed the strongest activation, followed by the corresponding methylated and GpC ODNs 2117 and 2137. ODN 2137 did not enhance (**Fig. 4**, donor 24) or enhanced only to a low degree (**Fig. 4**, donor 35) the expression of the activation marker CD80. LPS at a concentration of 1µg/ml was in both cases less stimulatory than ODN 2006.

### Example 3. Response of NK cells to methylated CpG oligonucleotides

NK cells account for up to 15% of blood lymphocytes, and their main function is to recognize and kill tumor and virus-infected cells. Roitt I, Brostoff J, and Male D, Immunology, Mosby, London, 1990. It has previously been reported that NK cells are activated, although not directly, by ODN-induced proinflammatory cytokines to secrete IFN-γ and to have increased lytic activity for tumor cells. Ballas ZK et al. (1996) J Immunol 157:1840-5; Takahashi T et al. (2000) J Immunol 164:4458-64; Bohle B et al. (1999) Eur J Immunol 29:2344-53; Krieg AM (1999) Biochim Biophys Acta 1489:107-16. To evaluate the extent of activation mediated by methylated ODN, increased expression of the early activation marker CD69 on NK cells, T cells, and NKT cells, upon their activation was measured in relation to exposure to methylated ODN.

PBMC (2x10⁶ cells/ml) obtained from several blood donors were incubated for 24h with 6µg/ml ODN 2006, 2117 (methylated 2006), or 2137 (GpC 2006). Negative controls were similarly incubated for 24h in the absence of added ODN. As a positive control PBMC were incubated with 100U/ml IL-2 together with 50ng/ml IL-12. To enhance CD69 expression the respective ODN were added together with a submitogenic dose of IL-2 (10U/ml). PBMC were stained for CD56 (N-CAM, NK cell marker), CD3 (T cell marker) or CD69 (early activation marker). Expression of CD69 in the CD56+ cell population was measured by flow cytometry. Representative results obained from two donors are shown in **Fig. 5**.

As'shown for both donors in **Fig. 5**, the methylated ODN 2117 stimulated NK cells to enhance expression of the activation marker CD69, although the degree of activation was low in almost every experiment. Culture with ODN 2137 (GpC 2006) in contrast resulted in only very weak stimulation of human NK cells. Similar to the results in Example 2 for human monocytes (**Fig. 4**) a ranking 2006 > 2117 > 2137 was observed for activation of NK cells.

As also shown in **Fig. 5****,** NK cell activation by the different ODN was enhanced by addition of IL-2. Takahashi T et al. (2000) J Immunol 164:4458-64. By this way stimulation of NK cells by ODN 2006 matched even the very strong stimulus furnished by the simultaneous addition of IL-2 and IL-12. The same pattern of activation strength, 2006 > 2117 > 2137, was observed.

Further experiments investigated the activation of T cells and NKT cells in PBMC by the different ODN. NKT cells have similarities to both NK cells and T cells. They are defined to be activated by glycolipids, to exhibit cytotoxicity against tumor cells and to influence the Th1/Th2 pattern of the immune response. Takahashi T et al. (2000) J Immunol 164:4458-64. PBMC (2×10⁶ cells/ml) were incubated with 6µg/ml of ODN 2006, 2117 and 2137 for 24h at 37°C, as above. Negative controls were similarly incubated for 24h in the absence of added ODN. After harvesting, cells were stained for CD3 (T cell marker), CD56 (NK cell marker), and CD69. Expression of CD69 on CD56+/CD3+ NKT cells was analyzed by flow cytometry. Representative results for NKT cells are shown in **Fig. 6**.

Both T-cell and NKT-cell populations enhanced CD69 expression upon cultivation with ODN 2006. They also showed a similar pattern of stimulation 2006 > 2117 > 2137. This result was in conformity with a uniform scheme of lymphocyte activation in human peripheral blood by unmethylated and methylated ODN.

### Example 4. Secretion of proinflammatory cytokines IL-6 and TNF-α induced by methylated CpG oligonucleotides.

Proinflammatory cytokines such as IL-6 and TNF-α play an important role in the pathogenesis of diseases. Akira S, (1990) FASEB J 4:2860-7. Both cytokines are mainly produced by macrophages and monocytes, but also by T cells (IL-6 and TNF-α) and B cells (IL-6 only). Earlier studies demonstrated that CpG ODNs effectively induce the secretion of IL-6 and TNF-α. Hartmann G et al. (1999) Gene Therapy 6:893-903. To investigate further the immunostimulatory effect of methylated CpG ODN, the secretion of these cytokines by PBMC was measured after their cultivation with the different ODN.

PBMC (3x10⁶ cells/ml) were obtained from several blood donors and incubated for ' 24h with 0.4, 1.0, or 6 p.g/ml ODN 2006, 2117 (methylated 2006), 2137 (GpC 2006), or 1µg/ml LPS as positive control. Negative controls were similarly incubated for 24h in the absence of added ODN or LPS. Alter 24h supernatants were collected and IL-6 and TNF-α were measured by ELISA as described above. Representative results are shown in **Fig. 7**.

Secretion of IL-6 and TNF-α was induced by all ODNs tested (**Fig. 7A** and **Fig. 7B**). Again a clear rank order of activation 2006 > 2117 > 2137 was observed. The same dose response as for the induction of activation markers on human B cells was observed (Fig. 7B). IL-6 is well known to mediate differentiation and activation of B cells as well as secretion of antibodies (Akira S (1990) FASEB J 4:2860-7), therefore possibly reflecting the degree of B cell activation.

### Example 5. Secretion of IL-1 β induced by methylated CpG oligonucleotides.

Of the two functionally almost equivalent forms of IL-1, IL-1β is the predominant form in humans. It is mainly secreted by monocytes and has a wide variety of immunological functions. Bomford R and Henderson B, Interleukin-1, Inflammation and Disease, Elsevier, New York, 1989. IL-1β plays a role in the stimulation of B, T and NK cells; participates in the conversion of Langerhans cells to professional antigen-presenting dendritic cells; acts as a chemoattractant for leukocytes; and directly affects the central nervous system. No previous report demonstrated IL-1β secretion induced by CpG ODN in human PBMC, although such reports exist for murine PBMC. Chelvarajan RL et al. (1999) Eur J Immunol 29:2808-18. The following experiments investigated the effect of CpG ODN on the secretion of human EL-1β by incubating PBMC isolated from the blood of healthy donors with ODN 2006, 2117, and 2137 (**Fig. 8**).

PBMC (3x10⁶ cells/ml) obtained from several blood donors were incubated for 8h with 6µg/ml ODN 2006, 2117, 2137, or 1µg/ml LPS as positive control. Negative controls were similarly incubated for 8h in the absence of added ODN or LPS. After 8h supernatants were collected and IL-1β was measured by ELISA as described above.

Two important results were obtained by measuring IL-1β. First, the experiments showed (**Fig. 8**) that CpG ODN are indeed potent inducers of IL-1β secretion. Second, the results demonstrated the same rank order of differential activation by the unmethylated, methylated and non-CpG ODN 2006, 2117 and 2137, respectively.

### Example 6. Secretion of Th1 and Th2 cytokines induced by methylated CpG oligonucleotides.

One important feature of CpG ODN is their potential to induce a Th1-like response in vivo. Monocytes and other cell types are directly activated to secrete Th1 cytokines, e.g., IL-12. IL-12 itself mediates the activation of NK cells and, therefore, the secretion of IFN-γ by this cell type. Krieg AM (2000) Curr Opin Immunol 12:35-43. A previous study demonstrated that IFN-γ secretion of NK cells can be enhanced by addition of submitogenic doses of IL-2. Iho S et al. (1999) J Immunol 163:3642-52.

To examine the effect of methylated CpG ODN on IFN-γ secretion, human PBMC (5x10⁶ cells/ml) isolated from peripheral blood of healthy blood donors were cultured with 6µg/ml ODN 2006, 2117, 2137 or 0.1µg/ml LPS for 16-24h in 48-well plates in a humified atmosphere at 37°C. Submitogenic amounts of IL-2 (10U/ml) were added to some wells containing cells and ODN for the overnight incubation. Supernatants were collected and cytokines were measured by ELISA as described above. Results are presented in **Fig. 9****.**

**Fig. 9A** illustrates that ODN 2117 indeed induced the secretion of IFN-γ upon incubation of human peripheral blood monocytes (PBMC) with this ODN. The addition of submitogenic amounts of IL-2 enhanced IFN-γ secretion by a factor of 5 to 10 (**Fig. 9B**). Both figures show representative results for 7 blood donors tested. Although there were no great differences among the ODN, ODN 2006 consistently induced the highest amount of IFN-γ, followed by ODN 2117 and 2137.

IL-10 is a Th2 cytokine which has anti-inflammatory properties and is thought to play a role as a regulator of the immune response induced by CpG ODN. A previous report demonstrated the induction of IL-10 after cultivation of murine splenocytes with CpG ODN. Redford TW et al. (1998) J Immunol 161:3930-5.

To evaluate the possible effect of methylated CpG ODN on IL-10 secretion, human PBMC (5x10⁶ cells/ml) isolated from peripheral blood of healthy blood donors were cultured with 6µg/ml ODN 2006, 2117, or 2137 for 16-24h in 48-well plates in a humified atmosphere at 37°C. Negative controls were similarly incubated overnight without added ODN. Supernatants were collected and IL-10 was measured by ELISA as described above. Results are presented in **Fig. 10**.

**Fig. 10** demonstrates the induction of IL-10 after incubation of human PBMC with different ODN. As shown above for IFN-γ, ODN 2006 and 2117 induce higher cytokine concentrations than ODN 2137. Consistent with the results above, the rank of immunostimlation obtained was: non-methylated ODN > methylated ODN > non-CpG GpC ODN.

### Example 7. Response of human B cells to CpI oligonucleotides.

PBMC (2x10⁶ cells/ml) obtained from healthy blood donors (n = 3)were incubated for 48h with 0.4 µg/ml, 1.0 µg/ml, and 10.0 µg/ml of each ODN (2006, 1982, 5126, 5246, 5247, 5248, 5249, 5250 and 5251). Negative controls were similarly incubated for 48h in the absence of added ODN. Cells were harvested and stained with mAb to CD19 and CD86. Expression of the activation marker CD86 on CD 19+ B cells was measured by flow cytometry. Representative results from 2 donors (Donor 60 and Donor 62) are shown in **Fig. 11****.**

The results in **Fig. 11** demonstrate that 2006 represents the best immunostimulating ODN of this group, followed by the non-CpG ODN 5126 and 1982. ODN containing 2'-methoxy backbone-modified cytosine residues (5246-5248) were not affected by these modifications, i.e., were just as immunostimulatory as the corresponding unmodified ODN (2006, 1982, and 5126) (**Fig. 11**). Significantly, ODN containing inosine instead of guanosine (5249-5251) were not affected by these modifications, i.e., were just as immunostimulatory as the corresponding unmodified ODN (2006, 1982, and 5126) (**Fig. 11**).

In related experiments designed to investigate B cell proliferation rather than activation, PBMC (2x10⁶ cells/ml) of 2 donors (Donor 48 and Donor 62) were stained with the dye CFSE and then incubated with 0.4, 1.0 and 10.0 µg/ml of the same panel of ODN for 5 days at 37°C. Negative controls were similarly incubated for 5 days in the absence of added ODN. Cells were harvested and stained with a mAb to CD 19. Proliferation of CD 19+ B cells was measured by flow cytometry and expressed as the percentage of B cells with decreased brightness with the CFSE stain. Results are shown in **Fig. 12**.

Comparing the results for induction of B cell proliferation (**Fig. 12**) with the B cell activation (**Fig. 11**), one can observe essentially the same differences, although in the case of the 2'-methoxy modified PS ODN, the effect of the modification is not as profound. Significantly, ODN containing inosine instead of guanosine (5249-5251) were not affected by these modifications, i.e., were just as immunostimulatory in this assay as the corresponding unmodified ODN (2006, 1982, and 5126)

### Example 8. Activation of NK cells and monocytes by CpI oligonucleotides.

To examine the effect on NK cells, PBMC (2x10⁶ cells/ml) of two different donors were incubated for 24h with 1.0 µg/ml and 6.0 µg/ml of each ODN (2006,1982, 5126, 5246, 5247, 5248, 5249, 5250 and 5251). Negative controls were similarly incubated for 24h in the absence of added ODN. Positive controls were similarly incubated for 24h with addition of IL-2 (100U/ml) plus IL-12 (50ng/ml). Subsequently, cells were harvested and stained with the cell surface markers CD56 (NK cells), CD3 (T cells) and CD69 (early activation marker). Expression of CD69 on CD56+/CD3- NK cells was measured by flow cytometry. Results are shown in **Fig. 13**.

To examine the effect on monocyes, PBMC (2x10⁶ cells/ml) of three different donors (Donors 41, 66, and 67) were incubated for 24h with 1.0 µg/ml and 6.0 µg/ml of the same panel of ODN. Negative controls were similarly incubated for 24h in the absence of added ODN. Positive controls were similarly incubated for 24h with addition of LPS (Ing/ml). Subsequently, cells were harvested and stained with the cell surface markers CD14, CD19, and CD80 to measure the expression of the cell surface marker B7-1 (CD80) on CD14+ monocytes (excluding CD19+CD14+ B cells) by flow cytometry. Results are shown in **Fig. 14****.**

For NK cells, ODN 2006 clearly enhanced expression of CD69 (**Fig. 13**), 5126 was less active, and 1982 showed nearly no stimulating effect. Immunostimulation by ODN with 2'-methoxy backbone-modified cytosines (5246-5248) and inosine for guanosine exchanges (5249-5251) was not altered from the respective unmodified PS ODN (2006, 1982, and 5126).

Essentially the same picture of immunostimulatory activity was obtained for monocytes (**Fig. 14**). The rank of activation was again: PS ODN = inosine for guanosine PS ODN = 2'-methoxy cytosine PS ODN. Therefore, for all cell types tested, ODN with 2'-methoxy backbone-modified cytosines or inosine instead of guanosine showed the same effects as the reference PS ODN themselves.

### Example 9. Secretion of cytokines induced by CpI oligonucleotides.

PBMC (5x10⁶ cells/ml) of different donors were incubated for 20 h with 6.0 µg/ml of ODN (2006, 1982, 5126, 5246, 5247, 5248, 5249, 5250 and 5251). Negative controls were similarly incubated for 20h in the absence of added ODN. Positive controls were similarly incubated for 20h in the presence of LPS (0.1 u.g/ml). Cell culture supernatants were then analyzed by ELISA for the pro-inflammatory cytokine TNF-α, the Th1 cytokine IFN-γ, the Th2 cytokine IL-10, and the cytokine IL-6. Results are shown in **Figs. 15-18**.

**Fig. 15** shows representative results (from 2 of 4 donors) for TNF-α. TNF-α is a cytokine that is not strongly upregulated by CpG ODN; typically amounts between 10 and 200pg/ml can be obtained, compared to about 1000pg/ml with LPS. In these experiments, all tested ODN induced TNF-α secretion (**Fig. 15**). For ODN 5246, 5247, and 5248 (2'-OMe-C PS ODN) and ODN 5249. 5250, and 5251 (inosine PS ODN) these results confirm our previous observations with TNF-α levels similar to those induced by reference ODN 2006, 1982 and 5126.

For IFN-γ, the results varied depending on the donor. Representative results from 2 of 3 donors studied are shown in **Fig.16**. The amounts of IFN-γ in human *in vitro* cultures were very low, resulting in intra-experimental variations. Nevertheless, the results again demonstrated that ODN with 2'-methoxy backbone-modified cytosines or with inosine had immunostimulatory potential. For these ODN, the amount of IFN-γ obtained was higher than for the original reference PS ODN.

For IL-10 mainly the same result was obtained for the 2 donors studied (**Fig. 17**). ODN 5246-5248 and 5249-5251 (2'-methoxy backbone-modified cytosine ODN and inosine for guanosine PS ODN) induced IL-10, with the inosine-containing ODN inducing the highest amounts.

The results for IL-6 (**Fig. 18**) were consistent with those obtained for B cell activation and proliferation (Example 5). The rank order of activation for the 4 donors studied was again found to be: PS ODN = inosine for guanosine PS ODN = 2'-methoxy backbone-modified cytosine PS ODN.

### Example 10. Secretion of Th1 chemokine IP-10 induced by CpI oligonucleotides.

In this experiment ODN 5331, in which CpG dinucleotides in ODN 2006 were replaced with CpI dinucleotides, was examined for its ability to induce secretion of the IFN-γ-induced chemokine IP-10. Thawed or fresh PBMCs were resuspended at a concentration of 5x10⁶/ml and added to 48-well flat-bottomed plates (1 ml/well) with or without ODNs. ODNs were added before the cells to achieve a variety of final ODN concentrations ranging from 0.4 µg/ml to 3.0 µg/nil. The cells were cultured in a humidified atmosphere at 37°C. Culture supernatants were collected after 48 hours for ELISAs. If not used immediately, supernatants were frozen at -20°C until required.

ODN 5331 (CpI) induced comparable and, at 3.0 µg/ml even higher, levels of IP-10 compared to ODN 2006.

### Example 11. Nucleotides including dSpacers in place of the C, G, or C and G nucleotides of CpG stimulate human B cells.

The following set of experiments examined whether the exchange of a so-called "dSpacer" (a "nucleotide" with only the sugar moiety; dSp) for the C, the G or both nucleotides of the CpG dinucleotide would affect the immunostimulatory activity of the ODNs 2006 or 5122. Therefore, such ODNs were cultivated in vitro with PBMCs derived from the blood of healthy volunteers to measure either the expression of cell surface activation markers on B cells or proliferation of B cells. Surprisingly, all of these ODNs (5280 (CpG to CpdSpacer), 5281 (CpG to dSpacerpG), and 5282 (CpG to dSpacerpdSpacer)) exhibited at least some immunostimulatory activity. ODN 5280 in particular showed very high activity, nearly comparable to ODN 2006 (only about 10-15% less activity). In contrast, ODNs 5281 and 5282 induced about 50% as much stimulation of B cells as ODN 2006, and stimulation was not further enhanced by using higher ODN concentrations (up to 10µg/ml). The same result was observed for reference ODN 5122 and corresponding CpG-like ODNs 5283 - 5285. ODN 5122 differs from ODN 2006 by having fewer stimulatory CpG motifs.

Essentially the same effect as above was observed for the induction of proliferation of B cells by these ODNs, although the difference between 2006, 5280, and the other ODNs was more pronounced. As above, the exchange of the G did not have the same effect as the exchange of the C.

These results show that the C and/or the G of the CpG dinucleotide can be substituted by the "dSpacer" resulting in a decrease but not a loss of immunostimulatory activity. This preserved effect appeared to be stronger for look-alikes of ODN 2006 (the optimized human CpG ODN) than for corresponding look-alikes of ODN 5122. ODN 2006 has not only CpG dinucleotide motifs but also a high T content. As described earlier, such poly T sequences mediate an immunostimulatory effect. Therefore, ODN 2006 might preserve part of its immunostimulatory activity after substituting the normally essential CpG dinucleotides. For other CpG ODNs such as 5122, activity goes back closer to background (here immunostimulation by the non-CpG ODN 1982) after exchange of a "dSpacer" for the CpG dinucleotides. Another important observation is that the exchange of the G alone obviously had less effect on immunostimulation than exchange of the C or of both nucleotides.

### Example 12. Secretion of cytokines induced by ZpY oligonucleotides.

In this series of experiments variants of ODN 2006 incorporating various substitutions for CpG, other than CpI, were examined for their ability to induce secretion of the cytokines IL-10 and IFN-γ. The ODN included ODN 5279 (CpG-Cp2,2=2-aminopurine), 5280 (CpG→CpdSpacer (CpdSp), 5282 (CpG-dSppdSp), and 5290 (CpG→CpW, W=nebularine). Thawed or fresh PBMCs were resuspended at a concentration of 5x10⁶/ml and added to 48-well flat-bottomed plates (1 ml/well) with or without ODNs. ODNs were added before the cells to achieve a variety of final ODN concentrations ranging from 0.2 µg/ml to 0.8 µg/ml. The cells were cultured in a humidified atmosphere at 37°C. Culture supernatants were collected after 48 hours for ELISAs. If not used immediately, supernatants were frozen at -20°C until required.

ODNs 2006, 5279, 5280, 5282, and 5290, at concentrations of 0.4 µg/ml and 0.8 µg/ml, all induced IL-10 well above that observed for negative controls (no added ODN). ODN 5280 (CpdSp) induced similar levels of IL-10 compared to ODN 2006. In addition, ODN 5290 (CpW) also induced comparably high levels of IL-10 (700 pg/ml at 0.8 µg/ml). ODN 5279 (Cp2) was less stimulatory but still induced substantial amounts of IL-10 at 0.8 µg/ml. Similar results were obtained for the secretion of IFN-γ, although ODNs 5280 and 5290 induced somewhat lower amounts of this Th1 cytokine than did ODN 2006.

### Example 13. Activation of B cells induced by ZpY oligonucleotides.

Human PBMC were incubated with various ZpY ODNs and expression of an activation marker (CD86) on the surface of B cells was measured. Human PBMC obtained from three donors were incubated with the various concentrations (0.2 µg/ml to 5.0 µg/ml) of each ODN for 48h. After cells were harvested and stained for the B cell marker CD 19 and for CD86, data were acquired by flow cytometry. Data were evaluated as stimulation index calculated in reference to samples incubated without ODNs. ODN 5286 with a CpG to Cp7 (7=7-deaza-guanosine) exchange retained a lot of its stimulation at all concentrations examined compared to ODN 2006. Nevertheless, ODN 5286 activated human B cells much more efficiently than the non-CpG ODN 1982, used as a control ODN. The following ODNs (also derived from the sequence of ODN 2006) were also tested for their potential to activate human B cells: CpG to UpG (ODN 5384), CpG to GpG (ODN 5385), CpG to CpC (ODN 5386), CpG to GpI (ODN 5387), CpG to 2pG (ODN 5388) and CpG to CpM (5389, M=2,6-diaminopurine). All of these ODNs exhibited at least some immune stimulation of human immune cells. The best ODNs were those with the CpG to CpC, CpM, UpG and 2pG exchanges.

### Example 14. Stimulation of TLR9.

Stimulation by CpG ODN depends to a great extend on the activation of toll-like receptor 9 (TLR9). In this example the activation of TLR9 transfectants by CpG and CpG-like ODN were examined and compared. As demonstrated above, the CpG to CpI exchange retained about 80% of the original (i.e., ODN 2006) activity; In addition, the next best ODN examined (with about 40% activity) on these transfectants was ODN 5280 (CpG to CpdSpacer), followed by ODN 5286 (CpG to Cp7) and ODN 5290 (CpG to CpW). These results were consistent with the observations above on activation of human PBMC.

## Claims

1. A composition, comprising:
an immunostimulatory nucleic acid having a sequence including at least the following formula:
5'X₁X₂CGX₃X₄3'
wherein C is a nucleotide in which the nucleoside moiety is 2'-methoxy cytidine and wherein X₁ X₂, X₃, and X₄ are nucleotides, in an amount effective to induce an immune response.

2. A composition as claimed in claim 1, wherein the immunostimulatory nucleic acid:
i) is an isolated nucleic acid,
ii) has between 6 and 100, or 8 and 40, nucleotides,
iii) has a modified backbone, preferably a phosphate modified backbone,
iv) is a synthetic nucleic acid, and/or
v) is at least 18 nucleotides long and is not an antisense nucleic acid.

3. A composition as claimed in claim 1 or 2, further comprising a pharmaceutically acceptable carrier.

4. A composition as claimed in any of claims 1-3, wherein the pharmaceutically acceptable carrier is a sustained-release device.

5. A composition as claimed in claim 2 or 3, further comprising:
i) an antigen,
ii) an anti-cancer medicament, preferably wherein the anti-cancer medicament is selected from the group consisting of a monoclonal antibody, a chemotherapeutic agent, and a radiotherapeutic agent,
iii) an antiviral agent,
iv) an antibacterial agent,
v) an antifungal agent,
vi) an antiparasitic agent,
vii) an ulcer medicament,
viii) an allergy medicament,
ix) an asthma medicament,
x) an anemia medicament,
xi) a thrombocytopenia medicament,
xii) a neutropenia medicament, or
xiii) a cytokine, preferably wherein the cytokine is selected from the group consisting of interleukin-2 (IL-2), IL-3, IL-4, IL-18, interferon alpha (IFN-α), IFN-γ, tumor necrosis factor alpha (TNF-α), Flt3 ligand, granulocyte colony-stimulating factor (GM-CSF).

6. A composition as claimed in claim 3 or 4, wherein the composition includes at least two immunostimulatory nucleic acids having different sequences.

7. A composition as claimed in any of claims 1-6, for use in inducing an immune response in a subject.

8. Use of a composition as claimed in any of claims 1-6, for the manufacture of a medicament for inducing an immune response in a subject.

9. A use as claimed in claim 8, wherein the subject is a dog, cat, horse, cow, pig, sheep, goat, rabbit, guinea pig, non-human primate, chicken, or fish.

10. A use as claimed in claim 8 or 9, wherein the medicament is for administration:
i) to a subject at risk of developing an infectious disease, in an effective amount for preventing the infectious disease,
ii) to a subject with an infectious disease, in an effective amount for treating the infectious disease,
iii) to a subject at risk of developing a cancer, in an effective amount for preventing the cancer,
iv) to a subject with a cancer, in an effective amount for treating the cancer,
v) to a subject at risk of developing an allergy, in an effective amount for preventing the allergy,
vi) to a subject with an allergy, in an effective amount for treating the allergy,
vii) to a subject at risk of developing asthma, in an effective amount for preventing asthma,
viii) to a subject with asthma, in an effective amount for treating the asthma,
ix) to a subject that has or is at risk of developing an immunodeficiency, in an effective amount for enhancing stimulating bone marrow proliferation in the subject, preferably wherein the subject that has or is at risk of developing an immunodeficiency is a subject undergoing or at risk of undergoing chemotherapy,
x) to a subject that has or is at risk of developing anemia, in an effective amount for enhancing erythropoiesis in the subject,
xi) to a subject that has or is at risk of developing thrombocytopenia, in an effective amount for enhancing thrombopoiesis in the subject,
xii) to a subject that has or is at risk of developing neutropenia, in an effective amount for enhancing neutrophil proliferation in the subject,
xiii) in an effective amount for inducing cytokine production, preferably wherein the cytokine is selected from the group consisting of IL-1 beta (IL-1β), IL-2, IL-6, IL-12, IL-18, TNF-α, IFN-α, and IFN-γ, or
xiv) in an effective amount for stimulating natural killer cell activity.

11. A use as claimed in claim 8 or 9, wherein the medicament is for administration to the subject with an antigen, preferably wherein the antigen is selected from the group consisting of an allergen, a tumor antigen, a viral antigen, a bacterial antigen, a fungal antigen, and a parasitic antigen.

12. A use as claimed in claim 11, wherein the antigen is administered by:
i) a mucosal route, preferably wherein the mucosal route is selected from the group consisting of oral, nasal, rectal, vaginal, transdermal and ocular, or
ii) a parenteral route, preferably wherein the parenteral route is selected from the group consisting of intravenous, subcutaneous, intramuscular, and direct injection.

13. A use as claimed in claim 8 or 9, wherein the medicament is for administration to the subject with an anti-cancer therapy, preferably wherein the anti-cancer therapy is:
i) a monoclonal antibody specific for a tumor cell,
ii) a chemotherapy, or
iii) a radiotherapy.

14. A use as claimed in claim 8 or 9, wherein the medicament is for administration:
i) by a mucosal route, preferably wherein the mucosal route is selected from the group consisting of oral, nasal, rectal, vaginal, transdermal and ocular,
ii) by a parenteral route, preferably wherein the parenteral route is selected from the group consisting of intravenous, subcutaneous, intramuscular, and direct injection, or
iii) in a sustained-release vehicle.

## Patentansprüche

1. Zusammensetzung, umfassend:
Eine immunstimulatorische Nukleinsäure mit einer Sequenz, die mindestens die folgende Formel einschließt:
5'X₁X₂CGX₃X₄3'
worin C ein Nukleotid darstellt, worin der Nukleosidteil 2'-Methoxycytidin darstellt und worin X₁, X₂, X₃ und X₄ Nukleotide darstellen, in einer Menge, die zur Induktion einer Immunantwort wirksam ist.

2. Zusammensetzung nach Anspruch 1, worin die immunstimulatorische Nukleinsäure
i) eine isolierte Nukleinsäure darstellt,
ii) zwischen 6 und 100 oder 8 und 40 Nukleotide aufweist,
iii) ein modifiziertes Rückgrat, bevorzugt ein Phosphat modifiziertes Rückgrat aufweist,
iv) eine synthetische Nukleinsäure darstellt, und/oder
v) mindestens 18 Nukleotide lang ist und keine Antisense-Nukleinsäure darstellt.

3. Zusammensetzung nach Anspruch 1 oder 2, die ferner einen pharmazeutisch verträglichen Träger umfasst.

4. Zusammensetzung nach einem der Ansprüche 1 bis 3, worin der pharmazeutisch verträgliche Träger eine Vorrichtung mit hinhaltender Freisetzung darstellt.

5. Zusammensetzung nach Anspruch 2 oder 3, ferner umfassend:
i) ein Antigen
ii) ein Antikrebsmittel, worin das Antikrebsmittel bevorzugt aus der Gruppe ausgewählt ist, die aus einem monoklonalen Antikörper, einem Chemotherapeutikum und einem Strahlentherapeutikum besteht,
iii) ein Antivirusmittel,
iv) ein antibakterielles Mittel,
v) ein Antimykotikum,
vi) ein antiparasitäres Mittel,
vii) ein Antiulkusmittel,
viii) ein Antiallergikum,
ix) ein Antiasthmatikum,
x) ein Antianämikum,
xi) ein Arzneimittel gegen Thrombozytopenie,
xii) ein Arzneimittel gegen Neutropenie, oder
xiii) ein Zytokin, bevorzugt worin das Zytokin aus der Gruppe ausgewählt ist, bestehend aus Interleukin-2 (IL-2), IL-3, IL-4, IL-18, Interferon alfa (IFN-α), IFN-γ, Tumornekrosefaktor-alfa (TNF-α), einem Flt3-Liganden und einem Granulozyten-Kolonie stimulierenden Faktor (GM-CSF).

6. Zusammensetzung nach Anspruch 3 oder 4, worin die Zusammensetzung mindestens zwei immunstimulatorische Nukleinsäuren mit verschiedenen Sequenzen einschließt.

7. Zusammensetzung nach einem der Ansprüche 1 bis 6 zur Verwendung bei der Induktion einer Immunantwort in einem Patienten.

8. Verwendung einer Zusammensetzung nach einem der Ansprüche 1-6 zur Herstellung eines Arzneimittels zur Induktion einer Immunantwort in einem Patienten.

9. Verwendung nach Anspruch 8, worin der Patient ein Hund, eine Katze, ein Pferd, eine Kuh, ein Schwein, ein Schaf, eine Ziege, ein Kaninchen, ein Meerschweinchen, ein nicht humaner Primat, ein Huhn oder ein Fisch ist.

10. Verwendung nach Anspruch 8 oder 9, worin das Arzneimittel zur Verabreichung bestimmt ist:
i) an einen Patienten mit Risiko für die Entwicklung einer Infektionserkrankung, in einer wirksamen Menge zur Verhinderung der Infektionserkrankung,
ii) an einen Patienten mit einer Infektionserkrankung, in einer wirksamen Menge zur Behandlung der Infektionserkrankung,
iii) an einen Patienten mit Risiko für die Entwicklung einer Krebserkrankung, in einer wirksamen Mengen zur Verhinderung der Krebserkrankung,
iv) an einen Patienten mit einer Krebserkrankung, in einer wirksamen Menge zur Behandlung der Krebserkrankung,
v) an einen Patienten mit Risiko für die Entwicklung einer Allergie, in einer wirksamen Menge zur Verhinderung der Allergie,
vi) an einen Patienten mit einer Allergie, in einer wirksamen Menge zur Behandlung der Allergie,
vii) an einen Patienten mit Risiko für die Entwicklung von Asthma, in einer wirksamen Menge zur Verhinderung von Asthma,
viii) an einen Patienten mit Asthma, in einer wirksamen Menge zur Behandlung von Asthma,
ix) an einen Patienten, der an einer Immundefizienz leidet oder mit Risiko für die Entwicklung einer Immundefizienz, in einer wirksamen Menge zur Förderung der Stimulation der Knochenmarksproliferation in dem Patienten, worin der Patient, der an einer Immundefizienz leidet oder mit Risiko für die Entwicklung einer Immundefizienz, bevorzugt ein Patient ist, der sich einer Chemotherapie unterzieht oder mit Risiko, dass er sich einer Chemotherapie unterziehen muss,
x) an einen Patienten, der an einer Anämie leidet oder mit Risiko für die Entwicklung einer Anämie, in einer wirksamen Menge zur Förderung der Erythropoese in dem Patienten,
xi) an einen Patienten, der an einer Thrombozytopenie leidet oder mit Risiko für die Entwicklung einer Thrombozytopenie, in einer wirksamen Menge zur Förderung der Thrombopoese in dem Patienten,
xii) an einen Patienten, der an einer Neutropenie leidet oder mit Risiko für die Entwicklung einer Neutropenie, in einer wirksamen Menge zur Förderung der neutrophilen Granulozytenproliferation in dem Patienten,
xiii) in einer wirksamen Menge zur Induktion der Zytokinproduktion, worin das Zytokin bevorzugt aus der Gruppe ausgewählt ist, bestehend aus IL-1 beta (IL-1β), IL-2, IL-6, IL-12, IL-18, TNF-α, IFN-α und IFN-γ oder
xiv) in einer wirksamen Menge zur Stimulation der Aktivität der natürlichen Killerzellen.

11. Verwendung nach Anspruch 8 oder 9, worin das Arzneimittel zur Verabreichung an den Patienten mit einem Antigen vorgesehen ist, worin das Antigen bevorzugt aus der Gruppe ausgewählt ist, bestehend aus einem Allergen, einem Tumorantigen, einem Virusantigen, einem Bakterienantigen, einem Pilzantigen und einem Parasitenantigen.

12. Verwendung nach Anspruch 11, worin das Antigen verabreicht wird über:
i) eine mukosale Route, worin die mukosale Route bevorzugt aus der Gruppe ausgewählt ist, die aus einer oralen, nasalen, rektalen, vaginalen, transdermalen und okularen Route besteht, oder
ii) eine parenterale Route, worin die parenterale Route bevorzugt aus der Gruppe ausgewählt ist, die aus der intravenösen, subkutanen, intramuskulären und direkten Injektion besteht.

13. Verwendung nach Anspruch 8 oder 9, worin das Arzneimittel zur Verabreichung an den Patienten mit einer Antikrebstherapie vorgesehen ist, worin die Antikrebstherapie bevorzugt aus Folgendem besteht:
i) einem für eine Tumorzelle spezifischen monoklonalen Antikörper,
ii) einer Chemotherapie, oder
iii) einer Strahlentherapie.

14. Verwendung nach Anspruch 8 oder 9, worin das Arzneimittel zur Verabreichung wie folgt vorgesehen ist:
i) über eine mukosale Route, worin die mukosale Route bevorzugt aus der Gruppe ausgewählt ist, die aus einer oralen, nasalen, rektalen, vaginalen, transdermalen und okularen Route besteht,
ii) über eine parenterale Route, worin die parenterale Route bevorzugt aus der Gruppe ausgewählt ist, die aus der intravenösen, subkutanen, intramuskulären und direkten Injektion besteht, oder
iii) in einem Vehikel mit hinhaltender Freisetzung.

## Revendications

1. Composition qui comprend :
une séquence d'acide nucléique immunostimulatrice
comportant au moins le motif suivant :
5'X₁X₂CGX₃X₄3'
où C est un nucléotide dans lequel la portion nucléoside est la 2'-méthoxycytidine et où X₁, X₂, X₃ et X₄ sont des nucléotides, en une quantité efficace pour induire une réponse immunitaire.

2. Composition selon la revendication 1, où l'acide nucléique immunostimulateur :
i) est un acide nucléique isolé,
ii) comporte de 6 à 100 ou de 8 à 40 nucléotides,
iii) présente un squelette modifié, préférablement un squelette phosphate modifié,
iv) est un acide nucléique synthétique, et/ou
v) a une longueur de 18 nucléotides au moins et n'est pas un acide nucléique antisens.

3. Composition selon la revendication 1 ou 2, qui comprend également un véhicule pharmaceutiquement acceptable.

4. Composition selon l'une quelconque des revendications 1-3, où le véhicule pharmaceutiquement acceptable est un système à libération prolongée.

5. Composition selon la revendication 2 ou 3, qui comprend également :
i) un antigène,
ii) un médicament anticancéreux, où le médicament anticancéreux est préférablement sélectionné dans le groupe consistant en un anticorps monoclonal, un agent chimiothérapeutique et un agent radiothérapeutique,
iii) un agent antiviral,
iv) un agent antibactérien,
v) un agent antifongique,
vi) un agent antiparasitaire,
vii) un médicament antiulcéreux,
viii) un médicament antiallergique,
ix) un médicament antiasthmatique,
x) un médicament antianémique,
xi) un médicament contre la thrombocytopénie,
xii) un médicament contre la neutropénie, ou
xiii) une cytokine, où la cytokine est préférablement sélectionnée dans le groupe consistant en l'interleukine-2 (IL-2), l'IL-3, l'IL-4, l'IL-18, l'interféron alpha (IFN-α), l'IFN-γ, le facteur de nécrose tumorale alpha (TNF-α), le Flt3 ligand et le facteur de stimulation des colonies de granulocytes (GM-CSF).

6. Composition selon la revendication 3 ou 4, où ladite composition comprend au moins deux acides nucléiques immunostimulateurs de séquences différentes.

7. Composition selon l'une quelconque des revendications 1-6, qui est utilisée pour induire une réponse immunitaire chez un sujet.

8. Utilisation d'une composition selon l'une quelconque des revendications 1-6 dans la fabrication d'un médicament conçu pour induire une réponse immunitaire chez un sujet.

9. Utilisation selon la revendication 8, où le sujet est un chien, un chat, un cheval, une vache, un porc, un mouton, une chèvre, un lapin, un cobaye, un primate non humain, une poule ou un poisson.

10. Utilisation selon la revendication 8 ou 9, où le médicament est destiné à être administré :
i) à un sujet à risque de développer une maladie infectieuse, en une quantité efficace pour prévenir la maladie infectieuse,
ii) à un sujet atteint d'une maladie infectieuse, en une quantité efficace pour traiter la maladie infectieuse,
iii) à un sujet à risque de développer un cancer, en une quantité efficace pour prévenir le cancer,
iv) à un sujet atteint d'un cancer, en une quantité efficace pour traiter le cancer,
v) à un sujet à risque de développer une allergie, en une quantité efficace pour prévenir l'allergie,
vi) à un sujet atteint d'une allergie, en une quantité efficace pour traiter l'allergie,
vii) à un sujet à risque de développer un asthme, en une quantité efficace pour prévenir l'asthme,
viii) à un sujet atteint d'un asthme, en une quantité efficace pour traiter l'asthme,
ix) à un sujet atteint d'une immunodéficience ou à risque de développer une immunodéficience, en une quantité efficace pour augmenter la stimulation de la prolifération des cellules de la moelle osseuse chez le sujet, où le sujet atteint d'une immunodéficience ou à risque de développer une immunodéficience est préférablement un sujet soumis ou à risque d'être soumis à une chimiothérapie,
x) à un sujet atteint d'une anémie ou à risque de développer une anémie, en une quantité efficace pour stimuler l'érythropoïèse chez le sujet,
xi) à un sujet atteint d'une thrombocytopénie ou à risque de développer une thrombocytopénie, en une quantité efficace pour stimuler la thrombocytopoïèse chez le sujet,
xii) à un sujet atteint d'une neutropénie ou à risque de développer une neutropénie, en une quantité efficace pour stimuler la prolifération des polynucléaires neutrophiles chez le sujet,
xiii) en une quantité efficace pour induire la production d'une cytokine, où la cytokine est préférablement sélectionnée dans le groupe consistant en l'IL-1 bêta (IL-1β), l'IL-2, l'IL-6, 1'IL-12, l'IL-18, le TNF-α, l'IFN-α et l'IFN-γ, ou
xiv) en une quantité efficace pour stimuler l'activité des cellules tueuses naturelles.

11. Utilisation selon la revendication 8 ou 9, où le médicament est conçu pour être administré au sujet avec un antigène, ledit antigène étant préférablement sélectionné dans le groupe consistant en un allergène, un antigène tumoral, un antigène viral, un antigène bactérien, un antigène fongique et un antigène parasitaire.

12. Utilisation selon la revendication 11, où l'antigène est administré par :
i) une voie muqueuse, où la voie muqueuse est préférablement sélectionnée dans le groupe consistant la voie orale, nasale, rectale, vaginale, transdermique et oculaire, ou
ii)une voie parentérale, où la voie parentérale est préférablement sélectionnée dans le groupe consistant en la voie intraveineuse, sous-cutanée, intramusculaire ou en injection directe.

13. Utilisation selon la revendication 8 ou 9, où le médicament est destiné à être administré au sujet avec une thérapie anticancéreuse, la thérapie anticancéreuse étant préférablement :
i) un anticorps monoclonal spécifique d'une cellule tumorale,
ii) une chimiothérapie, ou
iii) une radiothérapie.

14. Utilisation selon la revendication 8 ou 9, où le médicament est destiné à être administré:
i) par une voie muqueuse, où la voie muqueuse est préférablement sélectionnée dans le groupe consistant la voie orale, nasale, rectale, vaginale, transdermique et oculaire,
ii) par une voie parentérale, où la voie parentérale est préférablement sélectionnée dans le groupe consistant en la voie intraveineuse, sous-cutanée, intramusculaire ou en injection directe, ou
iii) dans un véhicule à libération prolongée.


### SEQUENCE LISTING

<110> Coley Pharmaceutical Group, Ltd.
<120> CpG-LIKE NUCLEIC ACIDS AND METHODS OF USE THEREOF
<130> C1041/7018WO (AWS)
<150> US 60/254,341
   <151> 2000-12-08
<160> 36
<170> FastSEQ for Windows Version 3.0
<210> 1
   <211> 18
   <212> DNA
   <213> Artificial Sequence
<220>
   <223> Synthetic oligonucleotide
<400> 1
   tctcccagcg tgcgccat 18
<210> 2
   <211> 18
   <212> DNA
   <213> Artificial Sequence
<220>
   <223> Synthetic oligonucleotide
<221> modified_base
   <222> (2) ... (2)
   <223> m5c
<221> modified_base
   <222> (4)...(6)
   <223> m5c
<221> modified_base
   <222> (9) ... (9)
   <223> m5c
<221> modified_base
   <222> (13)...(13)
   <223> m5c
<221> modified_base
   <222> (15)...(16)
   <223> m5c
<400> 2
   tctcccagcg tgcgccat 18
<210> 3
   <211> 20
   <212> DNA
   <213> Artificial Sequence
<220>
   <223> Synthetic oligonucleotide
<400> 3
   tccaggactt ctctcaggtt 20
<210> 4
   <211> 24
   <212> DNA
   <213> Artificial Sequence
<220>
   <223> Synthetic oligonucleotide
<400> 4
   tcgtcgtttt gtcgttttgt cgtt 24
<210> 5
   <211> 24
   <212> DNA
   <213> Artificial Sequence
<220>
   <223> Synthetic oligonucleotide
<221> modified_base
   <222> (2) ... (2)
   <223> m5c
<221> modified_base
   <222> (5) ... (5)
   <223> m5c
<221> modified_base
   <222> (13)...(13)
   <223> m5c
<221> modified_base
   <222> (21) ... (21)
   <223> m5c
<400> 5
   tcgtcgtttt gtcgttttgt cgtt 24
<210> 6
   <211> 24
   <212> DNA
   <213> Artificial Sequence
<220>
   <223> Synthetic oligonucleotide
<400> 6
   tgctgctttt gtgcttttgt gctt 24
<210> 7
   <211> 27
   <212> DNA
   <213> Artificial Sequence
<220>
   <223> Synthetic oligonucleotide
<400> 7
   tcgtcgctgt ctccgcttct tcttgcc 27
<210> 8
   <211> 27
   <212> DNA
   <213> Artificial Sequence
<220>
   <223> Synthetic oligonucleotide
<221> modified_base
   <222> (2)...(2)
   <223> m5c
<221> modified base
   <222> (5) ... (5)
   <223> m5c
<221> modified base
   <222> (7) ... (7)
   <223> m5c
<221> modified base
   <222> (11)...(11)
   <223> m5c
<221> modified_base
   <222> (13)...(14)
   <223> m5c
<221> modified_base
   <222> (16)...(16)
   <223> m5c
<221> modified_base
   <222> (19) ... (19)
   <223> m5c
<221> modified_base
   <222> (22)...(22)
   <223> m5c
<221> modified_base
   <222> (26) ... (27)
   <223> m5c
<400> 8
   tcgtcgctgt ctccgcttct tcttgcc 27
<210> 9
   <211> 21
   <212> DNA
   <213> Artificial Sequence
<220>
   <223> Synthetic oligonucleotide
<400> 9
   gcgtttgctc ttcttcttgc g 21
<210> 10
   <211> 20
   <212> DNA
   <213> Artificial Sequence
<220>
   <223> Synthetic oligonucleotide
<400> 10
   gcccaagctg gcatccgtca 20
<210> 11
   <211> 21
   <212> DNA
   <213> Artificial Sequence
<220>
   <223> Synthetic oligonucleotide
<400> 11
   gtgctcgagg atgcgcttcg c 21
<210> 12
   <211> 20
   <212> DNA
   <213> Artificial Sequence
<220>
   <223> Synthetic oligonucleotide
<400> 12
   ggttcttttg gtccttgtct 20
<210> 13
   <211> 21
   <212> DNA
   <213> Artificial Sequence
<220>
   <223> Synthetic oligonucleotide
<221> modified_base
   <222> (2) ... (2)
   <223> m5c
<221> modified_base
   <222> (8) ... (8)
   <223> m5c
<221> modified_base
   <222> (10) ... (10)
   <223> m5c
<221> modified_base
   <222> (13) ... (13)
   <223> m5c
<221> modified_base
   <222> (16) ... (16)
   <223> m5c
<221> modified_base
   <222> (20) ... (20)
   <223> m5c
<400> 13
   gcgtttgctc ttcttcttgc g 21
<210> 14
   <211> 20
   <212> DNA
   <213> Artificial Sequence
<220>
   <223> Synthetic oligonucleotide
<221> modified_base
   <222> (2) ... (4)
   <223> m5c
<221> modified base
   <222> (8) ... (8)
   <223> m5c
<221> modified base
   <222> (12)...(12)
   <223> m5c
<221> modified_base
   <222> (15) ... (16)
   <223> m5c
<221> modified_base
   <222> (19) ... (19)
   <223> m5c
<400> 14
   gcccaagctg gcatccgtca 20
<210> 15
   <211> 24
   <212> DNA
   <213> Artificial Sequence
<220>
   <223> Synthetic oligonucleotide
<221> modified base
   <222> (2) ... (2)
   <223> cm
<221> modified_base
   <222> (5) ... (5)
   <223> cm
<221> modified_base
   <222> (13)...(13)
   <223> cm
<221> modified_base
   <222> (21)...(21)
   <223> cm
<400> 15
   tcgtcgtttt gtcgttttgt cgtt 24
<210> 16
   <211> 20
   <212> DNA
   <213> Artificial Sequence
<220>
   <223> Synthetic oligonucleotide
<221> modified_base
   <222> (2) ... (3)
   <223> cm
<221> modified_base
   <222> (8) ... (8)
   <223> cm
<221> modified_base
   <222> (11)...(11)
   <223> cm
<221> modified_base
   <222> (13)...(13)
   <223> cm
<221> modified_base
   <222> (15)...(15)
   <223> cm
<400> 16
   tccaggactt ctctcaggtt 20
<210> 17
   <211> 20
   <212> DNA
   <213> Artificial Sequence
<220>
   <223> Synthetic oligonucleotide
<221> modified base
   <222> (5) ... (5)
   <223> cm
<221> modified_base
   <222> (13) ... (14)
   <223> cm
<221> modified_base
   <222> (19)...(19)
   <223> cm
<400> 17
   ggttcttttg gtccttgtct 20
<210> 18
   <211> 24
   <212> DNA
   <213> Artificial Sequence
<220>
   <223> Synthetic oligonucleotide
<221> modified_base
   <222> (3) ... (3)
   <223> I
<221> modified_base
   <222> (6) ... (6)
   <223> I
<221> modified_base
   <222> (11)...(11)
   <223> I
<221> modified_base
   <222> (14)...(14)
   <223> I
<221> modified_base
   <222> (19)...(19)
   <223> I
<221> modified_base
   <222> (22)...(22)
   <223> I
<400> 18
   tcntcntttt ntcnttttnt cntt 24
<210> 19
   <211> 20
   <212> DNA
   <213> Artificial Sequence
<220>
   <223> Synthetic oligonucleotide
<221> modified base
   <222> (5) ... (6)
   <223> I
<221> modified_base
   <222> (17) ... (18)
   <223> I
<400> 19
   tccannactt ctctcanntt 20
<210> 20
   <211> 20
   <212> DNA
   <213> Artificial Sequence
<220>
   <223> Synthetic oligonucleotide
<221> modified_base
   <222> (1) ... (2)
   <223> I
<221> modified_base
   <222> (10)...(11)
   <223> I
<221> modified_base
   <222> (17)...(17)
   <223> I
<400> 20
   nnttcttttn ntccttntct 20
<210> 21
   <211> 24
   <212> DNA
   <213> Artificial Sequence
<220>
   <223> Synthetic oligonucleotide
<221> modified_base
   <222> (3) ... (3)
   <223> 2-aminopurine
<221> modified_base
   <222> (6) ... (6)
   <223> 2-aminopurine
<221> modified_base
   <222> (11) ... (11)
   <223> 2-aminopurine
<221> modified_base
   <222> (14) ... (14)
   <223> 2-aminopurine
<221> modified_ base
   <222> (19) ... (19)
   <223> 2-aminopurine
<221> modified_base
   <222> (22) ... (22)
   <223> 2-aminopurine
<400> 21
   tcntcntttt ntcnttttnt cntt 24
<210> 22
   <211> 24
   <212> DNA
   <213> Artificial Sequence
<220>
   <223> Synthetic oligonucleotide,
<221> modified base
   <222> (3) ... (3)
   <223> spacer
<221> modified_base
   <222> (6) ... (6)
   <223> spacer
<221> modified_base
   <222> (14) ... (14)
   <223> spacer
<221> modified base
   <222> (22) ... (22)
   <223> spacer
<400> 22
   tcntcntttt gtcnttttgt cntt 24
<210> 23
   <211> 24
   <212> DNA
   <213> Artificial Sequence
<220>
   <223> Synthetic oligonucleotide
<221> modified_base
   <222> (2) ... (2)
   <223> spacer
<221> modified_base
   <222> (5) ... (5)
   <223> spacer
<221> modified base
   <222> (13)...(13)
   <223> spacer
<221> modified_base
   <222> (21) ... (21)
   <223> spacer
<400> 23
   tngtngtttt gtngttttgt ngtt 24
<210> 24
   <211> 24
   <212> DNA
   <213> Artificial Sequence
<220>
   <223> Synthetic oligonucleotide
<221> modified_base
   <222> (2) ... (3)
   <223> spacer
<221> modified_base
   <222> (5) ... (6)
   <223> spacer
<221> modified_base
   <222> (13)...(14)
   <223> spacer
<221> modified_base
   <222> (21)...(22)
   <223> spacer
<400> 24
   tnntnntttt gtnnttttgt nntt 24
<210> 25
   <211> 21
   <212> DNA
   <213> Artificial Sequence
<220>
   <223> Synthetic oligonucleotide
<221> modified_base
   <222> (7) .... (7)
   <223> spacer
   <221> modified_base
   <222> (15)...(15)
   <223> spacer
<221> modified_base
   <222> (20) ... (20)
   <223> spacer
<400> 25
   gtgctcnagg atgcncttcn c 21
<210> 26
   <211> 21
   <212> DNA
   <213> Artificial Sequence
<220>
   <223> Synthetic oligonucleotide
<221> modified_base
   <222> (6) ... (6)
   <223> spacer
<221> modified_base
   <222> (14) ... (14)
   <223> spacer
<221> modified_base
   <222> (19)...(19)
   <223> spacer
<400> 26
   gtgctngagg atgngcttng c 21
<210> 27
   <211> 21
   <212> DNA
   <213> Artificial Sequence
<220>
   <223> Synthetic oligonucleotide
<221> modified_base
   <222> (6)...(7)
   <223> spacer
<221> modified_base
   <222> (14) ... (15)
   <223> spacer
<221> modified_base
   <222> (19) ... (20)
   <223> spacer
<400> 27
   gtgctnnagg atgnncttnn c 21
<210> 28
   <211> 24
   <212> DNA
   <213> Artificial Sequence
<220>
   <223> Synthetic oligonucleotide
<221> modified_base
   <222> (3) ... (3)
   <223> 7-deaza-guanosine
<221> modified_base
   <222> (6) ... (6)
   <223> 7-deaza-guanosine
<221> modified_base
   <222> (14) ... (14)
   <223> 7-deaza-guanosine
<221> modified_base
   <222> (22)...(22)
   <223> 7-deaza-guanosine
<400> 28
   tcntcntttt gtcnttttgt cntt 24
<210> 29
   <211> 24
   <212> DNA
   <213> Artificial Sequence
<220>
   <223> Synthetic oligonucleotide
<221> modified_base
   <222> (3) ... (3)
   <223> nebularine
<221> modified_base
   <222> (6) ... (6)
   <223> nebularine
<221> modified_base
   <222> (14) ... (14)
   <223> nebularine
<221> modified_base
   <222> (22)...(22)
   <223> nebularine
<400> 29
   tcntcntttt gtcnttttgt cntt 24
<210> 30
   <211> 24
   <212> DNA
   <213> Artificial Sequence
<220>
   <223> Synthetic oligonucleotide
<221> modified_base
   <222> (3) ... (3)
   <223> I
<221> modified_base
   <222> (6) ... (6)
   <223> I
<221> modified_base
   <222> (14)...(14)
   <223> I
<221> modified_base
   <222> (22)...(22)
   <223> I
<400> 30
   tcntcntttt gtcnttttgt cntt 24
<210> 31
   <211> 24
   <212> DNA
   <213> Artificial Sequence
<220>
   <223> Synthetic oligonucleotide
<400> 31
   tugtugtttt gtugttttgt ugtt 24
<210> 32
   <211> 24
   <212> DNA
   <213> Artificial Sequence
<220>
   <223> Synthetic oligonucleotide
<400> 32
   tggtggtttt gtggttttgt ggtt 24
<210> 33
   <211> 24
   <212> DNA
   <213> Artificial Sequence
<220>
   <223> Synthetic oligonucleotide
<400> 33
   tcctcctttt gtccttttgt cctt 24
<210> 34
   <211> 24
   <212> DNA
   <213> Artificial Sequence
<220>
   <223> Synthetic oligonucleotide
<221> modified_base
   <222> (3) ... (3)
   <223> I
<221> modified_base
   <222> (6) ... (6)
   <223> I
<221> modified_base
   <222> (14) ... (14)
   <223> I
<221> modified_base
   <222> (22)...(22)
   <223> I
<400> 34
   tgntgntttt gtgnttttgt gntt 24
<210> 35
   <211> 24
   <212> DNA
   <213> Artificial Sequence
<220>
   <223> Synthetic oligonucleotide
<221> modified_base
   <222> (2)...(2)
   <223> 2-aminopurine
<221> modified_base
   <222> (5) ... (5)
   <223> 2-aminopurine
<221> modified_base
   <222> (13) ... (13)
   <223> 2-aminopurine
<221> modified_base
   <222> (21)...(21)
   <223> 2-aminopurine
<400> 35
   tngtngtttt gtngttttgt ngtt 24
<210> 36
   <211> 24
   <212> DNA
   <213> Artificial Sequence
<220>
   <223> Synthetic oligonucleotide
<221> modified_base
   <222> (3) ... (3)
   <223> 2,6-diaminopurine
<221> modified_base
   <222> (6)...(6)
   <223> 2,6-diaminopurine
<221> modified_base
   <222> (14)...(14)
   <223> 2,6-diaminopurine
<221> modified_base
   <222> (22)...(22)
   <223> 2,6-diaminopurine
<400> 36
   tcntcntttt gtcnttttgt cntt 24